# EUROPEAN PATENT SPECIFICATION

(11) **EP 4 292 708 B1**
(45) Date of publication and mention of the grant of the patent: **04.03.2026**
(21) Application number: 22752574.8
(22) Date of filing: 25.01.2022
(51) Int. Cl.: C01B 32/80, C07C 51/56, C07C 63/06, C07C 67/08, C07C 68/00, C07C 69/78, C07C 69/96, C07C 263/10, C07C 265/04, C07C 265/14, C07D 263/44, C07D 317/38, C08G 64/24

(54) **METHOD FOR PRODUCING CARBONYL HALIDE**
VERFAHREN ZUR HERSTELLUNG VON CARBONYLHALOGENID
PROCÉDÉ DE PRODUCTION D'HALOGÉNURE DE CARBONYLE

(30) Priority: 12.02.2021 JP 2021021001
(43) Date of publication of application: 20.12.2023
(73) Proprietor: National University Corporation Kobe University, Kobe-shi, Hyogo 657-8501 (JP); AGC INC., Chiyoda-ku, Tokyo 1008405 (JP); MITSUBISHI GAS CHEMICAL COMPANY, INC., Chiyoda-ku Tokyo 100-8324 (JP)
(72) Inventor: TSUDA, Akihiko, Kobe-shi, Hyogo 657-8501 (JP); OKAZOE, Takashi, Tokyo 100-8405 (JP); HARADA, Hidefumi, Tokyo 125-8601 (JP)
(74) Representative: TBK
(86) International application number: PCT/JP2022/002661
(87) International publication number: WO 2022/172744

(56) References cited:
- WO-A1-2015/156245
- WO-A1-2020/050368
- WO-A1-2020/100971
- WO-A1-2021/045105
- JP-A- 2007 527 841
- JP-A- 2013 181 028
- JP-A- 2020 083 765
- JP-A- 2020 083 882
- LIANG FENGYING, YANAI MASAKI, SUZUKI YUTO, TSUDA AKIHIKO: "Photo-on-Demand Synthesis of Chloroformates with a Chloroform Solution Containing an Alcohol and Its One-Pot Conversion to Carbonates and Carbamates", ORGANIC LETTERS, AMERICAN CHEMICAL SOCIETY, US, vol. 22, no. 9, 1 May 2020 (2020-05-01), US , pages 3566 - 3569, XP055957509, ISSN: 1523-7060, DOI: 10.1021/acs.orglett.0c01013

## Description

### [TECHNICAL FIELD]

The present invention relates to a method for producing a carbonyl halide efficiently in terms of an amount of a used halogenated methane.

### BACKGROUND ART

A carbonyl halide such as phosgene is very important as a synthetic intermediate of various compounds and a raw material of a material. For example, a carbonate compound is generally produced from phosgene and an alcohol compound.

Phosgene is however very toxic. For example, phosgene is easily reacted with water to generate hydrogen chloride and has a history of being used as poisonous gas. In general, phosgene is produced by a high-heat-generating gas phase reaction between an anhydrous chlorine gas and high purity carbon monoxide in the presence of an activated carbon catalyst. Carbon monoxide used in this reaction is also toxic. The basic process to produce phosgene has not changed much since the 1920s. Such a process to produce phosgene requires expensive large-scale facilities. An extensive safety assurance is essential in plant design due to the high toxicity of phosgene and leads to increased production costs.

The inventors of the present invention have developed the method for producing a halogen and/or a carbonyl halide by irradiating light to a halogenated hydrocarbon in the presence of oxygen (Patent document 1). The method is safe, since the carbonyl halide produced by the method can be directly supplied to a reactive substrate compound such as an amine compound and an alcohol compound. In addition, the carbonyl halide that has not used for the reaction can be collected in order not to be leaked outside by using a trap. For example, the inventors also have developed the method for producing a halogenated carboxylate ester by irradiating light to a mixture containing a halogenated hydrocarbon and an alcohol in the presence of oxygen (Patent document 2). In addition, the inventors also have developed the method for producing a carbonate derivative by irradiating light to a composition containing a halogenated hydrocarbon, a nucleophilic functional group-containing compound and a base in the presence of oxygen (Patent document 3 and Patent document 4).

When a carbonyl halide is produced by the above-described methods, a large amount of a halogenated hydrocarbon remains in comparison with the produced carbonyl halide. A halogenated hydrocarbon cannot be easily discarded and is needed to be purified and reused due to high environment load.

It has been known from a long time ago that a carbonyl halide is decomposed by light. For example, Patent document 5 discloses the method for decomposing phosgene to be removed by photodecomposition by irradiating ultraviolet light to boron trichloride containing phosgene as an impurity. It is also described in Non-patent document 1 that phosgene is decomposed by irradiating light.

### PRIOR ART DOCUMENT

JP 2020 083765 A discloses a method for producing fluorinated carbonate and includes a step of irradiating a composition containing a halogenated methane and a specific hydroxyl group-containing fluorinated compound with high energy light in the presence of oxygen, and in which, characterized, the molar ratio of the hydroxyl group-containing fluorinated compound to 1 mol of the halogenated methane is 0.2 or more.

JP 2013 181028 A discloses a halogen and/or carbonyl halide derived from a mixture obtained by irradiating a halogenated hydrocarbon with light under the presence of oxygen as a chemical reaction raw material in a chemical reaction.

FENGYING LIANG, ET AL.: "Photo-on-demand synthesis of chloroformates with a chloroform solution containing an alcohol and its one-pot conversion to carbonates and carbamates", Org. Lett., Vol. 22, No. 9, 1 May 2020 (2020-05-01), pages 3566-3569 discloses two new reaction processes involving the in situ oxidative photochemical conversion of CHCl₃ to COCl₂ allowed selective syntheses of N-substituted ureas and isocyanates from amines. (I) A CHCl₃ solution containing an amine and an organic base under O₂ bubbling provided the urea derivative under exposure to UV light generated from a low-pressure mercury lamp at 20-40 °C. (II) A two-step reaction involving the oxidative photodecomposition of CHCl₃ at lower temperatures and subsequent sequential injections of an amine and organic base into the sample solution providing the isocyanate in high yield. The reaction processes of (I) and (II) capitalize on the solution conditions of [COCl₂] < [amine] and [COCl₂] > [amine], respectively, resulting in 1:2 and 1:1 reactions. In general, isocyanates, especially aromatic and haloalkyl ones, readily undergo hydrolysis in the presence of an organic base. However, with the advantage of synthesizing the isocyanates in CHCl₃ solvent, direct addition of monoalcohols and diols to the as-prepared sample solution containing the diisocyanate allowed the one-pot syntheses of biscarbamates and polyurethanes, respectively. The reactions developed in this study are simple, safe, and inexpensive methods of synthesizing N-substituted ureas and isocyanates, and derivatives of isocyanates such as carbamates and polyurethanes.

WO 2020 100971 A1 discloses a method that makes it possible to safely and efficiently produce an isocyanate compound. This production method for an isocyanate compound is characterized by including: a step for irradiating a halomethane with high-energy light in the presence of oxygen at a temperature of no higher than 15 °C; and a step for adding an amine compound and reacting without radiating the high-energy light.

JP 2020 083882 A discloses a method for producing an amino acid-N-carboxylic acid anhydride, characterized in that the amino acid-N-carboxylic acid anhydride is represented by the formula (II), and a step of irradiating a composition containing a halogenated methane and an amino acid compound represented by formula (I) with high energy light in the presence of oxygen is included.

WO 2020 050368 A1 discloses a method for preparing a Vilsmeier reagent, that can be carried out safely, easily, and at a low cost; and a method for producing an aromatic aldehyde or an aromatic ketone, a carboxylic halide, and a formic ester. A method for preparing a Vilsmeier reagent is characterized by comprising: a step for degrading C1-4 halogenated hydrocarbon by irradiating with light, in the presence of an enzyme, a composition that comprises a C1-4 halogenated hydrocarbon having at least one halogen group selected from the group consisting of chlorine, bromine and iodine; and a step for reacting a degraded product of the C1-4 halogenated hydrocarbon with a specific amide compound.

### PATENT DOCUMENT

Patent document 1: JP 2013-181028 A
Patent document 2: WO 2015/156245
Patent document 3: WO 2018/211952
Patent document 4: WO 2018/211953
Patent document 5: US 4,405,423 B

### NON-PATENT DOCUMENT

Non-patent document 1: C. W. Montgomery et al., J. Am. Chem. Soc., 1934, 56, 5, pp. 1089-1092

### DISCLOSURE OF THE INVENTION

### PROBLEMS TO BE SOLVED BY THE INVENTION

The inventors of the present invention have developed a method for producing a carbonyl halide by irradiating light to a halogenated hydrocarbon as described above, and the reaction efficiency of the produced carbonyl halide and an alcohol compound or the like is high. But the yield to the used halogenated hydrocarbon is low, since a large amount of a halogenated hydrocarbon is used.

Thus, the objective of the present invention is to provide a method for producing a carbonyl halide efficiently to the used halogenated methane.

### MEANS FOR SOLVING THE PROBLEMS

The inventors of the present invention repeated intensive studies in order to solve the above-described problems. For example, the inventors anticipated that if high energy light is irradiated to vaporized halogenated methane, the halogenated methane can be efficiently photodecomposed but the produced carbonyl halide may be also rapidly photodecomposed in a gas phase. On the one hand, the inventors examined various reaction conditions; as a result, the inventors completed the present invention by finding that a carbonyl halide can be surprisingly produced with high yield by irradiating high energy light to vaporized flowed halogenated methane.

The present invention is hereinafter described.

[1] A method for producing a carbonyl halide, comprising the steps of:
   preparing a mixed gas comprising oxygen and a halogenated methane having one or more halogeno groups selected from the group consisting of chloro, bromo and iodo, and
   flowing the mixed gas and irradiating a high energy light containing light having a wavelength of 180 nm or more and 830 nm or less to the flowed mixed gas.
[2] The method according to the above [1], wherein a shortest distance from a light source of the high energy light to the flowed mixed gas is 1 m or less.
[3] The method according to the above [1] or [2], wherein time to irradiate the high energy light to the flowed mixed gas is 1 second or more and 10000 seconds or less.
[4] The method according to any one of the above [1] to [3], wherein a temperature during the irradiation of the high energy light to the flowed mixed gas is 40°C or higher and 200°C or lower.
[5] The method according to any one of claims 1 to 4, wherein the high energy light contains light having a wavelength of 180 nm or more and 500 nm or less.
[6] A method for producing a fluorinated carbonate compound, comprising the steps of:
   producing a carbonyl halide by the method according to any one of the above [1] to [5], and
   reacting a fluorinated alcohol compound and the carbonyl halide,
   wherein a molar ratio of the fluorinated alcohol compound to the halogenated methane is adjusted to 1 or more.
[7] A method for producing a non-fluorinated carbonate compound, comprising the steps of:
   producing a carbonyl halide by the method according to any one of the above [1] to [5], and
   reacting a non-fluorinated alcohol compound and the carbonyl halide,
   wherein a molar ratio of the non-fluorinated alcohol compound to the halogenated methane is adjusted to 1 or more.
[8] A method for producing a halogenated formic acid fluorinated ester compound, comprising the steps of:
   producing a carbonyl halide by the method according to any one of the above [1] to [5], and
   reacting a fluorinated alcohol compound and the carbonyl halide,
   wherein a molar ratio of the fluorinated alcohol compound to the halogenated methane is adjusted to less than 1.
[9] A method for producing a halogenated formic acid non-fluorinated ester compound, comprising the steps of:
   producing a carbonyl halide by the method according to any one of the above [1] to [5], and
   reacting a non-fluorinated alcohol compound and the carbonyl halide,
   wherein a molar ratio of the non-fluorinated alcohol compound to the halogenated methane is adjusted to less than 1.
[10] A method for producing an isocyanate compound, comprising the steps of:
   producing a carbonyl halide by the method according to any one of the above [1] to [5], and
   reacting a primary amine compound and the carbonyl halide,
   wherein a molar ratio of the primary amine compound to the halogenated methane is adjusted to less than 1.
[11] A method for producing an amino acid-N-carboxylic anhydride, comprising the steps of:
   producing a carbonyl halide by the method according to any one of the above [1] to [5], and
   reacting an amino acid compound represented by the following formula (VII) and the carbonyl halide,
   wherein the amino acid-N-carboxylic anhydride is represented by the following formula (VIII): 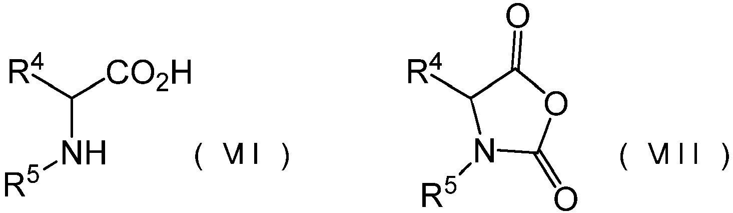 wherein
   R⁴ is an amino acid side chain group wherein a reactive group is protected,
   R⁵ is H or P¹-[-NH-CHR⁶-C(=O)-]_{I}- wherein R⁶ is an amino acid side chain group wherein a reactive group is protected, P¹ is a protective group of the amino group, I is an integer of 1 or more, and when I is an integer of 2 or more, a plurality of R⁶ may be the same as or different from each other.
[12] A method for producing a Vilsmeier reagent,
   wherein the Vilsmeier reagent is a salt represented by the following formula (X): 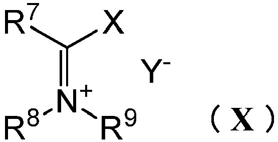 wherein
   R⁷ is a hydrogen atom, a C₁₋₆ alkyl group or an optionally substituted C₆₋₁₂ aromatic hydrocarbon group,
   R⁸ and R⁹ are independently a C₁₋₆ alkyl group or an optionally substituted C₆₋₁₂ aromatic hydrocarbon group, or R⁸ and R⁹ may form a 4 or more and 7 or less-membered ring structure together with each other,
   X is a halogeno group selected from the group consisting of chloro, bromo and iodo,
   Y⁻ is a counter anion,
   comprising the steps of:
      producing a carbonyl halide by the method according to any one of the above [1] to [5], and
      reacting the carbonyl halide and an amide compound represented by the following formula (IX): 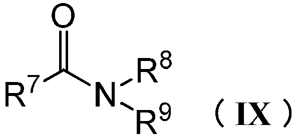
wherein R⁷ to R⁹ have the same meanings as the above.

### EFFECT OF THE INVENTION

The use and treatment of a halogenated methane is restricted due to a high environmental impact. On the one hand, a carbonyl halide can be produced efficiently from the used halogenated methane, and a halogenated methane can be effectively used by the present invention. Thus, the present invention is very industrially useful as the technology that enable effective utilization of a halogenated methane and efficient production of a carbonyl halide such as phosgene.

### BRIEF DESCRIPTION OF THE DRAWINGS

Fig. 1 is a schematic diagram to demonstrate one example of the constitution of a reaction system usable in the present invention method.
Fig. 2 is a schematic diagram to demonstrate one example of the constitution of a reaction system usable in the present invention method.
Fig. 3 is a schematic diagram to demonstrate one example of the constitution of a reaction system usable in the present invention method.
Fig. 4 is a schematic diagram to demonstrate one example of the constitution of a reaction system usable in the present invention method.
Fig. 5 is a schematic diagram to demonstrate one example of the constitution of a reaction system usable in the present invention method.
Fig. 6 is a schematic diagram to demonstrate one example of the constitution of a reaction system usable in the present invention method.

### MODE FOR CARRYING OUT THE INVENTION

The present invention method is hereinafter described step by step, and the present invention is not restricted to the following specific examples.

### 1. Step to prepare mixed gas

The mixed gas comprising oxygen and a halogenated methane comprising one or more halogeno groups selected from the group consisting of chloro, bromo and iodo in this step.

The halogenated methane used in the present invention means a methane that has one or more halogeno groups selected from the group consisting of chloro, bromo and iodo. The halogenated methane may be decomposed by oxygen and high energy light to become a carbonyl halide.

The halogenated methane may be decomposed by oxygen and high energy light as described above and play a role similarly to a carbonyl halide in the present invention. The halogenated methane is preferably a polyhalogenated methane having 2 or more halogeno groups and preferably a perhalogenated methane, in which all of the hydrogen atoms are substituted with halogeno groups.

An example of the specific halogenated methane includes a halogenated methane such as dichloromethane, chloroform, dibromomethane, bromoform, iodomethane and diiodomethane.

The halogenated methane may be appropriately selected depending on the target chemical reaction and the desired product. Only one kind of the halogenated methane may be used, or two or more kinds of the halogenated methanes may be used in combination. Only one kind of the halogenated methane is preferably used depending on the target compound to be produced. The halogenated methane having a chloro group is preferred among the halogenated methanes in terms of vaporization and cost.

A general halogenated methane product may contain a stabilizing agent such as an alcohol to inhibit the decomposition of the halogenated methane. Since the halogenated methane is oxidatively photodecomposed in the present invention, a stabilizing agent may be removed from the halogenated methane to be used. When the halogenated methane from which a stabilizing agent is removed is used, the halogenated methane may be possibly decomposed more efficiently. For example, high energy light of which energy is relatively low may be used, and the time to irradiate high energy light may be reduced. A method for removing a stabilizing agent from the halogenated methane is not particularly restricted. For example, the halogenated methane may be washed with water to remove a water soluble stabilizing agent and then dried.

Chloroform, which is used as a general-purpose solvent and is inexpensive, can be used as the halogenated methane used in the present invention method. For example, the halogenated methane that has been once used as a solvent may be recovered to be reused. It is preferred that such a used halogenated methane is purified to some extent for use, since if a large amount of an impurity and water is contained, the reaction may be possibly inhibited. For example, it is preferred that water and a water-soluble impurity is removed by washing with water and then the halogenated methane is dried over anhydrous sodium sulfate, anhydrous magnesium sulfate or the like. Excessive purification by which the productivity becomes less is not needed, since even when water about 1 mass% is contained, the reaction may proceed. The water content is preferably 0.5 mass% or less, more preferably 0.2 mass% or less, and even preferably 0.1 mass% or less. The water content is preferably detection limit or less, or 0 mass%. The above-described reused halogenated methane may contain the degradant of the halogenated methane.

In particular, when the halogenated methane is not liquid in an atmospheric temperature and an atmospheric pressure or difficult to vaporize, a solvent may be used in addition to the halogenated methane. Such a solvent may possibly accelerate the decomposition of the halogenated methane. In addition, the solvent may possibly inhibit the decomposition of the carbonyl halide produced by the decomposition of the halogenated methane. It is preferred that the solvent can appropriately dissolve the halogenated methane and does not inhibit the decomposition of the halogenated methane. An example of the solvent includes a ketone solvent such as acetone, methyl ethyl ketone, methyl isobutyl ketone and cyclohexanone; an ester solvent such as ethyl acetate; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; and a nitrile solvent such as acetonitrile.

An oxygen source may be a gas containing oxygen, and for example, air or purified oxygen may be used. Purified oxygen may be mixed with an inert gas such as nitrogen and argon to be used. It is preferred to use air in terms of cost and easiness. An oxygen content in an oxygen-containing gas used as an oxygen source is preferably about 15 vol% or more and about 100 vol% or less in terms of high decomposition efficiency of the halogenated methane by irradiation of high energy light. Substantially oxygen only other than an inevitable impurity is preferably used. The oxygen content may be appropriately determined depending on the kind of the halogenated methane or the like. For example, when a chloro methane such as dichloromethane and chloroform is used as the halogenated methane, the oxygen content is preferably 15 vol% or more and 100 vol% or less. When a bromo methane such as dibromomethane and bromoform is used, the oxygen content is preferably 90 vol% or more and 100 vol% or less. Even when oxygen having an oxygen content of 100 vol% is used, the oxygen content can be controlled in the above-described range by adjusting a flow amount of oxygen into the reaction system.

Dried air may be used as the oxygen source. Since even air containing water vapor may not excessively inhibit the reaction, air may be used without adjusting water vapor content. The oxygen concentration in the air is about 21 vol%, and the oxygen concentration in the oxygen source may be also adjusted to 20±5 vol%. The concentration is preferably 20±2 vol%. When air is used as the oxygen source, an air component other than oxygen may absorb excessive high energy light and reduces the concentration of the produced carbonyl halide; as a result, the decomposition of the produced carbonyl halide may possibly be inhibited.

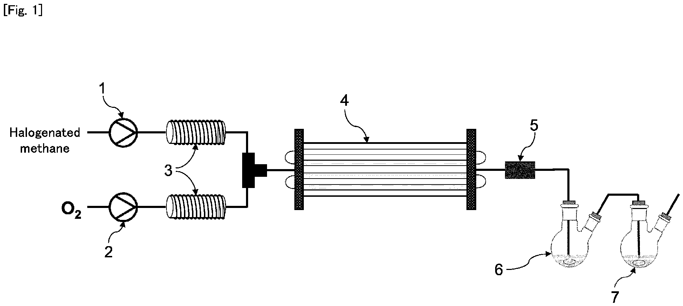

A mixed gas containing the gaseous halogenated methane and oxygen is prepared in this step. The condition to prepare the mixed gas is not particularly restricted. For example, the halogenated methane is supplied to the heater 3 using the syringe pump 1 at the predetermined flow amount to vaporize the halogenated methane by heating to the boiling point or higher, and the predetermined flow amount of an oxygen and the vaporized halogenated methane are mixed using the mass flow controller 2 to obtain a mixed gas as demonstrated in Figures 1, 2, 4 to 6.

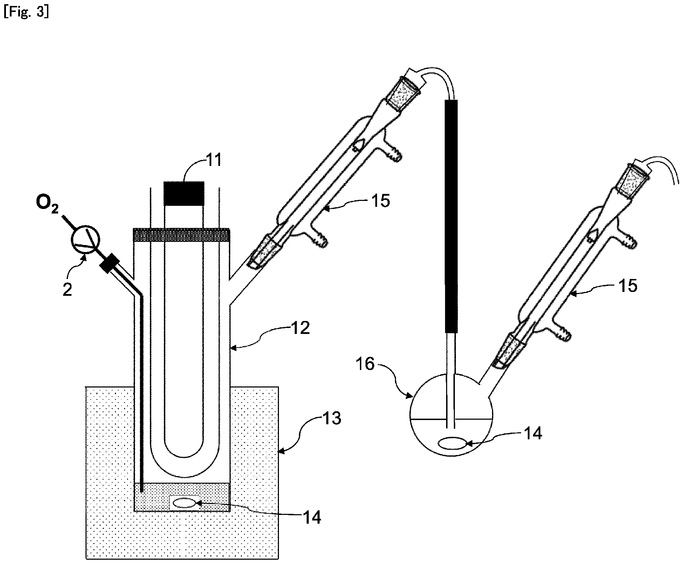

Alternatively, the bath temperature of the photoreaction vessel 12 equipped with the light source 11 and the bath 13 is preliminarily set to the boiling point or higher of the halogenated methane, and then the halogenated methane is supplied into the photoreaction vessel 12 to be vaporized as demonstrated in Figure 3. The supplied halogenated methane may be stirred using the stirring bar 14 to accelerate the vaporization of the halogenated methane. The halogenated methane is vaporized and an oxygen-containing gas is supplied into the gas phase of the photoreaction vessel 12 at the predetermined flow amount to prepare the mixed gas containing the halogenated methane and oxygen in the photoreaction vessel 12.

The ratios of the vaporized halogenated methane and oxygen in the mixed gas may be appropriately adjusted as long as a carbonyl halide can be successfully produced. For example, the ratio of the flow amount of oxygen in an oxygen-containing gas to the flow amount of the halogenated methane in the mixed gas may be adjusted to 0.1 or more and 10 or less. When the ratio is 0.1 or more, the halogenated methane may be oxidatively photodecomposed sufficiently. When the ratio is 10 or less, the produced carbonyl halide may be sufficiently prevented from further being oxidatively photodecomposed. The ratio is preferably 0.2 or more, more preferably 0.4 or more, even more preferably 0.5 or more, and preferably 8 or less, more preferably 6 or less. In particular, when the ratio is 0.5 or more, the production of a by-product and trouble of the reaction system due to a by-product may be suppressed more effectively.

When an oxygen-containing gas is supplied into the gas phase containing the vaporized halogenated methane in the reaction system demonstrated as Figure 3, an enough amount of oxygen that causes oxidative photodecomposition of the halogenated methane is preferably used. For example, the flow amount of oxygen per 1 minute to 1 mole of the halogenated methane may be adjusted to 0.1 L or more and 100 L or less. The ratio is preferably 1 L or more, more preferably 5 L or more, and even more preferably 10 L or more.

### 2. Oxidative photodecomposition step

The mixed gas containing the halogenated methane and oxygen is flowed, and high energy light is irradiated to the flowed mixed gas in the gas phase to produce a carbonyl halide by oxidative photodecomposition of the halogenated methane in this step.

The high energy light to be irradiated to the flowed mixed gas preferably contains short wavelength light and more preferably contains ultraviolet light. The high energy light contains the light having a wavelength of 180 nm or more and 830 nm or less and the light containing the light having the peak wavelength of 180 nm or more and 500 nm or less is preferred. The wavelength of the high energy light may be appropriately determined, is more preferably 400 nm or less and even more preferably 300 nm or less, and the light of which peak wavelength is included in the above ranges is also preferred. When the irradiated light contains the light of which wavelength is included in the above ranges, the halogenated methane can be oxidatively photodecomposed efficiently. For example, the light containing UV-B having the wavelength of 280 nm or more and 315 nm or less and/or UV-C having the wavelength of 180 nm or more and 280 nm or less may be used, the light containing UV-C having the wavelength of 180 nm or more and 280 nm or less is preferably used, and the light of which peak wavelength is included in the ranges is also preferred.

Since the gaseous halogenated methane is oxidatively photodecomposed in the present invention, even the high energy light having relatively low energy may possibly oxidatively photodecompose the halogenated methane. In particular, when the halogenated methane that does not contain a stabilizing agent is used, even the high energy light having relatively low energy may possibly oxidatively photodecompose the halogenated methane. An example of the high energy light having relatively low energy includes the light of which peak wavelength is included in visible light wavelength range. The visible light wavelength range may be 350 nm or more and 830 nm or less, and is preferably 360 nm or more, more preferably 380 nm or more, even more preferably 400 nm or more, preferably 800 nm or less, more preferably 780 nm or less, even more preferably 500 nm or less.

A means for the light irradiation is not particularly restricted as long as the light having the above-described wavelength can be irradiated by the means. An example of a light source of the light having such a wavelength range includes sunlight, low pressure mercury lamp, medium pressure mercury lamp, high pressure mercury lamp, ultrahigh pressure mercury lamp, chemical lamp, black light lamp, metal halide lamp and LED lamp. A low pressure mercury lamp is preferably used in terms of a reaction efficiency and a cost.

The condition such as a strength of the irradiation light may be appropriately determined depending on the halogenated methane or the like. For example, a light strength at the shortest distance position of the flowed mixed gas from the light source is determined depending on the production scale and the wavelength of the irradiation light and is preferably 1 mW/cm² or more and 200 mW/cm² or less. For example, when the wavelength of the irradiation light is relatively short, the light strength is more preferably 100 mW/cm² or less or 50 mW/cm² or less and even more preferably 20 mW/cm² or less or 10 mW/cm² or less. When the wavelength of the irradiation light is relatively long, the light strength is more preferably 10 mW/cm² or more and 20 mW/cm² or more and may be 50 mW/cm² or more or 100 mW/cm² or more. The shortest distance between the light source and the flowed mixed gas is preferably 1 m or less, more preferably 50 cm or less, and even more preferably 10 cm or less or 5 cm or less. The lower limit of the shortest distance is not particularly restricted and may be 0 cm. In other words, the light source is placed in the flowed mixed gas.

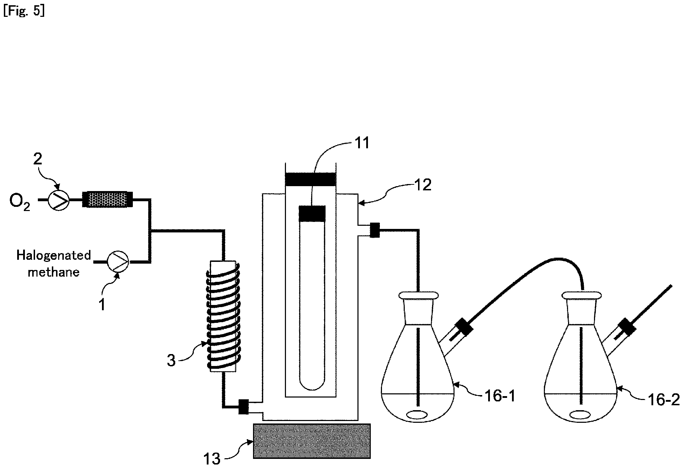

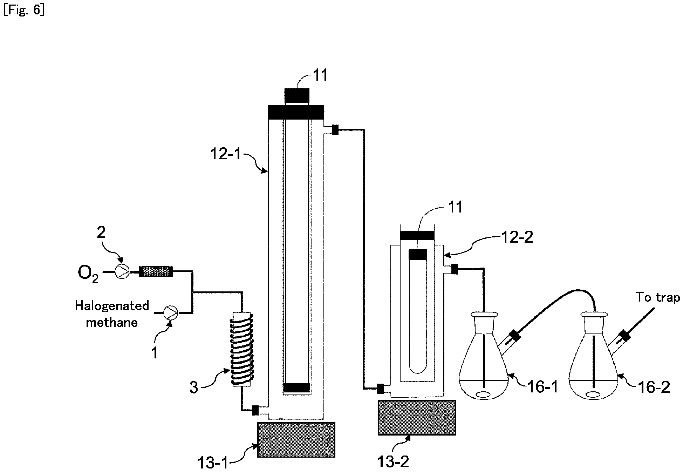

The condition to irradiate the high energy light to the flowed mixed gas is not particularly restricted. For example, the flow photoreaction device 4 is constructed by arranging one or more reaction tubes around the light source, or the flow photoreaction device 4 may has a gas inlet and a gas outlet at both ends and the light source is inserted thereinto. The mixed gas may be supplied through the flow photoreaction device 4 as Figures 1, 2 and 4. A reaction tube may be spirally wrapped around the light source in order to efficiently irradiate high energy light to the mixed gas in the flow photoreaction device 4. The flow photoreaction device 4 may be equipped with a heating means to maintain the gas state of the halogenated methane. An example such a heating means includes a hot bath into which a part or all of the flow photoreaction device 4 can be immersed and a heater that can heat a part or all of the outside of the flow photoreaction device 4. Alternatively, the photoreaction vessel 12 having the light source 11 inside is constructed, the halogenated methane is vaporized in the photoreaction vessel 12, and high energy light may be irradiated from the light source 11 with supplying an oxygen-containing gas into the photoreaction vessel 12 as demonstrated in Figure 3. Also, the vaporized halogenated methane and an oxygen-containing gas may be supplied into the photoreaction vessel 12 as demonstrated in Figures 5 and 6.

The vaporized halogenated methane may be oxidatively photodecomposed to a carbonyl halide by oxygen and the high energy light. It also has been known that a carbonyl halide is decomposed by high energy light. Thus, it is important to adjust the condition to irradiate the high energy light in order not to excessively decompose the produced carbonyl halide.

For example, the time to irradiate the above-described high energy light to the above-described flowed mixed gas may be adjusted depending on the wavelength of the irradiation light and the reaction temperature and is preferably 1 second or more and 2000 seconds or less. The time to irradiate the high energy light corresponds to the retention time of the flowed mixed gas in the photoreaction vessel in which the high energy light is continuously irradiated to the flowed mixed gas. When the time is 1 second or more, the vapored halogenated methane can be oxidatively photodecomposed more surely. When the time is 2000 seconds or less, the excessive decomposition of the produced carbonyl halide may be inhibited more surely. The time is preferably 5 seconds or more, more preferably 10 seconds or more, even more preferably 20 seconds or more or 30 seconds or more, and preferably 1500 seconds or less, 1000 seconds or less, 500 seconds or less or 300 seconds or less, more preferably 100 seconds or less, even more preferably 60 seconds or less or 50 seconds or less. When the halogenated methane that does not contain a stabilizing agent is used, the decomposition of the produced carbonyl halide can be further inhibited by using the light of which wavelength is relatively long. The light irradiation time may be adjusted within 1 second or more and 10000 seconds or less in such a case. The light irradiation time is preferably 5000 seconds or less and more preferably 1000 seconds or less in terms of the production efficiency.

When the time to irradiate the high energy light is longer, the halogenated methane may be decomposed more efficiently but the produced carbonyl halide may be further oxidatively photodecomposed. On the one hand, when the oxygen concentration in the mixed gas is adjusted to be low, the halogenated methane may be oxidatively photodecomposed with suppressing the further oxidative photodecomposition of the carbonyl halide. For example, when the oxygen concentration in the mixed gas is adjusted to 15±5 vol% and preferably 15±2 vol%, the time to irradiate light to the mixed gas may be adjusted to 50 seconds or more, 100 seconds or more, 150 seconds or more, 200 seconds or more, 500 seconds or more or 1000 seconds or more.

The flow rate of the flowed mixed gas in the photoreaction vessel to irradiate high energy light to the flowed mixed gas is preferably determined in consideration of the internal volume of the photoreaction vessel. For example, when the internal volume of the photoreaction vessel is large, the residence time of the mixed gas tends to become longer and thus the flow rate is preferably adjusted to be increased. On the one hand, when the internal volume of the photoreaction vessel is small, the flow rate of the mixed gas is preferably adjusted to be reduced. Specifically, since the internal volume of the photoreaction vessel (L) / the flow rate of the flowed mixed gas (L/s) corresponds to the residence time (s) of the flowed mixed gas, the flow rate of the flowed mixed gas can be determined in consideration of the desired residence time and the internal volume of the photoreaction vessel. The flow rate of the flowed mixed gas can be regarded as the same as the flow rate of the oxygen-containing gas in the embodiment demonstrated in Figure 3.

The linear velocity of the flowed mixed gas in the photoreaction vessel may be adjusted to 0.001 m/min or more and 100 m/min or less. When the linear velocity is 0.001 m/min or more, the photodecomposition of the carbonyl halide produced from the halogenated methane by the gas phase reaction can be inhibited more surely. When the linear velocity is 100 m/min or less, the time to transform the halogenated methane to a carbonyl halide can be sufficiently ensured more surely. The linear velocity can be calculated by dividing the flow rate of the flowed mixed gas passing through the photoreaction vessel by the cross-sectional area of the photoreaction vessel. When the cross-sectional area of the photoreaction vessel is not constant, the cross-sectional area may be regarded as the average value of the cross-sectional areas of the photoreaction vessel in the moving direction of the flowed mixed gas. The average value can be calculated by dividing the internal volume of the photoreaction vessel by the length of the photoreaction vessel in the moving direction of the flowed mixed gas. The linear velocity is preferably 0.01 m/min or more, and preferably 50 m/min or less or 20 m/min or less, more preferably 10 m/min or less or 5 m/min or less, even more preferably 1 m/min or less or 0.5 m/min or less.

The temperature during the irradiation of the high energy light to the vaporized halogenated methane may be appropriately adjusted as long as the vaporization of the halogenated methane can be maintained and the excessive decomposition of the produced carbonyl halide can be inhibited. For example, though the boiling point of dichloromethane is 40°C and the boiling point of chloroform is 61.2°C under atmospheric pressure, the gas state of the halogenated methane can be maintained even under the temperature of less than the boiling point by mixing the gaseous halogenated methane with oxygen or air. For example, the temperature may be adjusted to 35°C or higher and 250°C or lower. The temperature is preferably 40°C or higher or 50°C or higher, more preferably 70°C or higher or 80°C or higher, even more preferably 85°C or higher, and preferably 200°C or lower, more preferably 150°C or lower, even more preferably 120°C or lower. The temperature may be adjusted by the temperature of the vaporized halogenated methane and/or the temperature of the oxygen-containing gas supplied into the reaction vessel. The reaction vessel may be heated using a heat medium to maintain the temperature of the mixed gas in the reaction vessel.

When the high energy light is irradiated to the halogenated methane, the mixed gas containing the halogenated methane and oxygen may not be pressurized but may be pressurized to the extent that at least the mixed gas can pass through the reaction vessel. In addition, the productivity may be improved by pressurizing the mixed gas. The gauge pressure of the mixed gas in the reaction vessel may be adjusted to 0 MPaG or more and 2 MPaG or less and is preferably 1 MPaG or less and more preferably 0.5 MPaG or less.

The halogenated methane is oxidatively photodecomposed and a carbonyl halide [X-C(=O)-X wherein X is one or more halogeno groups selected from the group consisting of chloro, bromo and iodo.] may be produced. A carbonyl halide-like compound which plays a similar role to a carbonyl halide may be also produced in addition to a carbonyl halide. The carbonyl halide-like compound is included in the carbonyl halide of the present invention. The representative reactions in which the carbonyl halide is used is hereinafter described.

### 3. Post reaction step - production of carbonate compound

A carbonate compound can be produced by reacting the carbonyl halide and an alcohol compound.

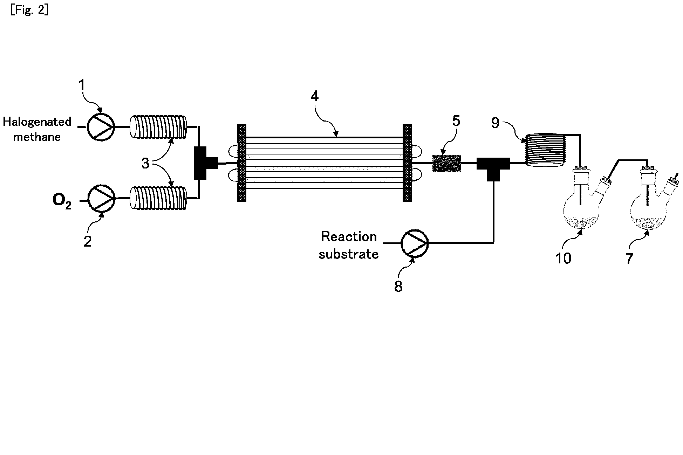

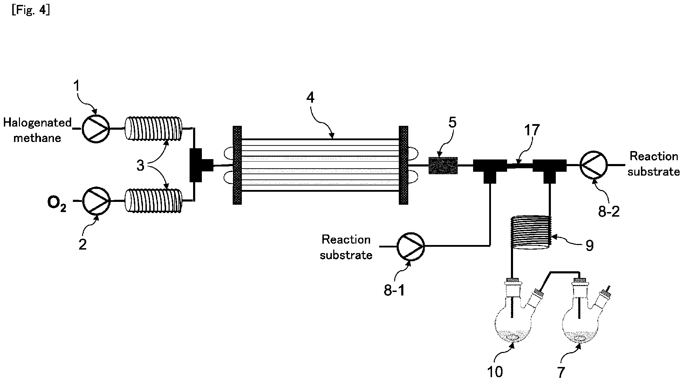

The conditions of the reaction are not particularly restricted. For example, the gas containing the produced carbonyl halide may be blown into the composition containing an alcohol compound in the reaction vessel 6 as demonstrated in Figure 1. Also, an alcohol compound is supplied into the temperature-adjustable coil reaction device 9, and the carbonyl halide and an alcohol compound may be reacted in the coil reaction device as demonstrated in Figure 2 and Figure 4. The alcohol compound may be vaporized by adjusting the temperature of the coil reaction device in order to react the carbonyl halide and the alcohol compound in a gas phase in this case. Furthermore, the carbonyl halide produced in the photoreaction vessel 12 is pushed out from the photoreaction vessel 12 by the supplied oxygen-containing gas and blown into the composition containing an alcohol compound in the reaction vessel 16 as demonstrated in Figures 3, 5 and 6. The cooling condenser 15 may be set between the photoreaction vessel 12 and the reaction vessel 16 as demonstrated in Figure 3. The temperature of the cooling condenser is preferably adjusted so that the produced carbonyl halide can pass therethrough. For example, since the boiling point of phosgene among a carbonyl halide is 8.2°C, the temperature of the cooling condenser 15 is preferably adjusted to 10°C or higher in the case of phosgene.

An alcohol compound means an organic compound having a hydroxy group and may be exemplified by a monovalent alcohol compound represented by the following formula (I) or a divalent alcohol compound represented by the following formula (II). Hereinafter, the compound represented by formula x is abbreviated as "compound x" in some cases. For example, the monovalent alcohol compound represented by the formula (I) is abbreviated as the "monovalent alcohol compound (I)" in some cases.

R¹-OH ··· (I)

HO-R²-OH ··· (II)

wherein R¹ is a monovalent organic group and R² is a divalent organic group.

An organic group is not particularly restricted as long as the organic group is inactive in the reaction of this step and is exemplified by an optionally substituted C₁₋₁₀ aliphatic hydrocarbon group, an optionally substituted C₆₋₁₂ aromatic hydrocarbon group, an optionally substituted heteroaryl group, an organic group constructed by binding 2 or more and 5 or less of an optionally substituted C₁₋₁₀ aliphatic hydrocarbon group and an optionally substituted C₆₋₁₂ aromatic hydrocarbon group, and an organic group constructed by binding 2 or more and 5 or less of an optionally substituted C₁₋₁₀ aliphatic hydrocarbon group and an optionally substituted heteroaryl group.

An example of the C₁₋₁₀ aliphatic hydrocarbon group includes a C₁₋₁₀ chain aliphatic hydrocarbon group, a C₃₋₁₀ cyclic aliphatic hydrocarbon group, and an organic group constructed by binding 2 or more and 5 or less of a C₁₋₁₀ chain aliphatic hydrocarbon group and a C₃₋₁₀ cyclic aliphatic hydrocarbon group.

The "C₁₋₁₀ chain aliphatic hydrocarbon group" means a linear or branched saturated or unsaturated aliphatic hydrocarbon group having the carbon number of 1 or more and 10 or less. An example of the monovalent C₁₋₁₀ chain aliphatic hydrocarbon group includes a C₁₋₁₀ alkyl group, a C₂₋₁₀ alkenyl group and a C₂₋₁₀ alkynyl group.

An example of the C₁₋₁₀ alkyl group includes methyl, ethyl, n-propyl, isopropyl, n-butyl, 1-methylpropyl, 2-methylpropyl, 1,1-dimethylethyl, 2,2-dimethylethyl, n-pentyl, n-hexyl, 2-hexyl, 3-hexyl, 4-methyl-2-pentyl, n-heptyl, n-octyl and n-decyl. The C₁₋₁₀ alkyl group is preferably a C₂₋₈ alkyl group and more preferably a C₄₋₆ alkyl group.

An example of the C₂₋₁₀ alkenyl group includes ethenyl (vinyl), 1-propenyl, 2-propenyl (allyl), butenyl, hexenyl, octenyl and decenyl. The C₂₋₁₀ alkenyl group is preferably a C₂₋₈ alkenyl group and more preferably a C₄₋₆ alkenyl group.

An example of the C₂₋₁₀ alkynyl group includes ethynyl, propynyl, butynyl, hexynyl, octynyl and pentadecynyl. The C₂₋₁₀ alkynyl group is preferably C₂₋₈ alkynyl group and more preferably C₂₋₆ alkynyl group.

The "C₃₋₁₀ cyclic aliphatic hydrocarbon group" means a cyclic saturated or unsaturated aliphatic hydrocarbon group having the carbon number of 1 or more and 10 or less. An example of the monovalent C₃₋₁₀ cyclic aliphatic hydrocarbon group includes a C₃₋₁₀ cycloalkyl group, a C₄₋₁₀ cycloalkenyl group and a C₄₋₁₀ cycloalkynyl group.

An example of the organic group constructed by binding 2 or more and 5 or less of the C₁₋₁₀ chain aliphatic hydrocarbon group and the C₃₋₁₀ cyclic aliphatic hydrocarbon group includes a C₃₋₁₀ monovalent cyclic aliphatic hydrocarbon group - C₁₋₁₀ divalent chain aliphatic hydrocarbon group, and a C₁₋₁₀ monovalent chain aliphatic hydrocarbon group - C₃₋₁₀ divalent cyclic aliphatic hydrocarbon group - C₁₋₁₀ divalent chain aliphatic hydrocarbon group.

The "C₆₋₁₂ aromatic hydrocarbon group" means an aromatic hydrocarbon group having the carbon number of 6 or more and 12 or less. An example of the monovalent C₆₋₁₂ aromatic hydrocarbon group includes phenyl, indenyl, naphthyl and biphenyl, and phenyl is preferred.

The "heteroaryl group" means a 5-membered aromatic heterocyclic group, a 6-membered aromatic heterocyclic group and a condensed ring aromatic heterocyclic group having at least one hetero atom such as a nitrogen atom, an oxygen atom and a sulfur atom. An example of the heteroaryl group includes a monovalent 5-membered aromatic heterocyclic group such as pyrrolyl, imidazolyl, pyrazolyl, thienyl, furyl, oxazolyl, isoxazolyl, thiazolyl, isothiazolyl and thiadiazolyl; a monovalent 6-membered aromatic heterocyclic group such as pyridinyl, pyrazinyl, pyrimidinyl and pyridazinyl; and a condensed ring aromatic heterocyclic group such as indolyl, isoindolyl, quinolinyl, isoquinolinyl, benzofuranyl, isobenzofuranyl and chromenyl.

An example of the "organic group constructed by binding 2 or more and 5 or less of the C₁₋₁₀ aliphatic hydrocarbon group and the C₆₋₁₂ aromatic hydrocarbon group" includes a C₆₋₁₂ aromatic hydrocarbon group - C₁₋₁₀ chain aliphatic hydrocarbon group, a C₁₋₁₀ chain aliphatic hydrocarbon group - C₆₋₁₂ aromatic hydrocarbon group, a C₁₋₁₀ chain aliphatic hydrocarbon group - C₆₋₁₂ aromatic hydrocarbon group - C₁₋₁₀ chain aliphatic hydrocarbon group, and a C₆₋₁₂ aromatic hydrocarbon group - C₁₋₁₀ chain aliphatic hydrocarbon group - C₆₋₁₂ aromatic hydrocarbon group. An example of the "organic group constructed by binding 2 or more and 5 or less of the C₁₋₁₀ aliphatic hydrocarbon group and the heteroaryl group" includes a heteroaryl group - C₁₋₁₀ chain aliphatic hydrocarbon group, a C₁₋₁₀ chain aliphatic hydrocarbon group - heteroaryl group, a C₁₋₁₀ chain aliphatic hydrocarbon group - heteroaryl group - C₁₋₁₀ chain aliphatic hydrocarbon group, and a heteroaryl group - C₁₋₁₀ chain aliphatic hydrocarbon group - heteroaryl group.

An example of the substituent group that the C₁₋₁₀ aliphatic hydrocarbon group may optionally have includes one or more substituent groups selected from the group consisting of a halogeno group, a nitro group and a cyano group, and a halogeno group is preferred. An example of the substituent group that the C₆₋₁₂ aromatic hydrocarbon group and the heteroaryl group may optionally have includes one or more substituent groups selected from the group consisting of a C₁₋₆ alkyl group, a C₁₋₆ alkoxy group, a halogeno group, a nitro group and a cyano group, and a halogeno group is preferred. An example of the halogeno group includes fluoro, chloro, bromo and iodo, and fluoro is preferred.

An alcohol compound may be classified into a fluorinated alcohol compound and a non-fluorinated alcohol compound. The fluorinated alcohol compound indispensably has a fluoro group as a substituent group, and the non-fluorinated alcohol compound is not substituted with a fluoro group. The halogeno group that a non-fluorinated alcohol compound may optionally have is one or more halogeno groups selected from chloro, bromo and iodo. The group "R^{x}" having a fluoro as substituent group may be described as "R_{F}^{x}".

The "C₁₋₆ alkyl group" means a linear or branched monovalent saturated aliphatic hydrocarbon group having the carbon number of 1 or more and 6 or less. An example of the C₁₋₆ alkyl group includes methyl, ethyl, n-propyl, isopropyl, n-butyl, isobutyl, s-butyl, t-butyl, n-pentyl and n-hexyl. The C₁₋₆ alkyl group is preferably a C₁₋₄ alkyl group, more preferably a C₁₋₂ alkyl group and even more preferably methyl.

The "C₁₋₆ alkoxy group" means a linear or branched monovalent saturated aliphatic hydrocarbon oxy group having the carbon number of 1 or more and 6 or less. An example of the C₁₋₆ alkoxy group includes methoxy, ethoxy, n-propoxy, isopropoxy, n-butoxy, isobutoxy, t-butoxy, n-pentoxy and n-hexoxy. The C₁₋₆ alkoxy group is preferably a C₁₋₄ alkoxy group, more preferably a C₁₋₂ alkoxy group and even more preferably methoxy.

The monovalent alcohol compound (I) may be a fluorinated alcohol compound. An example of the monovalent fluorinated alcohol compound (I) includes a fluorinated ethanol such as difluoroethanol and trifluoroethanol; a fluorinated propanol such as monofluoropropanol, difluoropropanol, trifluoropropanol, tetrafluoropropanol, pentafluoropropanol and hexafluoropropanol.

An example of the divalent organic group includes divalent organic groups derived from the examples of the monovalent organic group. For example, the divalent organic group derived from the C₁₋₁₀ alkyl group, the C₂₋₁₀ alkenyl group and the C₂₋₁₀ alkynyl group as the monovalent organic group is a C₁₋₁₀ alkane-diyl group, a C₂₋₁₀ alkene-diyl group and a C₂₋₁₀ alkyne-diyl group.

The divalent organic group may be a divalent (poly)alkylene glycol group: -[-O-R²-]ₙ- wherein R² is a C₁₋₆ alkane-diyl group, and n is an integer of 1 or more and 50 or less.

In addition, an example of the divalent alcohol compound (II) includes the following divalent alcohol compound (II-1): 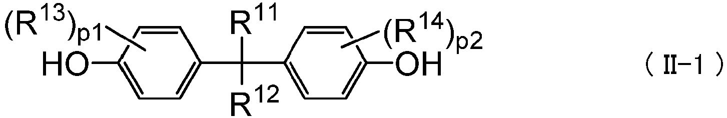 wherein
R¹¹ and R¹² are independently H, a C₁₋₆ alkyl group, a C₁₋₆ fluoroalkyl group or a C₆₋₁₂ aromatic hydrocarbon group, or form a C₃₋₆ cycloalkyl group optionally having a C₁₋₆ alkyl as a substituent together with each other,
R¹³ and R¹⁴ are independently H, a C₁₋₆ alkyl group or a C₆₋₁₂ aromatic hydrocarbon group, and when p1 or p2 is an integer of 2 or more, a plurality of R¹¹ or R¹² are the same as or different from each other,
p1 and p2 are independently integers of 0 or more and 4 or less.

An example of the specific divalent non-fluorinated alcohol compound (II-1) includes 2,2-bis(4-hydroxyphenyl)propane, 1,1-bis(4-hydroxyphenyl)-1-phenylethane, 2,2-bis(4-hydroxyphenyl)hexafluoropropane, 2,2-bis(4-hydroxyphenyl) butane, bis(4-hydroxyphenyl)diphenylmethane, 2,2-bis(3-methyl-4-hydroxyphenyl)propane, 1,1-bis(4-hydroxyphenyl)ethane, bis(4-hydroxyphenyl)methane and 2,2-bis(4-hydroxy-3-isopropylphenyl)propane, and 2,2-bis(4-hydroxyphenyl)propane, i.e. Bisphenol A, is preferred.

**The** divalent alcohol compound (II) may be a fluorinated alcohol compound. An example of such a divalent fluorinated alcohol compound (II) includes fluorinated ethylene glycol; a fluorinated propylene glycol such as monofluoropropylene glycol and difluoropropylene glycol; a fluorinated butanediol such as monofluorobutanediol, difluorobutanediol, trifluorobutanediol and tetrafluorobutanediol; a fluorinated pentanediol such as monofluoropentanediol, difluoropentanediol, trifluoropentanediol, tetrafluoropentanediol, pentafluoropentanediol and hexafluoropentanediol; a fluorinated hexanediol such as monofluorohexanediol, difluorohexanediol, trifluorohexanediol, tetrafluorohexanediol, pentafluorohexanediol, hexafluorohexanediol, heptafluorohexanediol and octafluorohexanediol; a fluorinated heptanediol such as monofluoroheptanediol, difluoroheptanediol, trifluoroheptanediol, tetrafluoroheptanediol, pentafluoroheptanediol, hexafluoroheptanediol, heptafluoroheptanediol, octafluoroheptanediol, nonafluoroheptanediol and decafluoroheptanediol; a fluorinated octanediol such as monofluorooctanediol, difluorooctanediol, trifluorooctanediol, tetrafluorooctanediol, pentafluorooctanediol, hexafluorooctanediol, heptafluorooctanediol, octafluorooctanediol, nonafluorooctanediol, decafluorooctanediol, undecafluorooctanediol and dodecafluorooctanediol; a fluorinated nonanediol such as monofluorononanediol, difluorononanediol, trifluorononanediol, tetrafluorononanediol, pentafluorononanediol, hexafluorononanediol, heptafluorononanediol, octafluorononanediol, nonafluorononanediol, decafluorononanediol, undecafluorononanediol, dodecafluorononanediol, tridecafluorononanediol and tetradecafluorononanediol; a fluorinated decanediol such as monofluorodecanediol, difluorodecanediol, trifluorodecanediol, tetrafluorodecanediol, pentafluorodecanediol, hexafluorodecanediol, heptafluorodecanediol, octafluorodecanediol, nonafluorodecanediol, decafluorodecanediol, undecafluorodecanediol, dodecafluorodecanediol, tridecafluorodecanediol, tetradecafluorodecanediol, pentadecafluorodecanediol and hexadecafluorodecanediol; a fluorinated polyethylene glycol such as fluorinated diethylene glycol, fluorinated triethylene glycol, fluorinated tetraethylene glycol, fluorinated pentaethylene glycol and fluorinated hexaethylene glycol.

The usage amount of the alcohol compound may be appropriately adjusted as long as the reaction successfully proceeds. For example, 1 or more molar ratio of the divalent alcohol compound to the produced carbonyl halide may be used, and 2 or more molar ratio of the monovalent alcohol compound may be used to the produced carbonyl halide. A carbonate compound can be produced by using excessive amount of the alcohol compound more efficiently. Since the yield of the carbonyl halide to the used halogenated methane is not constant, the molar ratio of the divalent alcohol compound to the halogenated methane is preferably adjusted to 1 or more and the molar ratio of the monovalent alcohol compound to the halogenated methane is preferably adjusted to 2 or more. The molar ratio of the divalent alcohol is preferably 1.5 or more, more preferably 2 or more, and preferably 10 or less, more preferably 5 or less. The molar ratio of the monovalent alcohol is preferably 2 or more, more preferably 4 or more, and preferably 20 or less, more preferably 10 or less.

A base may be used for accelerating the reaction of the carbonyl halide and the alcohol compound. A base is classified into an inorganic base and an organic base. An example of the inorganic base includes a carbonate salt of an alkali metal, such as lithium carbonate, sodium carbonate, potassium carbonate and cesium carbonate; a carbonate salt of a Group 2 metal, such as magnesium carbonate, calcium carbonate and barium carbonate; a hydrogencarbonate salt of an alkali metal, such as lithium hydrogencarbonate, sodium hydrogencarbonate, potassium hydrogencarbonate and cesium hydrogencarbonate; a hydroxide of an alkali metal, such as lithium hydroxide, sodium hydroxide and potassium hydroxide; a hydroxide of a Group 2 metal, such as magnesium hydroxide and calcium hydroxide; a fluoride salt of an alkali metal, such as lithium fluoride, sodium fluoride, potassium fluoride and cesium fluoride. A carbonate salt or a hydrogencarbonate salt of an alkali metal or a Group 2 metal is preferred due to low moisture absorbency and low deliquescency, and a carbonate salt of an alkali metal is more preferred. For example, a tri(C₁₋₄ alkyl)amine such as trimethylamine, triethylamine and diisopropylethylamine; a tert-butoxide of an alkali metal, such as sodium tert-butoxide and potassium tert-butoxide; a non-nucleophilic organic base such as diazabicycloundecene, lithium diisopropylamide, lithium tetramethylpiperidine, 1,4-diazabicyclo[2.2.2]octane (DABCO), 1,5,7-triazabicyclo[4.4.0]dec-5-ene (TBD), 7-methyl-1,5,7-triazabicyclo[4.4.0]dec-5-ene (MTBD), 1,8-diazabicyclo[5.4.0]undec-7-ene (DBU), 1,5-diazabicyclo[4.3.0]non-5-ene (DBN), 1,1,3,3-tetramethylguanidine (TMG) and N-methylmorpholine can be used as an organic base in terms of low reactivity with the product by the photoreaction of tetrahaloethylene. Also, low-nucleophilic organic base such as pyridine and lutidine may be used.

A hydrogen halide such as hydrogen chloride is produced as a by-product during an oxidative photodecomposition reaction of a halogenated methane and the reaction of a carbonyl halide and an alcohol compound. A base is useful for capturing such a hydrogen halide. But when a reaction tube having a small diameter, such as a coil reaction device demonstrated in Figure 2 and Figure 4, is used, a salt of a hydrogen halide and a base may precipitate and thus the reaction tube may become clogged in some cases. The base of which salt with a hydrogen halide is an ionic liquid is preferably used in such a case. An example of the base includes an organic base such as an imidazole derivative, for example, 1-methylimidazole. In addition, the base of which hydrochloride salt has a relatively low melting point, such as pyridine, may be used.

The usage amount of the base may be appropriately adjusted as long as the reaction successfully proceeds, and for example, the usage amount to 1 mol of the halogenated methane may be adjusted to 1 mol or more and 10 mol or less.

For example, the base may be added to the alcohol compound, or the base may be continuously supplied with the alcohol compound.

When the carbonyl halide and the alcohol compound are reacted, a solvent may be used. For example, a solvent may be added to the composition containing the alcohol compound. An example of the solvent includes a ketone solvent such as acetone, methyl ethyl ketone, methyl isobutyl ketone and cyclohexanone; an ester solvent such as ethyl acetate; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; a nitrile solvent such as acetonitrile; and a halogenated hydrocarbon solvent such as dichloromethane and chloroform.

The temperature for reacting the carbonyl halide and the alcohol compound is not particularly restricted and may be appropriately adjusted. For example, the temperature may be adjusted to 0°C or higher and 250°C or lower. The temperature is more preferably 10°C or higher, even more preferably 20°C or higher, and more preferably 200°C or lower or 150°C or lower, even more preferably 100°C or lower or 80°C or lower. When the base is not used or the base is used to further accelerate the reaction, the temperature may be adjusted to be relatively higher, for example, 50°C or higher or 100°C or higher.

The time to react the carbonyl halide and the alcohol compound is not particularly restricted and may be appropriately adjusted. For example, the time is preferably 0.5 hours or more and 50 hours or less. The reaction time is more preferably 1 hour or more, even more preferably 5 hours or more, and more preferably 30 hour or less, even more preferably 20 hours or less. For example, even after the production of the carbonyl halide is finished, the reaction mixture may be continuously stirred until the consumption of the alcohol compound is confirmed.

When the monovalent alcohol compound (I) is used, the chain carbonate compound represented by the following formula (III) is produced by the reaction of the carbonyl halide and the alcohol compound. When the divalent alcohol compound (II) is used, the polycarbonate compound comprising the unit represented by the following formula (IV-1) or the cyclic carbonate compound represented by the following formula (IV-2) is produced. When the divalent alcohol compound (II) is used, whether the polycarbonate compound (IV-1) is produced or the cyclic carbonate compound (IV-2) is produced and the production ratio thereof are mainly dependent on the distance between two hydroxy groups and the flexibility of the chemical structure of the divalent alcohol compound (II) and may be specifically determined by a preliminary experiment or the like.

R¹-O-C(=O)-O-R¹ (III)

[-A-R²-A-C(=O)-] (IV-1)

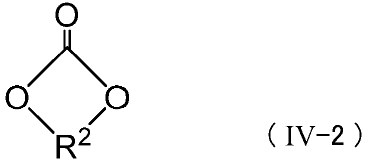

### 4. Post-reaction step - Production of halogenated formic acid ester

A halogenated formic acid ester can be produced by adjusting the molar ratio of the alcohol compound to the halogenated methane to less than 1 without using a base in the above-described method for producing a carbonate compound. The molar ratio is preferably 0.9 or less and more preferably 0.8 or less. The above-described monovalent alcohol compound (I) may be used as the alcohol compound. A halogenated formic acid fluorinated ester can be produced from the fluorinated monovalent alcohol compound (I), and a halogenated formic acid non-fluorinated ester can be produced from the non-fluorinated monovalent alcohol compound (I).

### 5. Post-reaction step - Production of isocyanate compound

An isocyanate compound can be produced by reacting the carbonyl halide and a primary amine compound. An isocyanate compound is useful as a raw material of a carbamate compound, a urethane compound or the like. A primary amine compound may be used in place of the alcohol compound in the above-described method for producing a carbonate compound except for the following points as the reaction embodiment.

A primary amine compound is not particularly restricted as long as the compound has 1 or more amino groups (-NH₂ groups). An example of the primary amine compound includes the primary amine compound (V): R³-(NH₂)ₘ wherein R³ is a m-valent organic group, and m is integer of 1 or more and 6 or less, preferably 5 or less, 4 or less or 3 or less, more preferably 1 or 2, and even more preferably 2.

An example of a monovalent organic group among the organic group R³ includes the same group as the monovalent organic group R¹ in the above-described method for producing a carbonate compound. An example of a divalent organic group includes the same group as the divalent organic group R². An example of a tri or more-valent organic group includes a tri or more-valent organic group derived from the examples of the monovalent organic group R¹. For example, a trivalent organic group derived from a C₁₋₁₀ alkyl group, a C₂₋₁₀ alkenyl group and a C₂₋₁₀ alkynyl group as a monovalent organic group is a C₁₋₁₀ alkane triyl group, a C₂₋₁₀ alkene triyl group and a C₂₋₁₀ alkyne triyl group.

The isocyanate compound (VI): R³-(N=C=O)ₘ can be produced by reacting the carbonyl halide and the primary amine compound (V). The produced R³-(N=C=O)ₘ may be possibly reacted with the primary amine compound (V) to produce a urea compound: R³-[NH-C(=O)-NH-R³]ₘ, and it is preferred to inhibit the reaction that the molar ratio of the primary amine compound (V) to the halogenated methane is adjusted to 1 or less, a salt is used as the primary amine compound (V), or a base is not used. In addition, an isocyanate compound can be efficiently produced by the following condition: the produced carbonyl halide is dissolved in a solvent to prepare a carbonyl halide solution, and the molar ratio of the carbonyl halide to the primary amine compound (V) is maintained at more than 1 by adding the primary amine compound (V) or a solution thereof to the carbonyl halide solution.

When the target compound is an isocyanate compound, the molar ratio of the primary amine compound (V) to the produced carbonyl halide is preferably adjusted to 1 or less. Since it may be difficult in some cases to predict the accurate amount of the produced carbonyl halide, the molar ratio of the primary amine compound (V) to the used halogenated methane is preferably adjusted to less than 1. The molar ratio is preferably 0.5 or less, more preferably 0.2 or less, and preferably 0.001 or more, more preferably 0.05 or more. When the target compound is a urea compound, the ratio is preferably 2 or more, more preferably 4 or more, and preferably 20 or less, more preferably 15 or less.

When the target compound is an isocyanate compound, a salt as the primary amine compound (V) is used, since an isocyanate compound is hardly reacted with an amine salt. An example such a salt includes an inorganic acid salt such as hydrochloride salt, hydrobromide salt, hydroiodide salt, sulfate salt, nitrate salt, perchlorate salt and phosphate salt; and an organic salt such as oxalate salt, malonate salt, maleate salt, fumarate, lactate salt, malate salt, citrate salt, tartrate salt, benzoate salt, trifluoroacetate salt, acetate salt, methanesulfonate salt, p-toluenesulfonate salt and trifluoromethanesulfonate salt.

The temperature for the reaction of the carbonyl halide and the primary amine compound is preferably adjusted to be lower than the temperature for the reaction with the alcohol compound in order to maintain the liquid state of the carbonyl halide. For example, the temperature is preferably adjusted to 15°C or lower, and is preferably 10°C or lower, more preferably 5°C or lower and even more preferably 2°C or lower. The lower limit of the temperature is not particularly restricted, and the temperature is preferably -80°C or higher and more preferably -20°C or higher or -15°C or higher.

When the target compound is an isocyanate compound and a base is used, the base is preferably 1 or more bases selected from a heterocyclic aromatic amine and a non-nucleophilic strong base. The heterocyclic aromatic amine means a compound that contains at least one hetero ring and at least one amine functional group other than -NH₂. An example of the heterocyclic aromatic amine includes pyridine and a derivative thereof, such as pyridine, α-picoline, β-picoline, γ-picoline, 2,3-lutidine, 2,4-lutidine, 2,6-lutidine, 3,5-lutidine, 2-chloropyridine, 3-chloropyridine, 4-chloropyridine, 2,4,6-trimethylpyridine and 4-dimethylaminopyridine.

The "non-nucleophilic organic base" means a base in which the nucleophilicity of the lone electron pair on the nitrogen atom is weak due to a steric hindrance but of which basicity is strong. An example of the non-nucleophilic organic base includes triethylamine, N,N-diisopropylethylamine, tripropylamine, triisopropylamine, tributylamine, tripentylamine, trihexylamine, triheptylamine, trioctylamine, tridecylamine, tridodecylamine, triphenylamine, tribenzylamine, N,N-diisopropylethylamine, 1,5,7-triazabicyclo[4.4.0]dec-5-ene (TBD), 7-methyl-1,5,7-triazabicyclo[4.4.0]dec-5-ene (MTBD), 1,8-diazabicyclo[5.4.0]undec-7-ene (DBU), 1,5-diazabicyclo[4.3.0]non-5-ene (DBN) and 1,1,3,3-tetramethylguanidine (TMG). In addition, a base of which basicity is relatively high may be used. For example, TBD (pK_{BH+}: 25.98), MTBD (pK_{BH+}: 25.44), DBU (pK_{BH+}: 24.33), DBN (pK_{BH+}: 23.89) and TMG (pK_{BH+}: 23.30) can be used as a base of which basicity (pK_{BH+}) in acetonitrile is 20 or more.

In addition, a versatile organic amine such as trimethylamine, dimethylethylamine, diethylmethylamine, N-ethyl-N-methylbutylamine and 1-methylpyrrolidine can be used as the base.

When the target compound is a urea compound, the molar ratio of the primary amine compound to the halogenated methane or the produced carbonyl halide is preferably adjusted to more than 1. The molar ratio is preferably 1.5 or more and more preferably 2 or more.

### 6. Post-reaction step - Production of NCA

An amino acid-N-carboxylic anhydride (VIII) (NCA) can be produced by using the amino acid compound (VII) in place of the alcohol compound in the above-described method for producing a carbonate compound. 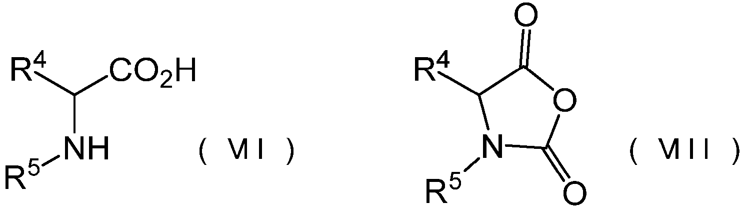 wherein
R⁴ is an amino acid side chain group wherein a reactive group is protected,
R⁵ is H or P¹-[-NH-CHR⁶-C(=O)-]_{I}- wherein R⁶ is an amino acid side chain group wherein a reactive group is protected, P¹ is a protective group of the amino group, I is an integer of 1 or more, and when I is an integer of 2 or more, a plurality of R⁶ may be the same as or different from each other.

### 7. Post-reaction step - Production of Vilsmeier reagent

A Vilsmeier reagent (X) can be produced by reacting the carbonyl halide and the amide compound (IX). A Vilsmeier reagent can be produced similarly to the above-described method for producing a carbonate compound except that the amide compound (IX) is used in place of the alcohol compound and a base is not used. 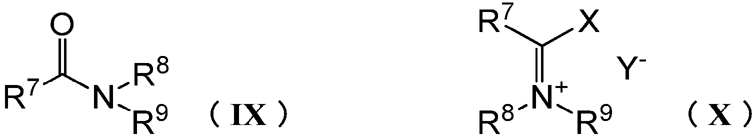 wherein
R⁷ is a hydrogen atom, a C₁₋₆ alkyl group or an optionally substituted C₆₋₁₂ aromatic hydrocarbon group,
R⁸ and R⁹ are independently a C₁₋₆ alkyl group or an optionally substituted C₆₋₁₂ aromatic hydrocarbon group, or R⁸ and R⁹ may form a 4 or more and 7 or less-membered ring structure together with each other,
X is a halogeno group selected from the group consisting of chloro, bromo and iodo,
Y⁻ is a counter anion.

The substituent group that the C₆₋₁₂ aromatic hydrocarbon group may optionally have is not particularly restricted as long as the substituent group does not inhibit the reaction of the present invention, and is exemplified by 1 or more substituent groups selected from the group consisting of a C₁₋₆ alkyl group, a C₁₋₆ alkoxy group, a halogeno group, a nitro group and a cyano group. The number of the substituent group is not particularly restricted as long as the C₆₋₁₂ aromatic hydrocarbon group can be substituted and may be 1 or more and 5 or less. The number is preferably 3 or less, more preferably 2 or less, and even more preferably 1. When the substituent group number is 2 or more, the substituent groups may be the same as or different from each other.

An example of the 4 or more and 7 or less-membered ring structure that is formed by R⁸, R⁹ and the nitrogen atom together with each other includes a pyrrolidyl group, a piperidyl group and a morpholino group.

An example of the specific amide compound (IX) includes N,N-dimethylformamide (DMF), N,N-dimethylacetamide (DMA), N-methyl-N-phenylformamide, N-methylpyrrolidone (NMP), 1,3-dimethylimidazolidinone (DMI), tetramethylurea, tetraethylurea and tetrabutylurea, and DMF is preferred in terms of versatility and cost.

The Y⁻ in the formula (X) is not particularly restricted and is exemplified by a chloride ion, a bromide ion and an iodide ion.

The usage amount of the amide compound may be appropriately adjusted as long as the reaction successfully proceeds. For example, the usage amount to 1 mL of the halogenated methane may be adjusted to 0.1 mol or more and 100 mol or less.

When the carbonyl halide and the amide compound are reacted, a solvent may be used. For example, a solvent is mixed in a composition containing the amide compound. An example of the solvent includes a ketone solvent such as acetone, methyl ethyl ketone, methyl isobutyl ketone and cyclohexanone; an ester solvent such as ethyl acetate; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; and a nitrile solvent such as acetonitrile.

The temperature for reacting the carbonyl halide and the amide compound is not particularly restricted and may be appropriately adjusted. For example, the temperature may be adjusted to 0°C or higher and 120°C or lower. The temperature is more preferably 10°C or higher, even more preferably 20°C or higher, and more preferably 100°C or lower, even more preferably 80°C or lower or 50°C or lower.

The time to react the carbonyl halide and the amide compound is not particularly restricted and may be appropriately adjusted. For example, the time is preferably 0.5 hours or more and 50 hours or less. The reaction time is more preferably 1 hour or more, even more preferably 5 hours or more, and more preferably 30 hour or less, even more preferably 20 hours or less. For example, even after the production of the carbonyl halide is finished, the reaction mixture may be continuously stirred until the consumption of the amide compound is confirmed.

The Vilsmeier-Haack reaction using a Vilsmeier reagent allows the formylation or ketonization of an aromatic compound having an active group. In addition, it has been known that the carboxy group of a carboxylic acid compound can be transformed to a haloformyl group by a Vilsmeier reagent. Furthermore, a formic acid ester can be produced by reacting a Vilsmeier reagent and a hydroxy group-containing compound.

An aromatic compound having an active group means an aromatic compound activated by a substituent group or the like. The aromatic compound is hereinafter described as an "active aromatic compound". For example, a hydroxy group and an amino group such as an alkylamino group substituted by an alkyl group strongly activate an aromatic compound. In addition, an alkylcarbonylamino group (-N(C=O)R), an alkylcarbonyloxy group (-O(C=O)R), an ether group (-OR), an alkyl group (-R) (R is an alkyl group and is preferably a C₁₋₆ alkyl group) and an aromatic group activate an aromatic compound. The substituent groups are hereinafter referred to as an activating group. In addition, a compound that is constructed by condensing aromatic rings and of which conjugated system is extended, such as anthracene, is also activated and can be subjected to formylation or ketonization by a Vilsmeier reagent. The pi electron at the activated part may be electrophilically reacted with a Vilsmeier reagent to be subjected to formylation or ketonization.

The active aromatic compound is not particularly restricted as long as the active aromatic compound is activated and can be subjected to formylation or ketonization by a Vilsmeier reagent. An example of the active aromatic compound includes a C₆₋₁₀ aromatic hydrocarbon, such as benzene and naphthalene, that may be substituted with the above-described activating group; a condensed aromatic hydrocarbon, such as phenanthrene and anthracene, that may be substituted with the above-described activating group; a 5-membered heteroaryl, such as pyrrole, imidazole, pyrazole, thiophen, furan, oxazole, isoxazole, thiazole, isothiazole and thiadiazole, that may be substituted with the above-described activating group; a 6-membered heteroaryl, such as pyridine, pyrazine, pyrimidine and pyridazine, that may be substituted with the above-described activating group; a condensed heteroaryl, such as indole, isoindole, quinoline, isoquinoline, benzofuran, isobenzofuran and chromene, that may be substituted with the above-described activating group. Though it has not been reported that unsubstituted furan and thiophen are subjected to formylation or ketonization by conventional Vilsmeier-Haack reaction, the formylation or ketonization at the carbon adjacent to a hetero element is possible by the present invention method.

An activating group-containing aromatic compound, a carboxylic acid compound and a hydroxy group-containing compound as a substrate compound of the above-described reaction may be added to the reaction mixture after the carbonyl halide-containing gas is blown into the composition containing the amide compound, or added to the reaction mixture before or while the carbonyl halide-containing gas is blown into the composition containing the amide compound.

The usage amount of the activating group-containing aromatic compound, the carboxylic acid compound and the hydroxy group-containing compound may be appropriately adjusted, and may be adjusted to, for example, 0.1 times or more by mole and 1.0 time or less by mole to the amide compound.

A Vilsmeier reagent is useful for producing a carboxylic acid halide from a carboxylic acid compound. A Vilsmeier reagent becomes an amide compound after the Vilsmeier reagent halogenates a carboxylic acid compound. An ester compound can be produced by reacting the produced carboxylic acid halide with an alcohol compound, and a carboxylic anhydride can be produced by reacting the produced carboxylic acid halide with a carboxylic acid. When a carboxylic acid compound and a base are used in place of the amide compound, the carboxylic acid compound may be anionized by the base and then the anionized carboxylate compound may be directly transformed to a carboxylic acid halide by the carbonyl halide. Such a carboxylic acid halide can be also used for producing an ester compound and a carboxylic anhydride.

### 8. Post-treatment step

Since many carbonyl halides are toxic, the produced carbonyl halide is not preferably leaked out of the reaction system. For example, it is preferred that the gas phase discharged from the reaction vessel for the reaction of the produced carbonyl halide is supplied into an alcohol trap, and the gas phase discharged from the alcohol trap is further supplied into an alkali trap as demonstrated in Figures 1 to 6. The alcohol trap may be cooled as long as the alcohol to be used is not coagulated. For example, the alcohol trap may be cooled to -80°C or higher and 50°C or lower. In addition, for example, a sodium hydroxide aqueous solution and a saturated sodium hydrogencarbonate aqueous solution may be used for the alkali trap.

When the compound produced from the carbonyl halide is relatively unstable compound such as an isocyanate compound, further a reactive substrate compound may be added to the reaction mixture after the carbonyl halide is reacted. When the compound produced from the carbonyl halide is relatively stable compound such as a carbonate compound, the target compound may be purified from the reaction mixture. For example, water and a water-insoluble organic solvent such as chloroform are added to the reaction mixture, the aqueous phase and the organic phase are separated, the organic phase is dried over anhydrous sodium sulfate or anhydrous magnesium sulfate and then concentrated under reduced pressure, and the target compound may be purified by chromatography or the like.

The present application claims the benefit of the priority date of Japanese patent application No. 2021-21001 filed on February 12, 2021.

### EXAMPLES

Hereinafter, the examples are described to demonstrate the present invention more specifically, but the present invention is in no way restricted by the examples, and the examples can be appropriately modified to be carried out within a range that adapts to the contents of this specification. Such a modified example is also included in the range of the present invention.

### Example 1: Production of phosgene

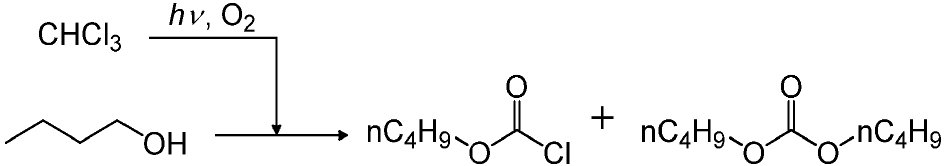

A gas phase photoreaction was conducted using the photoreaction system schematically demonstrated in Figure 1. Specifically, a low pressure mercury lamp ("SUV40D" manufactured by SEN Light, 40 W, φ22.3×380 mm, wavelength: 185-600 nm, peak wavelength: 184.9 nm and 253.7 nm) was inserted in a quartz glass jacket having a diameter of 30 mm × a length of 320 mm, and 12 quartz tubes having an inner diameter of 2.1 mm, a length of 320 mm and a volume of 1.033 mL were placed around the quartz glass jacket to prepare the cylindrical flow photoreaction device 4. The total volume containing the joint parts of the cylindrical flow photoreaction device 4 was 13.3 mL. The illumination intensity of the light having the wavelength of 185 nm at the position 5 mm away from the center of the low pressure mercury lamp was 3.93 mW/cm², and the illumination intensity of the light having the wavelength of 254 nm was 11.02 mW/cm².

Liquid chloroform was supplied into a PTFE tube having the inner diameter of 1 mm at the flow amount shown in Table 1 using the syringe pump 1 and vaporized using a heater heated to the temperature shown in Table 1. The vaporized chloroform was mixed with oxygen gas of which flow amount was adjusted by the mass flow controller 2, and the mixed gas was supplied to the flow photoreaction device 4. The pressure inside of the flow photoreaction device was adjusted using the back pressure regulator 5 attached to the outlet of the flow photoreaction device. The injected chloroform was vaporized and was mixed with oxygen gas, and the mixed gas was flowed at the heater temperature. Each of the maximum gas flow rate of the vaporized chloroform and the oxygen gas, and the linear velocity and the residence time of the mixed gas were calculated as Table 1.

The gas produced by the oxidative photodecomposition of the mixed gas of chloroform gas and oxygen was blown into a sufficient amount of stirred 1-butanol in the connected reaction vessel 6 at the atmospheric temperature for 2 to 6 hours. The unreacted gas was trapped by 1-butanol in the connected trap vessel 7, and the further unreacted gas was treated by supplying into a connected alkali trap so that a toxic gas was not leaked outside.

In addition, a similar experiment was conducted except that an air pump ("Nisso air pump silent β-60" manufactured by Marukan) was used and the air dried by a desiccant ("Silica Gel, Medium Granular, Blue" manufactured by FUJIFILM Wako Pure Chemical) was used in place of oxygen.

After the reaction, the reaction mixtures in the reaction vessel 6 and the trap vessel 7 were analyzed by ¹H NMR to measure the conversion ratios to the produced chloroformic acid ester and carbonate, and the amount of the produced phosgene was calculated from the total conversion ratios. The result is shown in Table 1. The phosgene yield is the yield to the used chloroform in Table 1.

**Table 1**

| Injected CHCl₃ | | Heater temp. | Max gas flow rate | | Mixed gas | | Total phosgene amount | Phosgene yield | Unreacted CHCl₃ |
|---|---|---|---|---|---|---|---|---|---|
| Amount | Flow rate | | CHCl₃ | O₂ | Linear velocity | Residence time | | | |
| 3.39g (28.4mmol) | 0.02 mL/min | 80°C | 7.2 mL/min | 6.0 mL/min | 3.8 m/min | 60s | 22.2 mmol | 78% | * |
| 11.06g (92.7mmol) | 0.02 mL/min | 90°C | 7.4 mL/min | 12.4 mL/min | 5.8 m/min | 40s | 85.2 mmol | 92% | 5.7% |
| 3.37g (28.2mmol) | 0.02 mL/min | 170°C | 9.0 mL/min | 5.0 mL/min | 4.0 m/min | 57s | 27.2 mmol | 96% | ND |

| Injected CHCl₃ | | Heater temp. | Max gas flow rate | | Mixed gas | | Total phosgene amount | Phosgene yield | Unreacted CHCl₃ |
|---|---|---|---|---|---|---|---|---|---|
| Amount | Flow rate | | CHCl₃ | Air | Linear velocity | Residence time | | | |
| 1.90g (15.9mmol) | 0.0072 mL/min | 80°C | 2.2 mL/min | 6.0 mL/min | 2.4 m/min | 97s | 15.3 mmol | 97% | ND |
| 2.23g (18.7mmol) | 0.0084 mL/min | 80°C | 2.6 mL/min | 7.0 mL/min | 2.8 m/min | 83s | 18.3 mmol | 98% | ND |
| 3.21g (26.9mmol) | 0.012 mL/min | 80°C | 3.6 mL/min | 10.0 mL/min | 3.9 m/min | 59s | 24.5 mmol | 91% | 10% |

| | | | | | | | | | |
|---|---|---|---|---|---|---|---|---|---|
| *:Unmeasured, ND:Not detected | | | | | | | | | |

A gas phase flow photoreaction was conducted by heating chloroform at 80°C using a coil heater for vaporization and mixing the vaporized chloroform with oxygen gas; as a result, phosgene could be produced from chloroform with the conversion ratio of 78% as the above-described result.

The temperature of the coil heater was further increased to 90°C and the flow amount of oxygen was increased; as a result, phosgene could be produced from chloroform with the conversion ratio of 92% and unreacted chloroform of 5.7%. The remaining about 2% may be hexachloroethane produced by the photodecomposition of chloroform as a side product and photodecomposed products of phosgene, such as CO₂, CO and Cl₂.

When the temperature of the coil heater was further increased to 170°C, the yield was increased.

When dried air was used in place of oxygen, phosgene could be produced with high yield but the yield amount was decreased in comparison with the case of using oxygen. When the amount of the injected air was increased, unreacted chloroform was somewhat increased.

### Example 2: Experiment of gas flow amount

It was examined whether a phosgene yield was changed or not by a light irradiation time similarly to Example 1 except that the gas flow rates of chloroform and oxygen were changed and thus the residence time in the flow photoreaction device was changed. The result is shown in Table 2.

**Table 2**

| Injected CHCl₃ | | Heater temp. | Max gas flow rate | | Mixed gas | | Total phosgene amount | Phosgene yield | Unreacted CHCl₃ |
|---|---|---|---|---|---|---|---|---|---|
| Amount | Flow rate | | CHCl₃ | O₂ | Linear velocity | Residence time | | | |
| 11.06g (92.65mmol) | 0.02 mL/min | | 7.4 mL/min | 12.4 mL/min | 5.8 m/min | 40s | 85.21 mmol | 92% | 5.7% |
| 5.94g (49.8mmol) | 0.01 mL/min | 90°C | 3.7 mL/min | 6.2 mL/min | 2.9 m/min | 81s | 41.4 mmol | 83% | 0.6% |
| 3.04g (25.5mmol) | 0.005 mL/min | | 1.8 mL/min | 3.1 mL/min | 1.4 m/min | 163s | 20.52 mmol | 80% | 0% |

It can be found that the amount of unreacted chloroform is decreased and the amount of produced phosgene is decreased with the increase of the residence time in the flow photoreaction device as the above-described result. The produced phosgene may be decomposed but is not dramatically decreased and the above-described phosgene yield is sufficiently practicable. Thus, the present invention system may have a function that inhibits the photodecomposition of the produced phosgene. For example, since oxygen gas has a light absorption band in 100 to 250 nm, excessive high energy light may be absorbed by oxygen and thus the decomposition of the produced phosgene may be inhibited.

### Example 3: Synthesis of isocyanate by light flow system

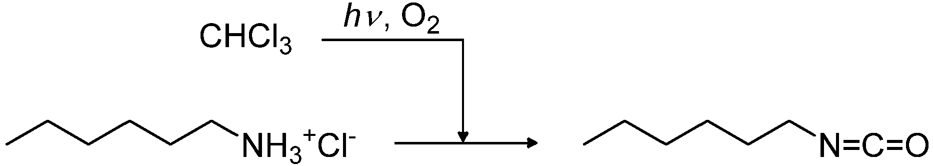

A solution (20 mL) of hexylamine hydrochloride (1.38 g, 10 mmol) in 1,1,2,2-tetrachloroethane was used in place of 1-butanol in the condition of Example 1 in which 11.06 g (92.7 mmol) of chloroform was used and the temperature of the coil heater was adjusted to 90°C, the solution was heated to 100°C and stirred, and the gas produced in the flow photoreaction device was blown into the solution. Dichloromethane was added to the reaction mixture as an internal standard material after the reaction, and the mixture was analyzed by ¹H NMR; as a result, it was confirmed that hexyl isocyanate was produced as the target compound with the yield of 83%.

### Example 4: Synthesis of isocyanate by light flow system

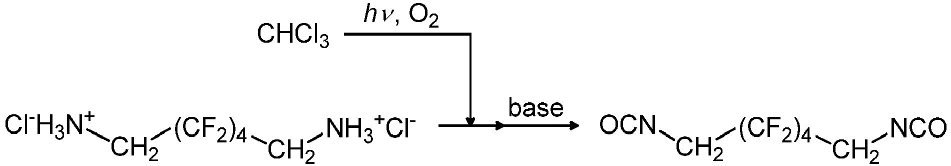

The gas produced by the flow photoreaction device of the photoreaction system schematically demonstrated in Figure 1 in the conditions shown in Table 3 was blown into the chloroform suspension (30 mL) of 2,2,3,3,4,4,5,5-octafluorohexane-1,6-diamine dihydrochloride (0.34 g, 1.0 mmol) for 3.5 hours, and then the mixture was stirred at 0°C. Into the reaction mixture, 2,6-lutidine (0.58 mL, 5 mmol) was injected for 1 hour using a syringe pump. The mixture was heated to 50°C and stirred for 1 hour.

The reaction mixture was analyzed by ¹H NMR after the reaction; as a result, it was confirmed that the target compound was produced with the yield of >99% to the raw material diamine compound.

**Table 3**

| CHCl₃ | Max gas flow rate | | Mixed gas | | Photoreaction temp | Base | Isocyanate yield |
|---|---|---|---|---|---|---|---|
| | CHCl₃ | O₂ | Linear velocity | Residence time | | | |
| 6.21g (52.0mmol) | 7.4 mL/min (0.25 mmol/min) | 12.4 mL/min (0.45 mmol/min) | 5.8 m/min | 40s | 90°C | Lutidine 5mmol | >99% |

### Example 5: Synthesis of carbonate by light flow system

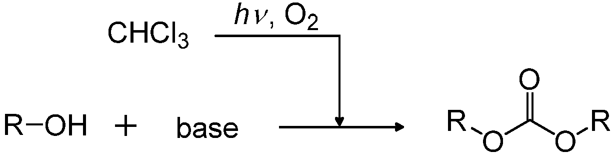

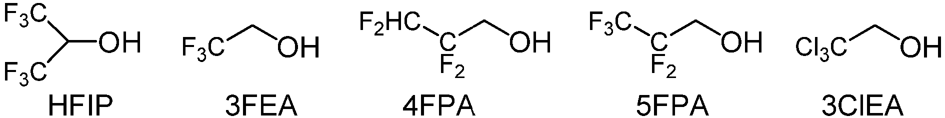

The gas produced by the flow photoreaction device of the photoreaction system schematically demonstrated in Figure 1 in the conditions shown in Table 4 was blown into the stirred alcohol containing pyridine or 1-methylimidazole (NMI) as a base in the reaction vessel 6 at an atmospheric temperature. In the trap vessel 7, 1-butanol was added to trap the gas leaked from the reaction vessel 6.

After the reaction, 1,1,2,2-tetrachloroethane was added to the reaction mixture as an internal standard material, and the mixture was analyzed by ¹H NMR; as a result, it was confirmed that the carbonates were produced as the target compound with high yields.

On the one hand, the phosgene yield calculated from the carbonate produced in the reaction vessel 6 and the chloroformic acid ester and the carbonate produced in the trap vessel 7 was relatively low in some cases. Pyridine may possibly cause the decomposition of the phosgene.

**Table 4**

| Injected CHCl₃ | | Heater temp. | Max gas flow rate | | Mixed gas | | R-OH | Base | Phosgene yield^{a} | Carbonate yield |
|---|---|---|---|---|---|---|---|---|---|---|
| Amount | Flow rate | | CHCl₃ | O₂ | Linear velocity | Residence time | | | | |
| 11.05g (92.6mmol) | | | | | | | PhOH 400mmol | Pyridine 300mol | 97% | 73%^{a} |
| 10.94g (91.6mmol) | | | | | | | HFIP 400mmol | Pyridine 300mol | 68% | 65%^{a} |
| 6.11g (51.2mmol) | | | | | | | HFIP 75mmol | NMI 188mmol | 74% | 70%^{a} |
| 10.35g (86.7mmol) | | | | | | | 3FEA 400mmol | Pyridine 300mmol | 94% | 94%^{a} |
| 10.8g (90.5mmol) | 0.02 mL/min | 90°C | 7.4 mL/mi n | 12.4 mL/mi n | 5.8 m/min | | 4FPA 400mmol | Pyridine 300mmol | 85% | 85%^{a} |
| 10.72g (89.8mmol) | | | | | | 40s | t-BuOH 400mmol | Pyridine 200mmol | 66% | 66%^{a} |
| 11.10g (92.2mmol) | | | | | | | i-PrOH 400mmol | Pyridine 300mmol | 79% | 79%^{a} |
| 8.97g (75.14mmol) | | | | | | | 5FPA 66.7mmol | Pyridine 200mmol | >54% | >99%^{b} |
| 5.88g (49.2mmol) | | | | | | | 3ClEA 74.9mmol | Pyridine 200mmol | >76% | >99%^{b} |
| 6.27g (52.5mmol) | | | | | | | MeOH 50mmol | Pyridine 175mmol | >48% | >99%^{b} |
| 4.63g (38.8mmol) | | | | | | | EtOH 50mmol | Pyridine 175mmol | >64% | >99%^{b} |

| | | | | | | | | | | |
|---|---|---|---|---|---|---|---|---|---|---|
| a:yield to the injected CHCl₃, b:yield to R-OH | | | | | | | | | | |

### Example 6: Synthesis of polycarbonate by light flow system (1) Homogeneous polymerization

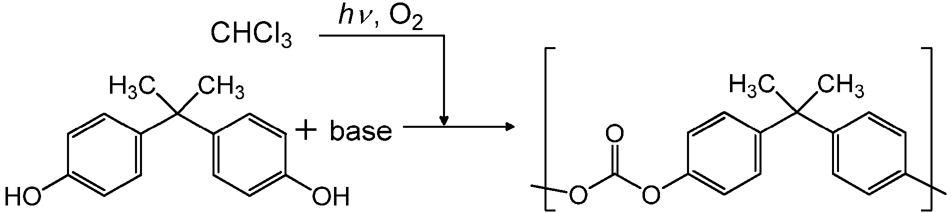

The mixed gas of vaporized chloroform and oxygen was supplied into the flow photoreaction device for photoreaction at 90°C in the conditions shown in Table 5 using the photoreaction system schematically demonstrated in Figure 1. The gas produced in the flow photoreaction device was blown into the stirred solution prepared by mixing Bisphenol A, pyridine (4 mL, 50 mmol) and chloroform in the reaction vessel 6. Then, the reaction mixture was further stirred at 50°C for 1 hour.

Next, methanol (150 mL) was added to the reaction mixture, and the produced precipitate was obtained by filtration and dried in vacuo to obtain the white target compound.

**Table 5**

| Entry | CHCl₃ | Flow amount/min | | Mixed gas | | | Reaction vessel 6 | | | PC |
|---|---|---|---|---|---|---|---|---|---|---|
| | | CHCl₃ | O₂ | Linear velocity | Residence time | Temp. | BPA | Pyridine | Temp. | Amount Yield |
| 1 | 93 mmol | 7.4mL (0.25mmo) | 12.4mL (0.45mmol) | 5.8 m/min | 40s | 90°C | 80 mmol | 400 mmol | 20°C | 73mmol |
| | | | | | | | | | | 91% vs BPA |
| | | | | | | | | | | 79% vs CHCl₃ |
| 2 | 15 mmol | 3.7mL (0.125mmol) | 12.4mL (0.45mmol) | 4.6 m/min | 50s | 90°C | 10 mmol | 50 mmol | 30°C | 8.8mmol |
| | | | | | | | | | | 88% vs BPA |
| | | | | | | | | | | 59% vs CHCl₃ |

The produced polycarbonate was analyzed by gel permeation chromatography (GPC) to determine the molecular weight. The result is shown in Table 6.

**Table 6**

| Entry | Mw | Mn | Mw/Mn |
|---|---|---|---|
| 1 | 4,505 | 2,360 | 1.91 |
| 2 | 49,000 | 14,000 | 3.50 |

### (2) Homogeneous polymerization

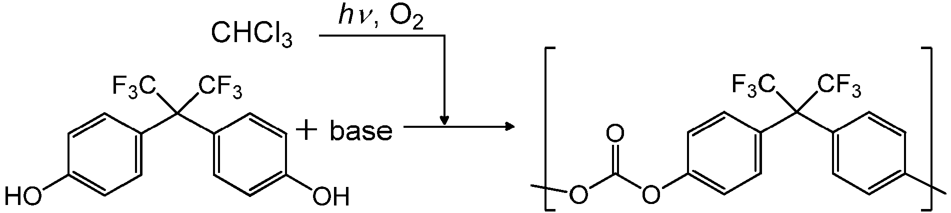

The mixed gas of vaporized chloroform and oxygen was supplied into the flow photoreaction device for photoreaction at 90°C in the conditions shown in Table 7 using the photoreaction system schematically demonstrated in Figure 1. The gas produced in the flow photoreaction device was blown into the stirred solution prepared by mixing Bisphenol AF (BPAF), pyridine and chloroform in the reaction vessel 6. Then, the reaction mixture was further stirred at 50°C for 1 hour.

Next, methanol (150 mL) was added to the reaction mixture, and the produced precipitate was obtained by filtration and dried in vacuo to obtain the white target compound.

**Table 7**

| CHCl₃ | Flow amount/min | | Mixed gas | | | Reaction vessel 6 | | | PC Amount Yield |
|---|---|---|---|---|---|---|---|---|---|
| | CHCl₃ | O₂ | Linear velocity | Residence time | Temp. | BPAF | Pyridine | Temp. | |
| 26 mmol | 7.4mL (0.25mmo) | 12.4mL (0.45mmol) | 5.8 m/min | 40s | 90°C | 10 mmol | 50 mmol | 30°C | 9. 1mmol |
| | | | | | | | | | 91% vs BPAF |
| | | | | | | | | | 35% vs CHCl₃ |

The produced polycarbonate was analyzed by gel permeation chromatography (GPC) to determine the molecular weight. The result is shown in Table 8.

**Table 8**

| Entry | Mw | Mn | Mw/Mn |
|---|---|---|---|
| 3 | 274,000 | 174,000 | 1.57 |

### (3) Interfacial polymerization

The mixed gas of vaporized chloroform and oxygen was supplied into the flow photoreaction device for photoreaction at 90°C in the conditions shown in Table 9 using the photoreaction system schematically demonstrated in Figure 1. The gas produced in the flow photoreaction device was blown into the stirred solution prepared by mixing Bisphenol A, 17 wt% sodium hydroxide aqueous solution and dichloromethane in the reaction vessel 6.

Then, water and dichloromethane were added to the reaction mixture, and the upper layer and the lower layer were separated. The lower layer was dried over anhydrous sodium sulfate, the mixture was filtrated, and the filtrate was concentrated under reduced pressure. Methanol was added to the residue, and the produced precipitate was obtained by filtration and dried in vacuo to obtain the yellowish white target compound (yield to chloroform: 19%, yield to BPA: 84%).

**Table 9**

| CHCl₃ | Flow amount/min | | Mixed gas | | | Reaction vessel 6 | | | | PC Amount Yield |
|---|---|---|---|---|---|---|---|---|---|---|
| | CHCl₃ | O₂ | Linear velocity | Residence time | Temp. | BPA | CH₂Cl₂ | NaOH aq. | Temp. | |
| 91 mmol | 7.4mL (0.25mmo) | 12.4mL (0.45mmol) | 5.8 m/min, | 40s | 90°C | 20 mmol | 40 mL | 40mL (17wt%) | 20°C | 17mmol |
| | | | | | | | | | | 84% vs BPA |
| | | | | | | | | | | 19% vs CHCl₃ |

The produced polycarbonate was analyzed by gel permeation chromatography (GPC) to determine the molecular weight. The result is shown in Table 10.

**Table 10**

| Mw | Mn | Mw /Mn |
|---|---|---|
| 16, 800 | 4,400 | 3.77 |

### Example 7: Synthesis of polycarbonate by light flow system

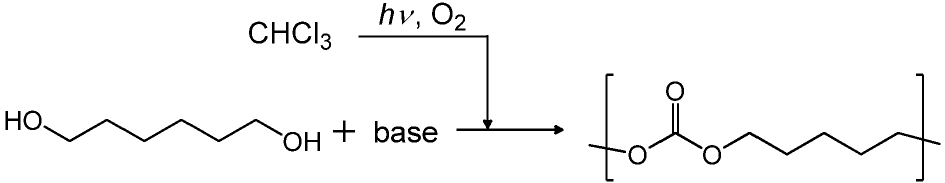

The mixed gas of vaporized chloroform and oxygen was supplied into the flow photoreaction device for photoreaction at 90°C in the conditions shown in Table 11 using the photoreaction system schematically demonstrated in Figure 1. The gas produced in the flow photoreaction device was blown into the stirred solution prepared by mixing 1,6-hexanediol (9.45 g, 80 mmol), pyridine (32 mL, 400 mmol) and dichloromethane (40 mL) in the reaction vessel 6.

Then, 10 wt% hydrochloric acid was added to the reaction mixture, and the upper layer and the lower layer were separated. The lower layer was dried over anhydrous sodium sulfate, and the mixture was filtrated. The solvent was distilled away from the filtrate under reduced pressure, and the residue was dried in vacuo at 50°C for 2 hours to obtain the white target solid (yield to chloroform: 58%, yield to the raw material diol: 73%).

**Table 11**

| CHCl₃ | Flow amount/min | | Mixed gas | | | Reaction vessel 6 | | | PC Amount Yield |
|---|---|---|---|---|---|---|---|---|---|
| | CHCl₃ | O₂ | Linear velocity | Residence time | Temp. | Diol | Pyridine | Temp. | |
| 94 mmol | 7.4mL (0.25mmo) | 12. 4mL (0.45mmol) | 5.8 m/min | 40s | 90°C | 80 mmol | 400 mmol | 20°C | 58mmol 73% vs diol 58% vs CHCl₃ |

The produced polycarbonate was analyzed by gel permeation chromatography (GPC) to determine the molecular weight. The result is shown in Table 12.

**Table 12**

| Mw | Mn | Mw/Mn |
|---|---|---|
| 5,740 | 3,530 | 1.62 |

### Example 8: Synthesis of urea compound by light flow system

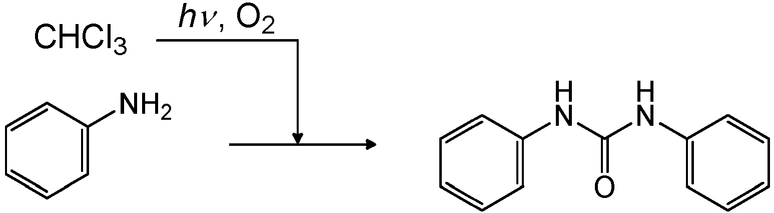

The gas produced by the flow photoreaction device using 11.46 g (94.3 mmol) of chloroform was blown into the stirred solution prepared by mixing aniline (42.3 g, 460 mmol) and dichloromethane (50 mL) in place of 1-butanol at an ordinary temperature in the reaction vessel 6 in the condition of Example 1 of which coil heater temperature was 90°C.

Then, water was added to the reaction mixture, and the produced precipitate was obtained by filtration. The precipitate was washed and then dried in vacuo at 50°C for 3 hours to obtain the white target solid (yield to chloroform: 93%). The urea compound may be produced by reacting phosgene and aniline to produce an isocyanate compound, which reacted with aniline.

The phosgene used for producing diphenylurea and the unreacted phosgene trapped by the alcohol trap (the trap vessel 7) were combined to calculate the conversion ratio from chloroform to phosgene as 98%.

### Example 9: Synthesis of amino acid N-carboxylic anhydride by light flow system (1) L-Phenylalanine N-carboxylic anhydride

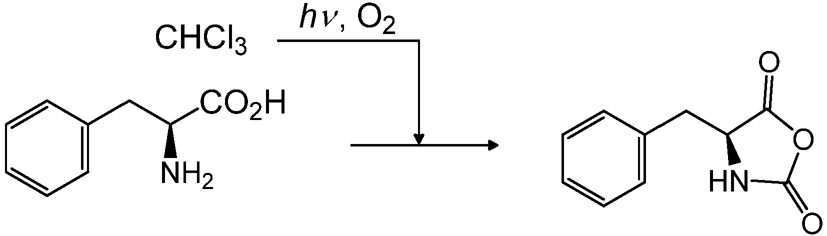

The gas produced by the flow photoreaction device using 5.7 g (47.7 mmol) of chloroform was blown into the stirred suspension prepared by mixing L-phenylalanine (1.65 g, 10 mmol), chloroform (20 mL) and acetonitrile (15 mL) in place of 1-butanol at 70°C in the reaction vessel 6 in the condition of Example 1 of which coil heater temperature was 90°C.

Then, the unreacted raw material was separated by filtration, and the filtrate was concentrated under reduced pressure to obtain the target compound (yield to raw material L-phenylalanine: 61%).

### (2) Synthesis of amino acid N-carboxylic anhydride using air

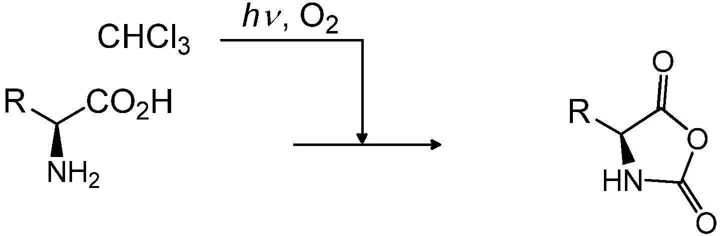

A gas phase photoreaction was conducted using the photoreaction system schematically demonstrated in Figure 1. Liquid chloroform was supplied to the PTFE tube having an inner diameter of 1 mm at the flow amount of 838 µL/min (0.1 mmol/min) and mixed with the air of which flow amount was adjusted to 7 mL/min using an air pump and a mass flow controller, and the mixed gas was supplied to the flow photoreaction device at 80°C. The injected chloroform was vaporized and mixed with air, and the mixed gas was flowed. The respective maximum gas flow rate of the vaporized chloroform and the oxygen gas, and the linear velocity and the residence time of the mixed gas were calculated as Table 13.

The gas produced by the oxidative photodecomposition of the mixed gas of the chloroform gas and the air was blown into the stirred THF solution containing L-amino acid (10 mmol) in the connected reaction vessel 6 at 60°C for 3 hours. The unreacted gas was treated by supplying to the further connected alkali trap so that toxic gas was not leaked outside. Then, the obtained solution was washed with water and subjected to extraction using dichloromethane. The organic layer was dried over anhydrous sodium sulfate, and the solvent was distilled away under reduced pressure. The white solid target compound was obtained by recrystallization from the residue using diethyl ether and hexane. The result is shown in Table 13.

**Table 13**

| Entry | CHCl₃ | Flow amount/min | | Mixed gas | | | Reaction vessel 6 | | | Yield* |
|---|---|---|---|---|---|---|---|---|---|---|
| | | CHCl₃ | Air | Linear velocity | Residence time | Temp. | AA | Solvent | Temp. | |
| 1 | 19 mmol | 8.38µL | 7.0mL | 2.74 m/min | 84s | 80°C | Phe | THF 30mL | 60°C | 73% |
| 2 | | | | | | | Leu | | | 66% |

| | | | | | | | | | | |
|---|---|---|---|---|---|---|---|---|---|---|
| *:isolated yield to raw material amino acid | | | | | | | | | | |

### Example 10: Synthesis of carbonyldiimidazole by light flow system

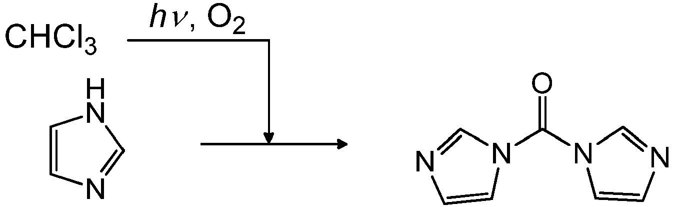

Chloroform (2.4 mL, 30 mmol) was used and the gas produced in the flow photoreaction device was blown into the stirred THF solution of imidazole (5.11 g, 75 mmol) in place of 1-butanol at 0°C in the condition of Example 1 in which the temperature of the coil heater was adjusted to 90°C.

Then, the imidazole hydrochloride salt precipitate produced as the by-product with proceeding the reaction was separated by filtration, and the filtrate was concentrated under reduced pressure to obtain the target compound (yield to chloroform: 74%).

### Example 11: Synthesis of polycarbonate by light flow system

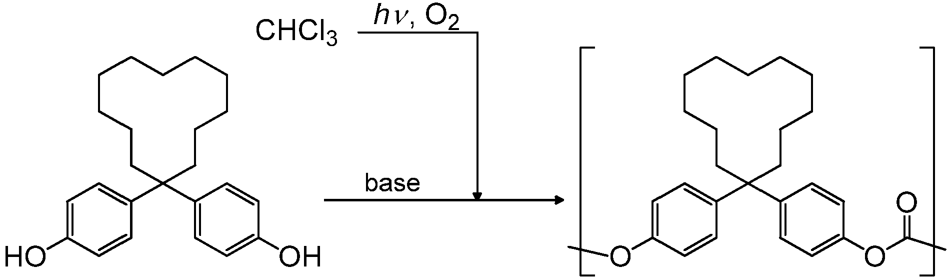

Chloroform (3.14 g, 26.3 mmol) was used and the gas produced in the flow photoreaction device was blown into the stirred solution prepared by mixing 4,4'-cyclododecylidene bisphenol (BisP-CDE) (3.52 g, 10 mmol), pyridine (4 mL, 50 mmol) and chloroform (40 mL) in place of 1-butanol at 30°C for 125 minutes in the condition of Example 1 in which the temperature of the coil heater was adjusted to 90°C.

Then, methanol (150 mL) was added to the reaction mixture, and the produced precipitate was obtained by filtration and dried in vacuo to obtain the white polycarbonate (yield to BisP-CDE: >99%).

The produced polycarbonate was analyzed by gel permeation chromatography (GPC) to determine the molecular weight. The result is shown in Table 14.

**Table 14**

| Mw | Mn | Mw /Mn |
|---|---|---|
| 269,000 | 184,000 | 1.45 |

### Example 12: Continuous flow synthesis by light flow system

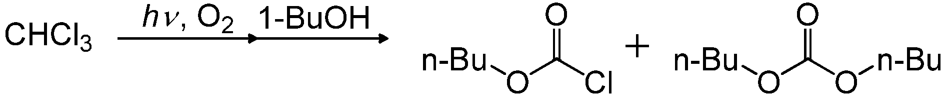

The syringe pump 8 for injecting a reactive substrate and the temperature-adjustable coil reactor 9 having the inner diameter of 1.0 mm, the length of 2830 mm and the volume of 2.22 mL were further connected to the flow photoreaction system of Figure 1 for the continuous reaction as demonstrated in Figure 2.

Into the phosgene gas produced in the flow photoreaction system, 1-butanol was further injected from the syringe pump 8 for injecting a reactive substrate at the flow amount of 0.052 mL/min to be reacted in the coil reactor 9 of which temperature was adjusted to 0 to 100°C in the conditions shown in Table 12. A two-neck flask was cooled to 0°C and attached to the outlet of the coil reactor as the recovery vessel 10 to obtain the product. The unreacted decomposed gas was trapped in the further attached alcohol trap as the trap vessel 7, and the waste gas was treated in an alkali trap in order not to be leaked outside.

The yields of the chloroformic acid ester and the carbonate produced in the recovery vessel 10 and the trap vessel 7 were calculated by ¹H NMR spectra by comparing 50 mmol of 1,1,2,2-tetrachloroethane as an internal standard material, and the amount of the produced phosgene was estimated on the basis of the total amounts thereof. The result is shown in Table 15.

**Table 15**

| Injected CHCl₃ | | Heater temp. | Max gas flow rate | | Mixed gas | | 1-BuOH | | Reaction coil temp. | vs CHCl₃ | | |
|---|---|---|---|---|---|---|---|---|---|---|---|---|
| Amount | Flow rate | | CHCl₃ | O₂ | Linear velocity | Residence time | Injection amount | Injection rate | | Phosgene yield | Unreacted | Coil yield |
| 10.8g (90.5mmol) | 0.02 mL/min | 90°C | 7.4 mL/min | 12.4 mL/min | 5.8 m/min | 40s | 90.5 mmol | 0.052 mL/min | 0°C | 91% | 5.7% | 89% |
| 11.39g (95. 4mmol) | | | | | | | 95.4 mmo1 | | 30°C | 88% | 7.5% | 87% |
| 10.96g (91.8mmol) | | | | | | | 91.8 mmol | | 100°C | 89% | 9.0% | 89% |

When 1-butanol as an aliphatic alcohol was injected from the syringe pump 8 for injecting a reactive substrate, the chloroformic acid ester and the carbonate could be produced with the yield of about 90% to the chloroform injected from the syringe pump 1 as the result shown in Table 15. The reaction efficiency was high in the reaction in a coil reactor, since the alcohol injected under high temperature can be vaporized to react with phosgene in a gas phase. As a result, phosgene was not detected at the outlet part of the coil reactor and thus it could be achieved that the detection value of phosgene at the system outlet was zero at the laboratory level.

A gas phase reaction under high temperature in a coil reactor becomes possible by developing a continuous flow reaction system as the above-described result.

In addition, 2-propanol (IPA) was injected; as a result, the target chloroformic acid ester could be produced with the yield of 74%, though 2-propanol is generally not reacted with phosgene under room temperature.

### Example 13: Synthesis of chloroformic acid ester by light flow system

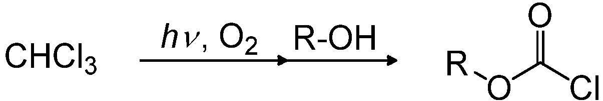

The coil heater temperature in the light flow system schematically demonstrated in Figure 2 was adjusted to 90°C, the oxidative photodecomposition gas produced from 7.4 mL/min of chloroform gas and 12.4 mL/min of oxygen gas was mixed with the alcohol injected by the syringe pump in the T-type mixer, and the mixed gas was supplied into the PTFE tube reactor at 30°C for a flow reaction. The product was obtained in the connected two-neck flask which was cooled to 0°C. The unreacted gas was supplied into the further connected alkali trap in order not to be leaked outside.

After the reaction, the reaction mixture was analyzed by ¹H NMR; as a result, the target chloroformic acid ester could be produced with the yields shown in Table 16.

**Table 16**

| Entry | CHCl₃ | Max gas flow rate | | Tube reactor | | | Reactive substrate | | | | Yield |
|---|---|---|---|---|---|---|---|---|---|---|---|
| | | CHCl₃ | O₂ | Inner diameter | Length | Volume | Structure | Amount | Solvent | Flow rate | |
| 1 | 90mmol | 7.4 mL/min | 12.0 mL/min | 2.4 mm | 2830 mm | 12.8 mL | 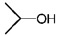 | 243 mmol | - | 0.052 mL/min | 74%^{a} |
| 2 | 90mmol | 7.4 mL/min | 12.0 mL/min | 2.4 mm | 7700 mm | 34.8 mL | 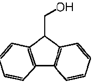 | 5 mmol | THF 9mL | 0.075 mL/min | 99%^{b} |

| | | | | | | | | | | | |
|---|---|---|---|---|---|---|---|---|---|---|---|
| a:yield to chloroform, b:yield to alcohol | | | | | | | | | | | |

### Example 14: Synthesis of ethylene carbonate

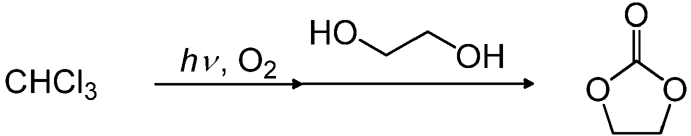

Ethylene glycol (1.62 mL, 29.0 mmol) was further injected into the phosgene gas produced from chloroform (total 2.37 mL, 29.4 mmol) using a syringe pump at the flow amount of 0.014 mL/min in similar conditions to Example 12 for 2 hours to be reacted through the coil reactor that was heated to 200°C. A two-neck flask that was cooled to 0°C was attached to the outlet of the coil reactor as the recovery vessel 10 to collect the product. The unreacted decomposed gas was trapped in the further connected alcohol trap as the trap vessel 7, and the waste gas was treated with an alkali trap. But phosgene was not detected at the stage of the alcohol trap.

To the obtained reaction mixture, 1,1,2,2-tetrachloroethane was added as an internal standard material. The mixture was analyzed by ¹H NMR; as a result, it was confirmed that ethylene carbonate was produced as the target compound with the yield of 54% to the used chloroform and ethylene glycol.

### Example 15: Continuous flow synthesis using NMI by light flow system

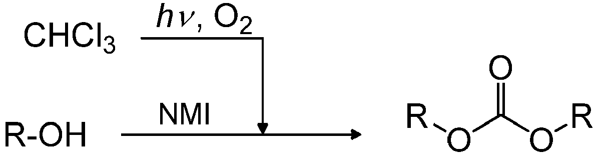

If an organic base is injected as a catalyst into the coil reactor in the continuous flow reaction system of Figure 2, HCl is produced with the progress of the reaction and reacted with the organic base to be a salt. The salt may possibly cause the clogging of a reactor tube. The inventors of the present invention came up with the idea to select the organic base of which salt with HCl is not precipitated, in other words, of which salt is an ionic liquid as a liquid salt in the present invention system.

### (1) Synthesis of bis-1,1,1,3,3,3-hexafluoro-1-propyl carbonate

A mixed liquid of hexafluoroisopropanol (HFIP) (14.8 mL, 188 mmol) and 1-methylimidazole (NMI) (7.9 mL, 75 mmol) was injected to the phosgene gas produced by the continuous flow photoreaction system from the syringe pump 8 for injecting a reactive substrate at the flow amount of 0.125 mL/min for total 3 hours similar conditions to Example 12 except that the temperature-adjustable coil reactor was changed to one having the inner diameter of 2.4 mm, the length of 2830 mm and the volume of 12.8 mL. The mixture was supplied to the coil reactor 9 heated to 100°C for the reaction. The product was collected in the recovery vessel 10-1 that was connected to the outlet of the coil reactor and the recovery vessel 10-2. The temperature of the recovery vessel 10-1 was adjusted to 100°C so that the liquid condition of the salt of NMI and HCl could be sufficiently maintained and the target carbonate could be recovered. On the one hand, the temperature of the recovery vessel 10-2 was adjusted to 0°C, since the target carbonate might be possibly leaked from the recovery vessel 10-1. The gas discharged from the recovery vessel 10-2 was trapped by the further connected alcohol trap as the trap vessel 7 and the waste gas was treated by an alkali trap but phosgene was not detected at the stage of the alcohol trap.

The collected products in the recovery vessel 10-1 and the recovery vessel 10-2 were combined. The mixture was separated to two layers. The lower layer was washed with 1.4 mol/L hydrochloric acid (20 mL), dried over anhydrous sodium sulfate and filtrated. The filtrate was concentrated to obtain the target compound as a colorless and transparent liquid (yield to the used HFIP: 61%).

### (2) Synthesis of bis-2,2,3,3-tetrafluoropropyl carbonate

A mixed liquid of 2,2,3,3-tetrafluoro-1-propanol (8.86 mL, 100 mmol) and NMI (12 mL, 150 mmol) was injected to the phosgene gas produced by the continuous flow photoreaction system from the syringe pump 8 for injecting a reactive substrate at the flow amount of 0.12 mL/min for total 3 hours similar conditions to Example 12 except that the temperature-adjustable coil reactor was changed to one having the inner diameter 2.4 mm, the length of 2830 mm and the volume of 12.8 mL. The mixture was supplied to the coil reactor 9 heated to 100°C for the reaction. The product was collected in the recovery vessel 10-1 that was connected to the outlet of the coil reactor and the recovery vessel 10-2. The recovery vessel 10-1 was heated to 100°C, and the recovery vessel 10-2 was cooled to 0°C. The unreacted decomposed gas was trapped by the further connected alcohol trap as the trap vessel 7 and the waste gas was treated by an alkali trap but phosgene was not detected at the stage of the alcohol trap.

To the obtained product sample solution, 1,1,2,2-tetrachloroethane was added as an internal standard material. The mixture was analyzed by ¹H NMR; as a result, it was confirmed that the target compound was produced with the yield of 70% to the used alcohol.

The raw material was removed from the obtained sample solution by distillation. The sample solution was washed with 1.4 mol/L HCl (20 mL), dried over anhydrous sodium sulfate, and filtrated. The filtrate was concentrated to obtain the target compound as a colorless and transparent liquid (yield to the used 2,2,3,3-tetrafluoro-1-propanol: 44%).

### (3) Synthesis of bis-2,2,2-trifluoroethyl carbonate

A mixed liquid of 2,2,2-trifluoroethanol (10 g, 100 mmol) and NMI (12 mL, 150 mmol) was injected to the phosgene gas produced by the continuous flow photoreaction system from the syringe pump 8 for injecting a reactive substrate at the flow amount of 0.11 mL/min for total 3 hours similar conditions to Example 12 except that the temperature-adjustable coil reactor was changed to one having the inner diameter 2.4 mm, the length of 2830 mm and the volume of 12.8 mL. The mixture was supplied to the coil reactor 9 heated to 100°C for the reaction. The product was collected in the recovery vessel 10-1 that was connected to the outlet of the coil reactor and the recovery vessel 10-2. The recovery vessel 10-1 was heated to 100°C, and the recovery vessel 10-2 was cooled to 0°C. The unreacted decomposed gas was trapped by the further connected alcohol trap as the trap vessel 7 and the waste gas was treated by an alkali trap but phosgene was not detected at the stage of the alcohol trap.

To the obtained reaction mixture, 1,1,2,2-tetrachloroethane was added as an internal standard material. The mixture was analyzed by ¹H NMR; as a result, it was confirmed that the target compound was produced with the yield of 60% to the used alcohol.

The raw material compound was removed from the obtained sample solution by distillation. The thus obtained residue was washed with 3 M hydrochloric acid (20 mL), dried over anhydrous sodium sulfate, and filtrated. The filtrate was concentrated to obtain the target compound as a colorless and transparent liquid (yield amount: 6.43 g, yield to the used 2,2,2-trifluoroethanol: 44%).

### (4) Synthesis of diphenyl carbonate

A mixed liquid of phenol (18.8 mL, 200 mmol) and NMI (24 mL, 300 mmol) was injected to the phosgene gas produced by the continuous flow photoreaction system from the syringe pump 8 for injecting a reactive substrate at the flow amount of 0.23 mL/min for total 3 hours similar conditions to Example 12 except that the temperature-adjustable coil reactor was changed to one having the inner diameter 2.4 mm, the length of 2830 mm and the volume of 12.8 mL. The mixture was supplied to the coil reactor 9 heated to 115°C for the reaction. The product was collected in the recovery vessel 10 that was connected to the outlet of the coil reactor and heated to 100°C. The unreacted decomposed gas was trapped by the further connected alcohol trap as the trap vessel 7 and the waste gas was treated by an alkali trap but phosgene was not detected at the stage of the alcohol trap.

To the obtained reaction mixture, 1,1,2,2-tetrachloroethane was added as an internal standard material. The mixture was analyzed by ¹H NMR; as a result, it was confirmed that the target compound was produced with the yield of 72% to the used phenol.

Dichloromethane and 1.4 mol/L HCl were added to the obtained sample solution, and the upper layer and the lower layer were separated. The lower layer was dried over anhydrous sodium sulfate and filtrated. The solvent was removed under reduced pressure from the filtrate. The residue was dried in vacuo in the heating condition using a glass tube oven to obtain the white target solid (isolated yield to the used chloroform: 56%).

**Table 17**

| Injected CHCl₃ | | Heater temp. | Max gas flow rate | | Mixed gas | | ROH | NMI | Reaction coil temp. | Carbonate yield^{a} [isolated yield^{a}] |
|---|---|---|---|---|---|---|---|---|---|---|
| Amount | Flow rate | | CHCl₃ | O₂ | Linear velocity | Residence time | | | | |
| 6.11g (51.2 mmol) | 0.02 mL/mi n | 90°C | 7.4 mL/min | 12.4 mL/mi n | 5.8 m/min | 40s | HFIP 12.6g (75mmol) | 14.81mL (187.5 mmol) | 100°C | [61%] |
| 5.51g (46.2 mmol) | | | | | | | 4FPA 8.86mL (100mmol) | 12.00mL (150.0 mmol) | 100°C | 70% [44%] |
| 5.37g (45.0 mmol) | | | | | | | 3FEA 10g (100mmol) | 12.00mL (150.0 mmol) | 110°C | 60% [44%] |
| 5.96g (49.9 mmol) | | | | | | | PhOH 18.81g (200mmol) | 24.00mL (300.0 mmol) | 115°C | 72% [56%] |

| | | | | | | | | | | |
|---|---|---|---|---|---|---|---|---|---|---|
| a:yield to injected CHCl₃ | | | | | | | | | | |

It was found as the above-described result that even when the base that forms an ionic liquid with hydrochloride produced as a by-product, such as 1-methylimidazole, is supplied into a coil reactor, the coil is not clogged and the continuous reaction can be successfully promoted.

### Example 16: Continuous flow synthesis by flow system using pyridine

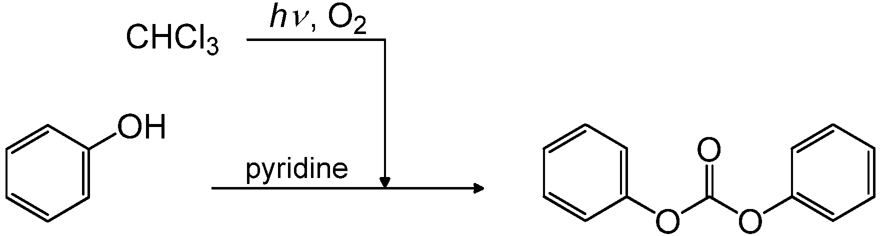

A mixed liquid of phenol (9.41 g, 100 mmol) and pyridine (12 mL, 150 mmol) was injected to the phosgene gas produced by the continuous flow photoreaction system from the syringe pump 8 for injecting a reactive substrate at the flow amount of 0.11 mL/min for total 3 hours similar conditions to Example 15. The mixture was supplied to the coil reactor 9 heated to 160°C for the reaction. The product was collected in the recovery vessel 10-1 that was connected to the outlet of the coil reactor and the recovery vessel 10-2. The temperature of the recovery vessel 10-1 was adjusted to 155°C so that the liquid state of the salt of pyridine and HCl could be sufficiently maintained and the target carbonate can be obtained. On the one hand, the temperature of the recovery vessel 10-2 was adjusted to -5°C, since the target carbonate might be possibly leaked from the recovery vessel 10-1. The gas discharged from the recovery vessel 10-2 was trapped by the further connected alcohol trap as the trap vessel 7 and the waste gas was treated by an alkali trap but phosgene was not detected at the stage of the alcohol trap.

As a result, the clogging of the pyridine hydrochloride salt was not recognized in the coil reactor, and thus the produced pyridine hydrochloride salt might be dissolved or melted to be flowed out.

To the obtained reaction mixture, 1,1,2,2-tetrachloroethane was added as an internal standard material. The mixture was analyzed by ¹H NMR; as a result, it was confirmed that the target diphenyl carbonate was produced (yield to the used phenol: 65%, yield to the used chloroform: 72%).

### Example 17: Continuous flow synthesis by flow system using solvent

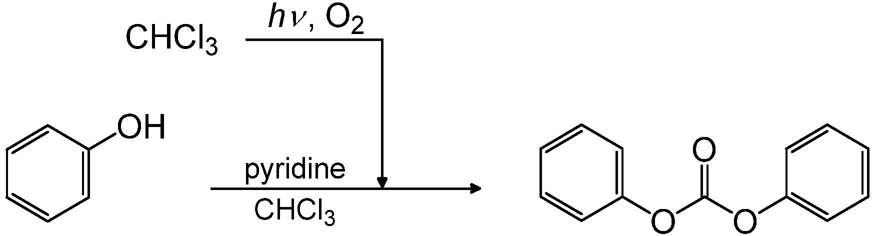

If an organic base is injected as a catalyst to the coil reactor in the continuous flow reaction system of Figure 2, HCl is produced with the progress of the reaction and reacted with the organic base to be a salt. The salt may possibly cause the clogging of a reactor tube. The inventors of the present invention came up with the idea to use the solvent to dissolve the produced HCl salt in the present invention system.

A mixed liquid prepared by dissolving phenol (1.88 g, 20 mmol) and pyridine (6.4 mL, 80 mmol) in chloroform (16 mL) was injected to the phosgene gas produced by the continuous flow photoreaction system from the syringe pump 8 for injecting a reactive substrate at the flow amount of 0.2 mL/min for total 2 hours similar conditions to Example 15. The mixture was supplied to the coil reactor 9 for the reaction at room temperature. The product was collected in a two-neck flask as the recovery vessel 10 connected to the outlet of the coil reactor. The waste gas was treated by an alkali trap as the trap vessel 7.

As a result, since the deposit of the pyridine hydrochloride salt was not recognized in the coil reactor, and thus the produced pyridine hydrochloride salt might be dissolved in chloroform to be flowed out.

To the obtained reaction mixture, 1,1,2,2-tetrachloroethane was added as an internal standard material. The mixture was analyzed by ¹H NMR; as a result, it was confirmed that the target diphenyl carbonate was produced (yield to the used phenol: 90%).

Then, the obtained sample solution was washed with 1 M hydrochloric acid and water, and extraction was carried out using dichloromethane. The organic layer was dried over anhydrous sodium sulfate, and the solvent was distilled away under reduced pressure. The residue was subjected to distillation under reduced pressure using a glass tube oven to obtain white solid diphenyl carbonate from the fraction of 100 to 155°C (yield amount: 1.79 g, yield: 84%).

### Example 18: Continuous flow synthesis by flow system using solvent

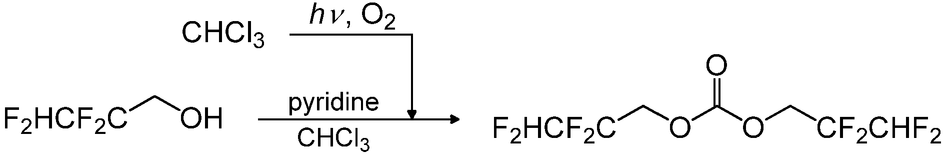

A mixed liquid prepared by dissolving 2,2,3,3-tetrafluoro-1-propanol (1.96 mL, 20 mmol) and pyridine (6.4 mL, 80 mmol) in 16 mL of chloroform was injected to the phosgene gas produced by the continuous flow photoreaction system from the syringe pump 8 for injecting a reactive substrate at the flow amount of 0.2 mL/min for total 2 hours similar conditions to Example 15. The mixture was supplied to the coil reactor 9 for the reaction at room temperature. The product was collected in a two-neck flask as the recovery vessel 10 connected to the outlet of the coil reactor. The waste gas was treated by an alkali trap as the trap vessel 7. To the obtained reaction mixture, 1,1,2,2-tetrachloroethane was added as an internal standard material. The mixture was analyzed by ¹H NMR; as a result, it was confirmed that the target bis(2,2,3,3-tetrafluoropropyl) carbonate was produced (yield to the used alcohol: 94%).

Then, the obtained sample solution was washed with 1 M hydrochloric acid and water, and extraction was carried out using dichloromethane. The organic layer was dried over anhydrous sodium sulfate, and the colorless liquid target compound was obtained from the fraction of 60 to 70°C by distillation under reduced pressure using a diaphragm pump (yield amount: 2.09 g, yield: 72%).

### Example 19: Continuous flow synthesis by flow system using solvent

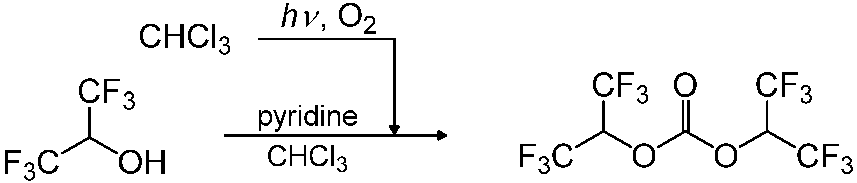

A mixed liquid prepared by dissolving HFIP (2.31 mL, 20 mmol) and pyridine (6.4 mL, 80 mmol) in chloroform (16 mL) was injected to the phosgene gas produced by the continuous flow photoreaction system from the syringe pump 8 for injecting a reactive substrate at the flow amount of 0.2 mL/min for total 2 hours similar conditions to Example 15. The mixture was supplied to the coil reactor 9 for the reaction at room temperature. The product was collected in a two-neck flask as the recovery vessel 10 connected to the outlet of the coil reactor. The waste gas was treated by an alkali trap as the trap vessel 7. To the obtained reaction mixture, 1,1,2,2-tetrachloroethane was added as an internal standard material. The mixture was analyzed by ¹H NMR; as a result, it was confirmed that the target bis(1,1,1,3,3,3-hexafluoropropane-2-yl) carbonate was produced (yield to the used alcohol: 61%).

Then, the obtained sample solution was washed with 1 M hydrochloric acid and water, and extraction was carried out using dichloromethane. The organic layer was dried over anhydrous sodium sulfate, and the colorless liquid target compound was obtained from the fraction of 65 to 75°C by distillation (yield amount: 1.45 g, yield: 40%).

### Example 20: Synthesis of carbonate by supplying gas method

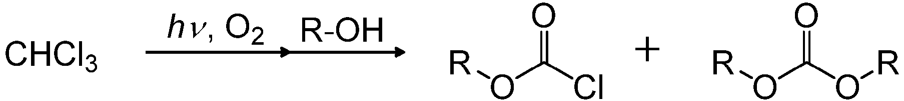

A photoreaction system was constructed by inserting a quartz glass jacket having the diameter of 30 mm in a cylindrical reaction vessel having the diameter of 42 mm and the volume of 100 mL and further inserting a low pressure mercury lamp ("UVL20PH-6" manufactured by SEN Light, 20 W, φ24 mm × 120 mm) in the quartz glass jacket as schematically demonstrated in Figure 3. The irradiated light from the low pressure mercury lamp contained UV-C having the wavelength of 185 nm and the wavelength of 254 nm, and the illumination intensity of the light having the wavelength of 185 nm at the position 5 mm away from the center of the lamp to the reaction mixture was 2.00 to 2.81 mW/cm², and the illumination intensity of the light having the wavelength of 254 nm was 5.60 to 8.09 mW/cm². The cylindrical reaction vessel 12 was equipped with the cooling condenser 15 that was used for selectively transporting the produced low-boiling point gas component and cooled to 10°C, and a two-neck flask as the reaction vessel 16 equipped with the cooling condenser 15 cooled to -10°C was connected thereto. The cooling condenser 15 was further connected to the two-neck eggplant flask containing an alcohol and the trap vessel containing an alkali aqueous solution.

The bath temperature of the photoreaction vessel 12 was adjusted to the temperature shown in Table 18, and then liquid chloroform was supplied to the photoreaction vessel from the PTFE tube having the inner diameter of 1 mm at the flow rate shown in Table 18 using a syringe pump. The chloroform was stirred to accelerate vaporization. Next, oxygen was supplied to the gas phase of the reaction vessel at the rate of 0.1 mL/min from another direction to prepare a mixed gas of chloroform and oxygen in the photoreaction vessel 12, and light was irradiated thereto using the low pressure mercury lamp. The gas produced by the oxidative photodecomposition of the mixed gas was blown into the stirred 1-hexanol (30 mL, 239 mmol) in the connected two-neck eggplant flask at room temperature. The unreacted gas was trapped by the further connected 1-hexanol trap as the trap vessel, and the waste gas from the trap vessel was treated by an alkali trap so that toxic gas was not leaked outside.

The yields of the chloroformic acid ester and the carbonate produced in the reaction vessel 16 and the trap vessel were estimated by ¹H NMR spectra, and the amount of the produced phosgene was determined on the basis of the total amounts thereof. The result is shown in Table 18.

**Table 18**

| Injected CHCl₃ | | Mixed gas | | Bath temp. | Condenser temp. | Phosgen yield^{a} [Amount] |
|---|---|---|---|---|---|---|
| Amount | Flow rate | Linear velocity | Residence time | | | |
| 10mL (125mmol) | 2.5mL/h | 0.312 m/min | 33s | 110°C | 10°C | 91% [113.4mmol] |
| 4mL (50mmol) | 1.0mL/h | 0.276 m/min | 37s | 90°C | 10°C | 97% [48.6mmol] |

| | | | | | | |
|---|---|---|---|---|---|---|
| a:yield to the injected halogenated hydrocarbon | | | | | | |

The gas phase photoreaction of vaporized chloroform and oxygen gas was carried out by continuously injecting chloroform into the photoreaction vessel heated to the boiling point or higher of chloroform from outside using a syringe pump; as a result, chloroform might be relatively rapidly decomposed to produce phosgene as the result shown in Table 8.

The phosgene gas produced with high conversion ratio of 97% or higher did not contain a notable by-product and was reacted with an alcohol to produce a chloroformic acid ester or a carbonate having high purity. A small amount of a by-product was carbon monoxide and carbon dioxide.

### Example 21: Synthesis of Vilsmeier reagent by gas phase photoreaction

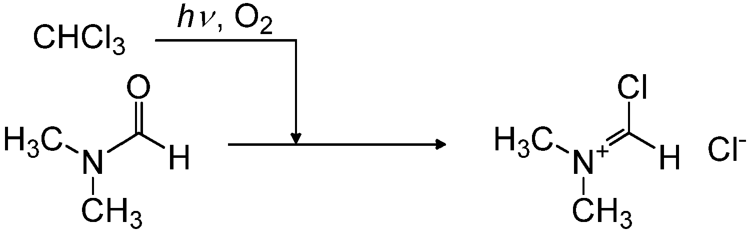

The oxidative photodecomposition gas produced from total 6.83 g (57.2 mmol) of chloroform using the photoreaction system schematically demonstrated in Figure 1 was blown into the stirred N,N-dimethylformamide (DMF) (5.5 mL, 70 mmol) at atmospheric temperature in the connected two-neck eggplant flask for 4 hours similarly to Example 1. The unreacted gas was supplied into a further connected alcohol trap and an alkali trap to be treated so that the gas was not leaked outside.

The reaction mixture was analyzed by ¹H NMR after the reaction; as a result, it was confirmed that the Vilsmeier reagent could be produced with the yield of 51% or more to the used chloroform. The actual yield amount may be higher than the result, since a Vilsmeier reagent is reacted with moisture in the air to be decomposed into a raw material amide. The result is shown in Table 19.

**Table 19**

| CHCl₃ | Max gas flow rate | | Mixed gas | | Photoreaction temp. | Substrate | Vilsmeier reagent yield |
|---|---|---|---|---|---|---|---|
| | CHCl₃ | O₂ | Linear velocity | Residence time | | | |
| 57.2mmol | 7.4mL/min (0.25mmol/min) | 12.4mL/min (0.45mmol/min) | 5.8 m/min | 40s | 90°C | DMF 70mmol | >51% |

Chloroform was heated to 90°C using a coil heater to be vaporized, and the vaporized chloroform was mixed with oxygen gas for the gas phase flow photoreaction; as a result, a Vilsmeier reagent could be produced with the yield of more than 51% from DMF and the phosgene produced from chloroform in the gas phase.

### Example 22: Synthesis of Vilsmeier reagent and acid chloride by gas phase photoreaction

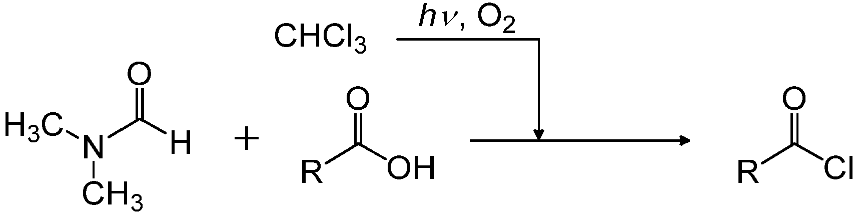

The oxidative photodecomposition gas produced from chloroform gas at 7.4 mL/min and oxygen gas at 12.4 mL/min was blown into the stirred chloroform solution (100 mL) prepared by dissolving benzoic acid or propionic acid and DMF in the connected two-neck eggplant flask at 30°C for 3 to 5 hours using the flow photoreaction system similarly to Example 21. The unreacted gas was supplied to the further connected alcohol trap and alkali trap in order not to be leaked outside.

The reaction mixture was analyzed by ¹H NMR after the reaction; as a result, the target carboxylic acid chloride could be produced as the result shown in Table 20.

**Table 20**

| CHCl₃ | Max gas flow rate | | Mixed gas | | Photoreaction temp. | Substrate | | Target yield |
|---|---|---|---|---|---|---|---|---|
| | CHCl₃ | O₂ | Linear velocity | Residence time | | DMF | Carboxylic acid | |
| 52.3 mmol | 7.4 mL/min (0.25 mmol/min) | 12.4 mL/min (0.45 mmol/min) | 5.8 m/min | 40s | 90°C | 100mmol | Benzoic acid 200mmol | 70% |
| 44.6 mmol | | | | | | 100mmol | Propionic acid 200mmol | 61% |
| 62.5 mmol | | | | | | 50mmol | Benzoic acid 50mmol | 97%* |
| 75.0 mmol | | | | | | 50mmol | Propionic acid 50mmol | >99%* |
| 62.5 mmol | | | | | | 50mmol | Nonanoic acid 50mmol | 76%* |
| 19.3 mmol | | | | | | 15mmol | 4-Fluoro benzoic acid 15mmol | 90%* |

| | | | | | | | | |
|---|---|---|---|---|---|---|---|---|
| *:yield to the used carboxylic acid | | | | | | | | |

It was found as the result shown in Table 20 that the conversion ratio from a carboxylic acid to a carboxylic acid chloride becomes higher when a chloroform amount and a DMF ratio to the carboxylic acid is larger.

### Example 23: Synthesis of Vilsmeier reagent and formic acid ester by gas phase photoreaction

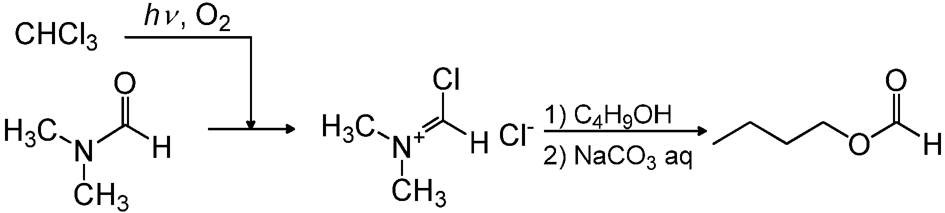

The oxidative photodecomposition gas produced from a mixed gas of chloroform gas at 7.4 mL/min and oxygen gas at 12.4 mL/min from 5.82 g (48.8 mmo1) of chloroform was blown into the stirred DMF (8.0 mL, 100 mmo1) in the connected two-neck eggplant flask at atmospheric temperature for 3 hours using the flow photoreaction system similarly to Example 21. The unreacted gas was supplied to the further connected alcohol trap and alkali trap in order not to be leaked outside.

To the reaction mixture, 1-butanol (4.57 mL, 50 mmo1) was added. The mixture was stirred at atmospheric temperature for 1.5 hours. Then, saturated sodium carbonate aqueous solution (50 mL) was added thereto for hydrolysis. Dichloromethane was added thereto, and the organic phase and the aqueous phase were separated. The organic phase was dried over anhydrous sodium sulfate and then filtrated.

The obtained filtrate was analyzed by ¹H NMR; as a result, it was confirmed that the target formic acid ester was produced with the yield of 60% to the used chloroform. The result is shown in Table 21.

**Table 21**

| CHCl₃ | Flow rate | | Mixed gas | | Photoreaction temp. | Substrate | | Target yield |
|---|---|---|---|---|---|---|---|---|
| | CHCl₃ | O₂ | Linear velocity | Residence time | | DMF | 1-Butanol | |
| 48.8mmol | 7.4mL/min (0.25 mmol/min) | 12.4mL/min (0.45 mmol/min) | 5.8 m/min | 40s | 90°C | 100mmol | 50mmol | 60% |

### Example 24: Synthesis of Vilsmeier reagent and formylating reaction by gas phase photoreaction

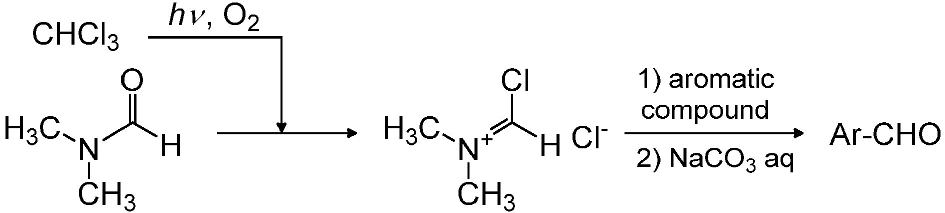

The oxidative photodecomposition gas produced from a mixed gas of chloroform gas at 7.4 mL/min and oxygen gas at 12.4 mL/min from 5.37 g (45.0 mmo1) of chloroform was blown into the stirred DMF (3.9 mL, 50 mmo1) in the connected two-neck eggplant flask at atmospheric temperature for 3 hours using the flow photoreaction system similarly to Example 21. The unreacted gas was supplied to the further connected alcohol trap and alkali trap in order not to be leaked outside.

To the reaction mixture, 1-methylpyrrole or 2-methylfurane (50 mmo1) was added as an aromatic compound. The mixture was stirred at 70°C for 2 hours. Then, saturated sodium carbonate aqueous solution (100 mL) was added thereto for hydrolysis. Water and dichloromethane were added thereto, and the organic phase and the aqueous phase were separated. The organic phase was dried over anhydrous sodium sulfate and then filtrated. The solvent was removed from the filtrate using an evaporator, and the target compound was obtained by purification from the obtained dark green oil by column chromatography with the isolated yield of 98% or 74%. The result is shown in Table 22.

**Table 22**

| CHCl₃ | Flow rate | | Mixed gas | | Photoreaction temp. | Substrate | | Target yield |
|---|---|---|---|---|---|---|---|---|
| | CHCl₃ | O₂ | Linear velocity | Residence time | | DMF | Aromatic compound | |
| 45.0mmol | 7.4mL/min | 12.4mL/min | 5.8 | 40s | 90°C | 100 mmol | 1-Methyl pyrrole | 98%* |
| | (0.25 mmol/min) | (0.45 mmol/min) | m/min | | | | 50mmol | |
| 47.7mmol | 7.4mL/min | 12.4mL/min | 5.8 m/min | 40s | 90°C | 100 mmol | 2-Methyl furane | 74%* |
| | (0.25 mmol/min) | (0.45 mmol/min) | | | | | 50mmol | |

| | | | | | | | | |
|---|---|---|---|---|---|---|---|---|
| *:isolated yield | | | | | | | | |

### Example 25: Synthesis of ester or carboxylic acid anhydride

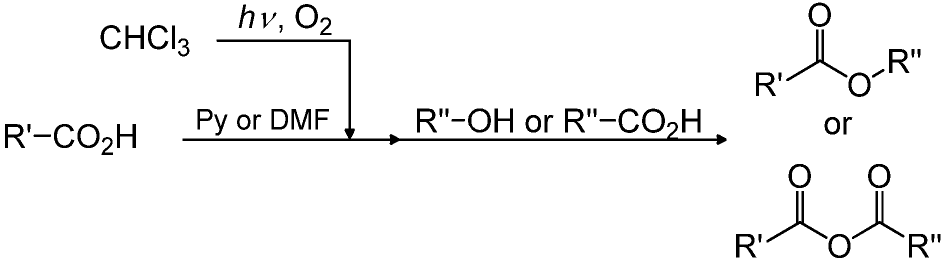

The coil heater temperature of the light flow system schematically demonstrated in Figure 2 was adjusted to 90°C. The oxidative photodecomposition gas produced from chloroform gas (0.02 mL/min) and oxygen gas (10 mL/min) and the solution injected from the syringe pump containing a carboxylic acid and 5 times amount by mole of pyridine or 1 time amount by mole of DMF were mixed using a T-type mixer, and the mixture was supplied to the PTFE tube reactor having the inner diameter of 2.4 mm, the length of 2830 mm and the volume of 12.8 mL for a flow reaction. The product was blown into the stirred chloroform solution of an alcohol or a carboxylic acid in a two-neck flask. The unreacted gas was supplied to the further connected alkali trap in order not to be leaked outside.

The reaction mixture was analyzed by ¹H NMR after the reaction; as a result, the ester or the carboxylic acid anhydride was produced with the yield shown in Table 23.

In addition, the reaction mixture was washed with water, and extraction was carried out using dichloromethane. The extraction liquid was dried over anhydrous sodium sulfate, and the solvent was distilled away under reduced pressure. Further, the target compound was purified by distillation under reduced pressure or column chromatography. In the table, "BA" is benzoic acid, "4-FBA" is 4-fluorobenzoic acid, "PA" is propionic acid, and "Py" is pyridine.

**Table 23**

| Entry | CHCl₃ | Carboxylic acid solution | | | | | Reaction vessel | | | | Product yield |
|---|---|---|---|---|---|---|---|---|---|---|---|
| | | Carboxylic acid | Amount | Base | Solvent | Flow rate | Alcohol | Amount | Solvent | Reaction temp. | |
| 1 | 1.2mL | BA | 3.66g | Py | CHCl₃ 15mL | 12.5 mL/min | EtOH | 10mL | CH₂Cl₂ | rt | Ester |
| | 15mmol | | 30mmol | | | | | 171mmol | | | 46%^{a} |
| 2 | 1.2mL | BA | 3.66g | Py | CHCl₃ 11mL | 12.5 mL/min | 1-BuOH | 10mL | -- | rt | Ester 90%^{a} |
| | 15mmol | | 30mmol | | | | | 109mmol | | | 83%^{b} |
| 3 | 1.2mL 15mmol | BA | 3.66g 30mmol | Py | CHCl₃ 16mL | 12.5 mL/min | *i* -PrOH | 1.52mL 20mmol | CH₂Cl₂ | rt | Ester 36%^{a} |
| 4 | 1.2mL | 4 - FBA | 2.8g | DMF | | 9 mL/min | 1-BuOH | 10mL | -- | rt | Ester >99%^{a} |
| | 15mmol | | 20mmol | | | | | 109mmol | | | 84%^{b} |
| 5 | 2.4mL | BA | 3.66g | Py | CHCl₃ 11mL | 12.6 mL/min | PhOH | 2.8g | CHCl₃ | 50°C | Ester 20%^{a} |
| | 30mmol | | 30mmol | | | | | 20mmol | | | 20%^{b} |
| 6 | 1.2mL | 4 - FBA | 2.8g | DMF | | 9 mL/min | PhOH | 1.88g | -- | reflux | Ester 90%^{a} |
| | 15mmol | | 20mmol | | | | | 20mmol | | | 84%^{b} |
| 7 | 1.2mL | PA | 2.24mL | DMF | CHCl₃ 16mL | 9.25 mL/min | 1-BuOH | 10mL | -- | rt | Ester 78%^{a} |
| | 15mmol | | 30mmol | | | | | 109mmol | | | 70%^{b} |
| 6 | 1.2mL | BA | 3.66g | Py | CHCl₃ 15mL | 12 mL/min | BA | 3.66g | -- | rt | Acid anhydride |
| | 15mmol | | 30mmol | | | | | 30mmol | | | 99%^{a} |
| | | | | | | | | | | | 84%^{b} |

| | | | | | | | | | | | |
|---|---|---|---|---|---|---|---|---|---|---|---|
| a:yield determined by NMR, b:isolated yield | | | | | | | | | | | |

The ester or the carboxylic acid anhydride may be produced by directly reacting the decomposed product of chloroform with the carboxylic acid or generating a Vilsmeier reagent from the decomposed product of chloroform and DMF and reacting the Vilsmeier reagent with the carboxylic acid to produce a carboxylic acid chloride, and further reacting the carboxylic acid chloride with the alcohol or the carboxylic acid in the coil reactor as the above-described result.

### Example 26: Synthesis of amide

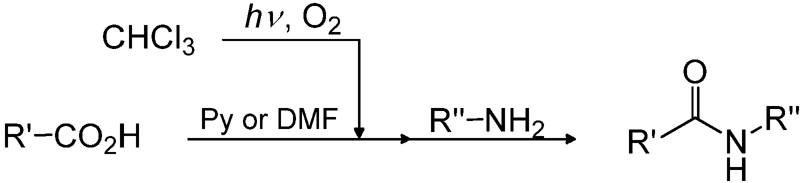

The coil heater temperature of the light flow system schematically demonstrated in Figure 2 was adjusted to 90°C. The oxidative photodecomposition gas produced from chloroform gas (2.4 mL, 30 mmol, 0.02 mL/min) and oxygen gas (10 mL/min) and the solution injected from the syringe pump containing a carboxylic acid and 5 times amount by mole of pyridine or 1 time amount by mole of DMF were mixed using a T-type mixer, and the mixture was supplied to the PTFE tube reactor having the inner diameter of 2.4 mm, the length of 2830 mm and the volume of 12.8 mL for a flow reaction. The product was blown into the stirred amine solution in a two-neck flask. The unreacted gas was supplied to the further connected alkali trap in order not to be leaked outside.

The reaction mixture was analyzed by ¹H NMR after the reaction; as a result, the amide was produced with the yield shown in Table 24.

In addition, the reaction mixture was washed with water, and extraction was carried out using dichloromethane. The extraction liquid was dried over anhydrous sodium sulfate, and the solvent was distilled away under reduced pressure. Further, the target compound was purified by recrystallization as needed. In the table, "cHex-NH₂" is cyclohexyllamine.

**Table 24**

| Entry | CHCl₃ | Carboxylic acid solution | | | | | Reaction vessel | | | | Yield |
|---|---|---|---|---|---|---|---|---|---|---|---|
| | | Carboxylic acid | Amount | Base | Solvent | Flow rate | Amine | Amount | Solvent | Reaction temp. | |
| 1 | 2.4mL | BA | 3.66g | Py | CHCl₃ 23mL | 20 mL/min | aniline | 8.22mL | CH₂Cl₂ | rt | 75%^{a} |
| | 30mmol | | 30mmol | | | | | 90mmol | | | 68%^{b} |
| 2 | 2.4mL | BA | 3.66g | DMF | CHCl₃ 20mL | 12.5 mL/min | aniline | 13.7mL | CH₂Cl₂ | rt | 75%^{a} |
| | 30mmol | | 30mmol | | | | | 150mmol | | | 68%^{b} |
| 3 | 2.4mL | 4-FBA | 2.8g | DMF | - | 9 mL/min | aniline | 9.1mL | CH₂Cl₂ | rt | 75%^{a} |
| | 30mmol | | 20mmol | | | | | 100mmol | | | 71%^{b} |
| 4 | 2.4mL | BA | 3.66g | Py | CHCl₃ 23mL | 20 mL/min | cHex-NH₂ | 3.78mL | CH₂Cl₂ | rt | 1%^{b} |
| | 30mmol | | 30mmol | | | | | 33mmol | | | 7 |
| 5 | 2.4mL | PA | 1.49mL | DMF | CHCl₃ 16mL | 9 mL/min | aniline | 9.1mL | CHCl₃ | 50°C | 90%^{a} |
| | 30mmol | | 20mmol | | | | | 100mmol | | | 74%^{b} |

| | | | | | | | | | | | |
|---|---|---|---|---|---|---|---|---|---|---|---|
| a:yield determined by NMR, b:isolated yield | | | | | | | | | | | |

The amide may be produced by directly reacting the decomposed product of chloroform with the carboxylic acid or generating a Vilsmeier reagent from the decomposed product of chloroform and DMF and reacting the Vilsmeier reagent with the carboxylic acid to produce a carboxylic acid chloride and further reacting the carboxylic acid chloride with the amine in the coil reactor as the above-described result.

### Example 27: Formylation of aromatic compound by Vilsmeier reagent

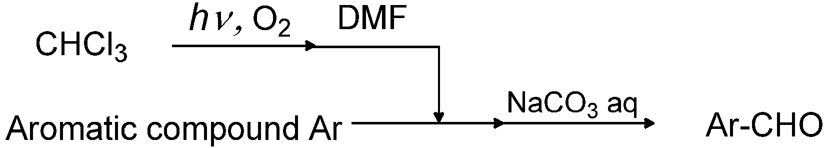

### (1) Formylation of 1-methylpyrrole

The syringe pump 8-1 for injecting a reactive substrate and the tube reactor 17 having the inner diameter of 2.4 mm, the length of 182 mm and the volume of 0.8 mL were connected to the flow photoreaction system of Figure 1 for the continuous reaction, and the syringe pump 8-2 for injecting a reactive substrate and the temperature-adjustable coil reactor 9 having the inner diameter of 2.4 mm, the length of 994 mm and the volume of 4.5 mL were further connected for the subsequent continuous reaction as demonstrated in Figure 4.

The mixed liquid of DMF (2.32 mL, 30 mmol) and chloroform (7.8 mL) was injected to the phosgene gas produced by the continuous flow photoreaction system from the syringe pump 8-1 for injecting a reactive substrate at the flow amount of 3.26 mL/h similarly to Example 1, and the mixture was supplied to the tube reactor 17 for the reaction at room temperature. The mixed liquid of 1-methylpyrrole (2.66 mL, 30 mmol) and dichloromethane (7.0 mL) was further injected from the other syringe pump 8-2 for injecting a reactive substrate at the flow amount of 3.2 mL/h for total 3 hours. The mixture was supplied to the coil reactor 9 for the reaction at room temperature.

The product was injected into saturated sodium carbonate aqueous solution (50 mL) in the two-neck flask as the recovery vessel 10 connected to the outlet of the coil reactor, and the mixture was stirred with the bath temperature of 0°C. Dichloromethane and water were added to the reaction mixture, and the organic phase and the aqueous phase were separated. The organic phase was dried over anhydrous sodium sulfate and then filtrated. Acetone (0.74 mL, 10 mmol) was added to the obtained dried organic phase as an internal standard material, and the mixture was analyzed by ¹H NMR; as a result, it was confirmed that 1-methyl-1H-pyrrole carboaldehyde was produced as the target compound (yield to the used 2-methylpyrrole and DMF: 53%).

### (2) Formylation of 2-methylfurane

The mixed liquid of DMF (2.32 mL, 30 mmol) and chloroform (7.8 mL) was injected to the phosgene gas produced by the continuous flow photoreaction system from the syringe pump 8-1 for injecting a reactive substrate at the flow amount of 3.26 mL/h similar conditions to Example 27(1), and the mixture was supplied to the tube reactor 17 for the reaction at room temperature. The mixed liquid of 2-methylfurane (2.32 mL, 30 mmol) and dichloromethane (7.0 mL) was further injected from the other syringe pump 8-2 for injecting a reactive substrate at the flow amount of 3.2 mL/h for total 3 hours. The mixture was supplied to the coil reactor 9 for the reaction at room temperature.

The product was injected into saturated sodium carbonate aqueous solution (50 mL) in the two-neck flask as the recovery vessel 10 connected to the outlet of the coil reactor, and the mixture was stirred with the bath temperature of 0°C. Dichloromethane and water were added to the reaction mixture, and the organic phase and the aqueous phase were separated. The organic phase was dried over anhydrous sodium sulfate and then filtrated. The obtained dried organic phase was analyzed by ¹H NMR; as a result, it was confirmed that 5-methyl-2-furaldehyde was produced as the target compound (conversion ratio to the used 2-methylfurane and DMF: 95%).

The solvent was distilled away from the above-described dried organic phase, and the obtained residue was subjected to silica gel column chromatography (eluent: dichloromethane) to isolate the target compound (yield to the used 2-methylfurane and DMF: 80%).

### Example 28: Selection of gas phase photoreaction vessel

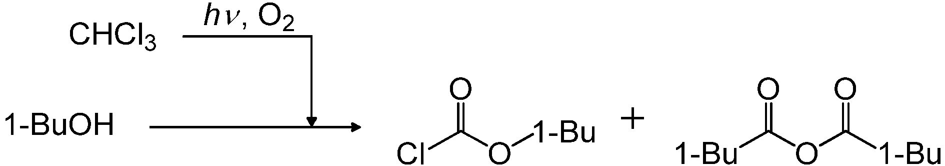

The gas phase photoreaction was carried out using the photoreaction system schematically demonstrated in Figure 5. Specifically, the reaction system was constructed by inserting a quartz glass jacket in a cylindrical reaction vessel, and further inserting a low pressure mercury lamp ("SUL-20P" manufactured by SEN Light, 20 W, length of light emitting part: 130 mm, wavelength: 185 to 600 nm, peak wavelength: 184.9 nm and 253.7 nm) in the quartz glass jacket. The sizes of the cylindrical reaction vessel as the outer tube and the quartz glass jacket as the inner tube are shown in Table 25.

Liquid chloroform and dried air of 20°C were supplied into the coil heater 3 heated to 90°C at the flow amount shown in Table 25 to be mixed and heated, and supplied to the photoreaction vessel 12 for 2 hours. The temperature of the heater 13 for heating the photoreaction vessel was adjusted to 100°C.

The gas discharged from the photoreaction vessel 12 was blown into 1-butanol in the reaction vessel 16-1, and the gas discharged from the reaction vessel 16-1 was further blown into 1-butanol in the reaction vessel 16-2. In the reaction vessel 16-1 and the reaction vessel 16-2, 1.5 to 2.0 times by mole of 1-butanol to the used chloroform was respectively added. The gas discharged from the reaction vessel 16-2 was further supplied into an alkali trap so that toxic gas was not leaked outside.

To the reaction mixtures in the reaction vessel 16-1 and the reaction vessel 16-2, 1,2-dichoroethane was added as an internal standard after the reaction, and the mixture was analyzed by ¹H NMR to determine the yield amounts and the yields of the produced chloroformic acid ester and carboxylic acid anhydride. The amount of the produced phosgene was calculated on the basis of the total thereof. The result is shown in Table 25. The yield of phosgene in Table 25 is the yield to the used chloroform.

**Table 25**

| Entry | CHCl₃ | | | Air flow amount | Mixed gas | | Experimental result | | |
|---|---|---|---|---|---|---|---|---|---|
| | Liquid flow amount | Gas flow amount | Total amount | | Linear velocity | Residence time | Produced phosgene amount | Phosgene yield | Unreacted CHCl₃ |
| | Photoreaction volume:335mL Louter tube (φ60x2.4t) - inner tube (φ30x1.5t)] | | | | | | | | |
| 1 | 25.15µL/min | 8.5mL/min | 4.5g | 100 mL/min | 0.071 m/min | 167s | 36 mmol | 98% | 1% |
| | | 0.31mmol/min | 37.5mmol | | | | | | |
| 2 | 100.6µL/min | 34mL/min | 17.9g | 100 mL/min | 0.086 m/min | 138s | 137 mmol | 92% | 6% |
| | | 1.24mmol/min | 150mmol | | | | | | |

| | Photoreaction volume:700mL [outer tube (φ80×2.5t) - inner tube (φ30x1.5t)] | | | | | | | | |
|---|---|---|---|---|---|---|---|---|---|
| 3 | 67.1µL/min | 25mL/min | 11.9g | 66.6 mL/min | 0.026 m/min | 432s | 96 mmol | 96% | 4% |
| | | 0.83mmol/min | 100mmol | | | | | | |
| 4 | 151µL/min | 56mL/min | 26.7g | 150 mL/min | 0.059 m/min | 192s | 201 mmol | 90% | 10% |
| | | 1.88mmol/min | 224mmol | | | | | | |

| | Photoreaction volume:2450mL [outer tube (φ140×2.5t) - inner tube (φ30×1.5t)] | | | | | | | | |
|---|---|---|---|---|---|---|---|---|---|
| 5 | 100.6µL/min | 37mL/min | 18.7g | 100 mL/min | 0.011 m/min | 1008s | 149 mmol | 95% | 5% |
| | | 1.25mmol/min | 157mmol | | | | | | |
| 6 | 402.4µL/min | 137mL/min | 71.0g | 400 mL/min | 0.043 m/min | 252s | 430 mmol | 72% | 28% |
| | | 5mmol/min | 595mmol | | | | | | |

When the volume of the photoreaction vessel is relatively small, the amount of the chloroform to be injected should be adjusted to be small in order to maintain the phosgene yield to be high and reduce unreacted chloroform due to a short residence time of the mixed gas as the result shown in Table 25.

Thus, the volume of the photoreaction vessel was adjusted to be larger. As a result, the injectable amount became larger and the yield amount of phosgene could be larger, since the residence time of the mixed gas became long. But the amount of the unreacted chloroform was slightly increased, since there might be an area to which high energy light was not sufficiently irradiated.

### Example 29: Selection of gas phase photoreaction vessel

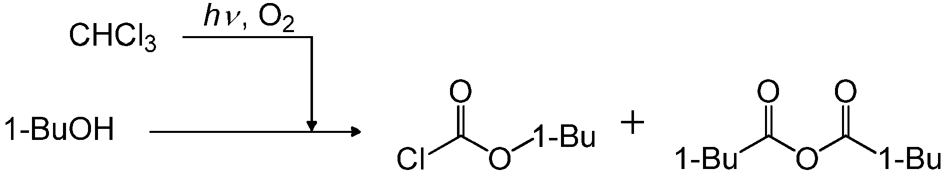

The gas phase photoreaction was carried out using the photoreaction system schematically demonstrated in Figure 5. Specifically, the reaction system was constructed by inserting a quartz glass jacket of φ30 × 1.5t and the length of 550 mm in a cylindrical reaction vessel of φ60 × 2.4t and the length of 550 mm, and further inserting a low pressure mercury lamp ("SUL-400" manufactured by SEN Light, 40 W, length of light emitting part: 380 mm, wavelength: 185 to 600 nm, peak wavelength: 184.9 nm and 253.7 nm) in the quartz glass jacket. The effective volume of the photoreaction vessel was 976 mL.

Liquid chloroform and oxygen of 20°C were supplied to the coil heater 3 heated to 150°C at the flow amount shown in Table 26 using the syringe pump 1 and the mass flow controller 2 ("MODEL8500MC" manufactured by KOFLOC) to be mixed and heated, and supplied to the photoreaction vessel 12 for 2 hours. The temperature of the heater 13 for heating the reaction vessel was adjusted to 100°C.

The gas discharged from the photoreaction vessel 12 was blown into 1-butanol in the reaction vessel 16-1, and the gas discharged from the reaction vessel 16-1 was further blown into 1-butanol in the reaction vessel 16-2. In the reaction vessel 16-1 and the reaction vessel 16-2, 1.5 to 2.0 times by mole of 1-butanol was respectively added to the used chloroform. The gas discharged from the reaction vessel 16-2 was further supplied into an alkali trap so that toxic gas was not leaked outside.

After the reaction, 1,2-dichloroethane was added as an internal standard in the reaction mixtures in the reaction vessel 16-1 and the reaction vessel 16-2, and the mixture was analyzed by ¹H NMR to determine the yield amounts and the yields of the produced chloroformic acid ester and carboxylic acid anhydride. The amount of the produced phosgene was calculated on the basis of the total thereof. The result is shown in Table 26. The yield of phosgene in Table 26 is the yield to the used chloroform.

**Table 26**

| Entry | CHCl₃ | | | Air flow rate | Mixed gas | | Experimental result | | |
|---|---|---|---|---|---|---|---|---|---|
| | Liquid flow amount | Gas flow amount | Total amount | | Linear velocity | Residence time | Produced phosgene amount | Phosgene yield | Unreacted CHCl₃ |
| 1 | 0.101mL/min | 43mL/min | 18.4g | 100 mL/min | 0.25 m/min | 401s | 133 mmol | 86% | ND |
| | | 1.25mmol/min | 154mmol | | | | | | |
| 2 | 0.201mL/min | 87mL/min | 36.2g | 100 mL/min | 0.31 m/min | 325s | 273 mmol | 89% | 0.2% |
| | | 2.5mmol/min | 303mmol | | | | | | |

The decomposition efficiency of chloroform could be further improved by using a long light source and thus irradiating strong high energy light to the chloroform/oxygen-containing gas for a long time.

### Example 30: Selection of oxygen source

The production efficiency of phosgene was tested similarly to Example 29 except that the reaction conditions were changed as Table 27. Air was used as an oxygen source, air was dried using a desiccant ("Silica Gel, Medium Granular, Blue" manufactured by FUJIFILM Wako Pure Chemical) in Entry 1, Entry 4 and Entry 5, air under the humidity of 60 to 80% during rain was directly used in Entry 2, and the air derived from a dried air tank was used in Entry 3. The result is shown in Table 27.

**Table 27**

| Entry | CHCl₃ | | | Air flow amount (Oxygen flow amount) | Mixed gas | | Experimental result | | |
|---|---|---|---|---|---|---|---|---|---|
| | Liquid flow amount | Gas flow amount | Total amount | | Linear velocity | Residence time | Produced phosgene amount | Phosgene yield | Unreacted CHCl₃ |
| | Photoreaction volume:976mL [outer tube (φ60×2.4t) - inner tube (φ30x1.5t)] | | | | | | | | |
| 1 | 0.201 mL/min | 87mL/min | 304 mmol | 0.2L/min (1.6mmol/min) | 0.51 m/min | 201s | 281 mmol | 93% | 0.1% |
| | | 2.5mmol/min | | | | | | | |
| 2 | 0.201 mL/min | 66mL/min | 300 mmol | 0.4L/min (3.3mmol/min) | 0.89 m/min | 114s | 272 mmol | 91% | 1.7% |
| | | 2.5mmol/min | | | | | | | |
| 3 | 0.201 | 66mL/min | 303 mmol | 0.4L/min (3.3mmol/min) | 0.89 m/min | 114s | 270 mmol | 89% | 5.3% |
| | mL/min | 2.5mmol/min | | | | | | | |

| | Photoreaction volume:2134mL [outer tube (φ80×2.4t) - inner tube (φ30×1.5t)] | | | | | | | | |
|---|---|---|---|---|---|---|---|---|---|
| 4 | 301.8 µL/min | 118mL/min | 449 mmol | 252mL/min (2.1mmol/min) | 0.10 m/min | 201s | 446 mmol | 99.4% | 0. 4% |
| | | 3.75mmol/min | | | | | | | |
| 5 | 603.5 µL/min | 236mL/min | 901 mmol | 504mL/min (4.1mmol/min) | 0.21 m/min | 61s | 843 mmol | 93.5% | 6.4% |
| | | 7.5mmol/min | | | | | | | |

It was found as the result shown in Table 27 that some amount of water does not become a problem as the decomposition efficiency of chloroform and the phosgene yield were not decreased even in the case where the air without drying was used (Entry 2).

When the flow amount of oxygen was smaller than the flow amount of chloroform (Entry 1 and Entry 4), unreacted chloroform was small and the phosgene yield to chloroform was high.

Though the flow amount of oxygen was smaller than the flow amount of chloroform, the ratio of unreacted chloroform was relatively large in Entry 5. The reason may be that the flow amounts of chloroform and air were large. But the efficiency of Entry 5 is high, since the amount of the produced phosgene was very large and the phosgene yield was also relatively high in the case of Entry 5.

### Example 31: Selection of gas phase photoreaction vessel

The gas phase photoreaction was carried out using the photoreaction system constructed by connecting two gas phase photoreaction vessels in series as schematically demonstrated in Figure 6. Specifically, the photoreaction vessel 12-1 was constructed by inserting a quartz glass jacket of φ30 × 1.5t in a cylindrical reaction vessel of φ80 × 2.5t or φ140 × 2.5t, and further inserting a low pressure mercury lamp ("SUL-20P" manufactured by SEN Light, 20 W, length of light emitting part: 130 mm, wavelength: 185 to 600 nm, peak wavelength: 184.9 nm and 253.7 nm) in the quartz glass jacket. Also, the photoreaction vessel 12-2 was constructed by inserting a quartz glass jacket of φ30 × 1.5t in a cylindrical reaction vessel of φ60× 2.5t, and further inserting the low pressure mercury lamp in the quartz glass jacket. The reaction system was constructed by connecting the photoreaction vessel 12-1 and the photoreaction vessel 12-2.

Liquid chloroform and oxygen of 20°C were supplied to the coil heater 3 heated to 90°C at the flow amount shown in Table 28 to be mixed and heated, and supplied to the photoreaction vessel 12-1 for 2 hours. The temperature of the heater 13 for heating the photoreaction vessel 12 was adjusted to 100°C.

The gas discharged from the photoreaction vessel 12-2 was blown into 1-butanol in the reaction vessel 16-1, and the gas discharged from the reaction vessel 16-1 was further blown into 1-butanol in the reaction vessel 16-2. In the reaction vessel 16-1 and the reaction vessel 16-2, 1.5 to 2.0 times by mole of 1-butanol was respectively added to the used chloroform. The gas discharged from the reaction vessel 16-2 was further supplied into an alkali trap so that toxic gas was not leaked outside.

After the reaction, 1,2-dichloroethane was added as an internal standard in the reaction mixtures in the reaction vessel 16-1 and the reaction vessel 16-2, and the mixtures were analyzed by ¹H NMR to determine the yield amounts and the yields of the produced chloroformic acid ester and carboxylic acid anhydride. The amount of the produced phosgene was calculated on the basis of the total thereof. The result is shown in Table 28. The yield of phosgene in Table 28 is the yield to the used chloroform.

**Table 28**

| Entry | CHCl₃ | | | Air flow amount (Oxygen flow amount) | Mixed gas | | Experimental result | | |
|---|---|---|---|---|---|---|---|---|---|
| | Liquid flow amount | Gas flow amount | Total amount | | Linear velocity | Residence time | Produced phosgene amount | Phosgene yield | Unreacted CHCl₃ |
| | Photoreaction volume:700mL + 976mL | | | | | | | | |
| 1 | 150.9 µL/min | 56mL/min | 26. 9g | 150mL/min (1.2mmol/min) | 0.081 m/min | 459s | 224 mmol | 99.7% | 0.3% |
| | | 1.88mmol/min | 225mmol | | | | | | |
| 2 | 201.2 µL/min | 74mL/min | 36.1g | 200mL/min (1.6mmol/min) | 0.108 m/min | 345s | 298 mmol | 98.4% | 1. 6% |
| | | 2.5mmol/min | 302mmol | | | | | | |
| | Photoreaction volume:2450mL + 976mL | | | | | | | | |
| 3 | 201.2 µL/min | 74mL/min | 36.1g | 200mL/min (1.6mmol/min) | 0.038 m/min | 704s | 296 mmol | 98.0% | 1% |
| | | 2.5mmol/min | 302mmol | | | | | | |

For example, it can be argued by comparing Entry 1 of Example 30 with Entry 2 of Example 31 that chloroform conversion ratio, phosgene yield amount and phosgene yield can be improved by connecting two photoreaction vessels. In addition, when the mixed gas passes through the thin tube to connect the first photoreaction vessel 12-1 and the second photoreaction vessel 12-2, unreacted vaporized chloroform and oxygen are mixed more homogeneously and thus the oxidative photodecomposition of chloroform may efficiently proceed in the second photoreaction vessel 12-2.

### Example 32: Selection of gas phase photoreaction vessel

The gas phase photoreaction was carried out using the photoreaction system schematically demonstrated in Figure 6. Specifically, the photoreaction vessel 12-1 was constructed by inserting a quartz glass jacket of φ30 × 1.5t in a cylindrical reaction vessel of φ80 × 2.4t, and further inserting a low pressure mercury lamp ("SUV-40D" manufactured by SEN Light, 40 W, length of light emitting part: 380 mm, wavelength: 185 to 600 nm, peak wavelength: 184.9 nm and 253.7 nm) in the quartz glass jacket. Also, the photoreaction vessel 12-2 was constructed by inserting a quartz glass jacket of φ30 × 1.5t in a cylindrical reaction vessel of φ80× 2.5t, and further inserting a low pressure mercury lamp ("SUL-20P" manufactured by SEN Light, 20 W, length of light emitting part: 130 mm, wavelength: 185 to 600 nm, peak wavelength: 184.9 nm and 253.7 nm) in the quartz glass jacket. The reaction system was constructed by connecting the photoreaction vessel 12-1 and the photoreaction vessel 12-2.

Liquid chloroform and dried air of 20°C were supplied to the coil heater 3 heated to 150°C at the flow amount shown in Table 29 to be mixed and heated, and supplied to the photoreaction vessel 12-1 for 2 hours. The temperature of the heater for heating the photoreaction vessel 12 was adjusted to 100°C.

The gas discharged from the photoreaction vessel 12 was blown into 1-butanol in the reaction vessel 16-1, and the gas discharged from the reaction vessel 16-1 was further blown into 1-butanol in the reaction vessel 16-2. In the reaction vessel 16-1 and the reaction vessel 16-2, 1.5 to 2.0 times by mole of 1-butanol was respectively added to the used chloroform. The gas discharged from the reaction vessel 16-2 was further supplied into an alkali trap so that toxic gas was not leaked outside.

After the reaction, 1,2-dichloroethane was added as an internal standard in the reaction mixtures in the reaction vessel 16-1 and the reaction vessel 16-2, and the mixtures were analyzed by ¹H NMR to determine the yield amounts and the yields of the produced chloroformic acid ester and carboxylic acid anhydride. The amount of the produced phosgene was calculated on the basis of the total thereof. The result is shown in Table 29. The yield of phosgene in Table 29 is the yield to the used chloroform.

**Table 29**

| Entry | CHCl₃ | | | Air flow rate (oxygen flow rate) | Mixed gas | | Experimental result | | |
|---|---|---|---|---|---|---|---|---|---|
| | Liquid flow amount | Gas flow amount | Total amount | | Linear velocity | Residence time | Produced phosgene amount | Phosgene yield | Unreacted CHCl₃ |
| 1 | 448.2 µL/min | 210mL/min | 802mmol | 448mL/min (3.7mmol/min) | 0.18 m/min | 249s | 791 mmol | 98.7% | 0.1% |
| | | 6.7mmol/min | | | | | | | |
| 2 | 670.6 µL/min | 262mL/min | 1007mmol | 560mL/min (4.6mmol/min) | 0.23 m/min | 199s | 1009 mmol | 99.0% | 1% |
| | | 8.3mmol/min | | | | | | | |

It was found as the result shown in Table 29 that the conversion ratio of chloroform, and the yield amount and the yield of phosgene are remarkably improved by mixing chloroform and the air containing oxygen at a molar ratio of about 0.55 to the chloroform and subjecting the mixed gas to a photodecomposition reaction. In addition, air may inhibit the further decomposition of the produced phosgene by absorbing excessive high energy light, especially ultraviolet light.

### Example 33

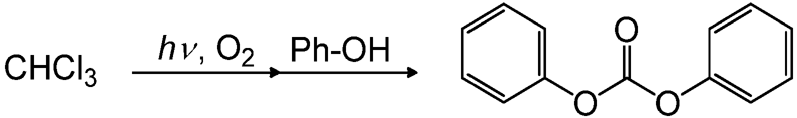

The gas phase photoreaction was carried out using the system similar to the photoreaction system as schematically demonstrated in Figure 5. Specifically, the photoreaction system was constructed by inserting a quartz glass jacket having the diameter of 30 mm in a cylindrical reaction vessel having the diameter of 60 mm and the volume of 1360 mL, and further inserting a low pressure mercury lamp ("SUV40 D" manufactured by SEN Light, 40 W, φ22.3 × 380 mm, wavelength: 185 to 600 nm, peak wavelength: 184.9 nm and 253.7 nm) in the quartz glass jacket. The irradiated light contained UV-C having the wavelength of 185 nm and the wavelength of 254 nm, and the illumination intensity of the light having the wavelength of 185 nm at the position 5 mm away from the center of the lamp to the reaction mixture was 2.00 to 2.81 mW/cm², and the illumination intensity of the light having the wavelength of 254 nm was 5.60 to 8.09 mW/cm². The total volume of the cylindrical flow photoreaction device 12 was 976 mL.

Liquid chloroform (450 mmol) was supplied into the PTFE tube having the inner diameter of 1 mm using the syringe pump 1 at the flow amount of 0.2 mL/min (2.5 mmol/min), mixed with the air of which flow amount was adjusted to 200 mL/min by the air pump and the mass flow controller 2, and vaporized by the heater 3 heated to 60°C to be supplied into the above-described flow photoreaction device 4. The mixed gas was heated again in the flow photoreaction vessel by the heater 13 attached to the bottom of the flow photoreaction device. The temperature of the heater was adjusted to 100°C. The linear velocity of the mixed gas in the photoreaction device could be estimated at 0.18 m/min, and the residence time could be estimated at 188 seconds.

Pyridine was injected into the stirred phenol (900 mmol) in the reaction vessel 16 using a syringe pump at the flow amount of 0.52 mL/min (6.5 mmol/min), and the gas produced by oxidative photodecomposition of the mixed gas of chloroform and air was blown thereinto at 0°C 3 hours. The unreacted gas was supplied into the further connected alkali trap in order not to be leaked outside.

Then, the reaction mixture in the reaction vessel 16 was washed with 1 M hydrochloric acid and water, and extraction was carried out using dichloromethane. The obtained extract was dried over anhydrous sodium sulfate, and the solvent was distilled away under reduced pressure to obtain white solid diphenyl carbonate (yield amount: 70.9 g, yield: 74%).

### Example 34

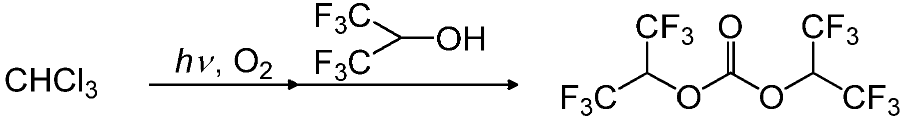

The gas phase photoreaction was carried out using the photoreaction system used in Example 33. Liquid chloroform (450 mmol) was supplied into the PTFE tube having the inner diameter of 1 mm using the syringe pump 1 at the flow amount of 0.2 mL/min (2.5 mmol/min), mixed with the air of which flow amount was adjusted to 200 mL/min by the air pump and the mass flow controller 2, and vaporized by the heater 3 heated to 60°C to be supplied into the flow photoreaction device 4. The mixed gas was heated again in the flow photoreaction device 4 by the heater 13 attached to the bottom of the flow photoreaction device. The temperature of the heater was adjusted to 100°C. The linear velocity of the mixed gas could be estimated at 0.18 m/min, and the residence time could be estimated at 188 seconds.

Pyridine was added to the stirred chloroform solution of HFIP (900 mmol) in the reaction vessel 16 using a syringe pump at the flow amount of 0.52 mL/min (6.5 mmol/min), and the gas produced by oxidative photodecomposition of the mixed gas of chloroform and air was blown thereinto at 0°C 3 hours. The unreacted gas was supplied into the further connected alkali trap to be treated so that toxic gas was not leaked outside.

Then, the reaction mixture in the reaction vessel 16 was washed with 1 M hydrochloric acid and water, and analyzed by ¹H NMR. As a result, it was confirmed that bis(hexafluoroisopropyl) carbonate (BHFC) was produced with the yield of 85%.

In addition, the washed reaction mixture was dried over anhydrous sodium sulfate, and colorless liquid BHFC was isolated by distillation (isolated yield amount: 88.0 g, 384 mmol, yield to HFIP: 54%).

### Example 35

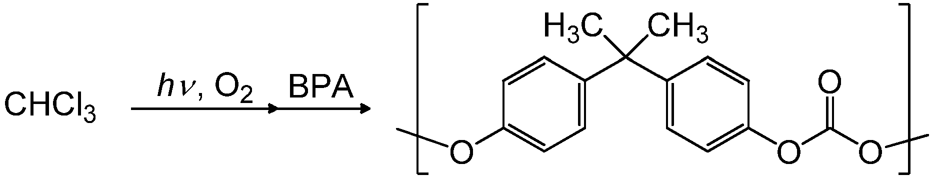

Vaporized chloroform was oxidatively photodecomposed using high energy light similarly to Example 34.

The gas produced by oxidative photodecomposition of the mixed gas of chloroform and air was blown into the stirred chloroform solution containing Bisphenol A (BPA) (405 mmol) and pyridine (2025 mmol) in the reaction vessel 16 at 0°C for 3 hours. The unreacted gas was supplied into the further connected alkali trap to be treated so that toxic gas was not leaked outside.

Then, the reaction mixture in the reaction vessel 16 was washed with 1 M hydrochloric acid and water, and extraction was carried out using dichloromethane. The obtained extract was dried over anhydrous sodium sulfate, and the solvent was distilled away under reduced pressure to obtain white solid polycarbonate (yield amount: 102 g, yield: 95%).

The produced polycarbonate was analyzed by gel permeation chromatography (GPC) to determine the molecular weight. The result is shown in Table 30.

**Table 30**

| Mw | Mn | Mw /Mn |
|---|---|---|
| 21,900 | 10,100 | 2.16 |

### Example 36

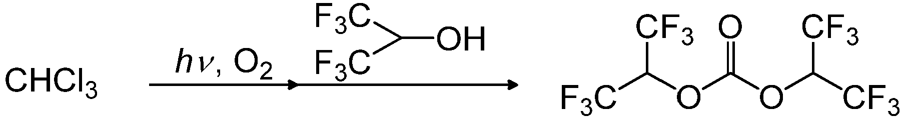

The gas phase photoreaction was carried out using the system similar to the photoreaction system as schematically demonstrated in Figure 5. Specifically, the photoreaction system was constructed by inserting a quartz glass jacket having the diameter of 30 mm in a cylindrical reaction vessel having the diameter of 140 mm and the volume of 2575 mL, and further inserting a low pressure mercury lamp ("UVL20PH-6" manufactured by SEN Light, 20 W, φ24 mm × 120 mm, wavelength: 185 to 600 nm, peak wavelength: 184.9 nm and 253.7 nm) in the quartz glass jacket. The light irradiated from the low pressure mercury lamp contained UV-C having the wavelength of 185 nm and the wavelength of 254 nm, and the illumination intensity of the light having the wavelength of 185 nm at the position 5 mm away from the center of the lamp to the reaction mixture was 2.00 to 2.81 mW/cm², and the illumination intensity of the light having the wavelength of 254 nm was 5.60 to 8.09 mW/cm². The total volume of the cylindrical flow photoreaction device 12 was 2450 mL.

Liquid chloroform (900 mmol) was supplied into the PTFE tube having the inner diameter of 1 mm using the syringe pump 1 at the flow amount of 0.4 mL/min (5.0 mmol/min), mixed with the air of which flow amount was adjusted to 200 mL/min by the air pump and the mass flow controller 2, and vaporized by the heater 3 heated to 60°C to be supplied into the above-described flow photoreaction device 12. The mixed gas was heated again in the flow photoreaction vessel by the heater 13 attached to the bottom of the flow photoreaction device. The temperature of the heater was adjusted to 100°C. The linear velocity of the mixed gas in the photoreaction device could be estimated at 0.046 m/min, and the residence time could be estimated at 235 seconds.

The gas produced by oxidative photodecomposition of the mixed gas of chloroform and air was blown into the stirred chloroform solution containing HFIP (1200 mmol) and pyridine (1800 mmol) in the reaction vessel 16 at 0°C 3 hours. The unreacted gas was supplied into the further connected alkali trap to be treated so that toxic gas was not leaked outside.

Then, the reaction mixture in the reaction vessel 16 was washed with 1 M hydrochloric acid and water, and analyzed by ¹H NMR. As a result, it was confirmed that bis(hexafluoroisopropyl) carbonate was produced (yield amount: 588 mmol, yield: 98%).

### Example 37: Decomposition of chloroform by visible light

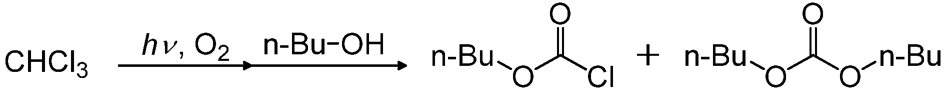

The chloroform product (500 mL) that contained ethanol as a stabilizing agent was washed with distilled water and then the chloroform layer and the aqueous layer were separated. This procedure was repeated 3 times. The chloroform layer was dried over anhydrous sodium sulfate, and the anhydrous sodium sulfate was removed by filtration. Calcium hydride (1 g) was added, and the mixture was stirred at 30°C overnight. Then, chloroform that did not contain the stabilizing agent was obtained by distillation.

The gas phase photoreaction was carried out using the system similar to the photoreaction system as schematically demonstrated in Figure 5. Specifically, the photoreaction system was constructed by inserting a quartz glass jacket of φ30 × 1.5t in a cylindrical reaction vessel of φ140 × 2.5t, inserting a low pressure mercury lamp ("UVL20PH-6" manufactured by SEN Light, 20 W, φ24 mm × 120 mm, wavelength: 185 to 600 nm, peak wavelength: 184.9 nm and 253.7 nm) in the quartz glass jacket, and further placing 365 nm LED lamp ("LKI-152" manufactured by Polarstar, 30 W, light emitting part: 250 × 150 mm, peak wavelength: 365 nm) at about 1 cm position away from the outside of the cylindrical reaction vessel. The effective volume of the inside of the photoreaction device 12 was 2.5 L. The illumination intensity of the light at the 5 mm position away from the used LED lamp was 34 to 38 mW/cm².

The liquid chloroform from which the stabilizing agent was removed as described above and oxygen of 20°C were supplied to the coil heater 3 heated to 110°C using the syringe pump 1 and the mass flow controller 2 ("MODEL8500MC" manufactured by KOFLOC) at the flow amount shown in Table 31 to be mixed and heated. The mixed gas was supplied to the photoreaction vessel 12 for 2 hours. The temperature of the heater 13 for heating the reaction device was adjusted to 100°C.

Visible light was irradiated to the mixed gas in the photoreaction device 12 using the LED lamp only or visible light was irradiated using the LED lamp for 5 minutes and then UV-C light was additionally irradiated using the low pressure mercury lamp for 1 minute and only visible light was irradiated again.

The gas discharged from the photoreaction vessel was blown into 1-butanol in the reaction vessel 16-1, and the gas discharged from the reaction vessel 16-1 was further blown into 1-butanol in the reaction vessel 16-2. In the reaction vessel 16-1 and the reaction vessel 16-2, 2.0 times by mole of 1-butanol was respectively added to the used chloroform. The gas discharged from the reaction vessel 16-2 was further supplied into an alkali trap to be treated so that toxic gas was not leaked outside.

After the reaction, 1,2-dichloroethane was added as an internal standard in the reaction mixtures in the reaction vessel 16-1 and the reaction vessel 16-2, and the mixture was analyzed by ¹H NMR to determine the yield amounts and the yields of the produced chloroformic acid ester and carboxylic acid anhydride. The amount of the produced phosgene was calculated on the basis of the total thereof. The result is shown in Table 31. The yield of phosgene in Table 31 is the yield to the used chloroform.

**Table 31**

| Entry | CHCl₃ | | | Air flow amount (Oxygen flow amount) | Mixed gas | | Irradiated light | Experimental result | | |
|---|---|---|---|---|---|---|---|---|---|---|
| | Liquid flow amount | Gas flow amount | Total amount | | Linear velocity | Residence time | | Produced phosgene amount | Phosgene yield | Unreacted CHCl₃ |
| 1 | 402.4 µL/min | 157mL/min | 603mmol | 67.2mL/min | 0.016 m/min | 696s | Visible light | 460 mmol | 76% | 23% |
| | | 5.0mmol/min | | 2.8mmol/min | | | | | | |
| 2 | 402.4 µL/min | 157mL/min | 603mmol | 67.2mL/min | 0.016 m/min | 696s | Visible light + UV | 466 mmol | 78% | 21% |
| | | 5.0mmol/min | | 2.8mmol/min | | | | | | |
| 3 | 212.4 µL/min | 79mL/min | 301mmol | 33.6mL/min | 0.008 m/min | 1386s | Visible light | 185 mmol | 61% | 36% |
| | | 2.5mmol/min | | 1.4mmol/min | | | | | | |
| 4 | 212.4 µL/min | 79mL/min | 313mmol | 33.6mL/min | 0.008 m/min | 1386s | Visible light + UV | 260 mmol | 83% | 16% |
| | | 2.5mmol/min | | 1.4mmol/min | | | | | | |

When chloroform that did not contain a stabilizing agent was used, the amount of an unreacted chloroform was somewhat increased but chloroform could be decomposed and phosgene could be produced even by visible light as the result shown in Table 31. The reason why chloroform was decomposed and phosgene was produced by irradiating light of which energy was relatively low may be that chloroform did not contain a stabilizing agent and the oxidative photodecomposition reaction was carried out in the gas phase.

In addition, the yield amount and the yield were increased and the amount of the unreacted chloroform was decreased by irradiating ultraviolet light for a short time in addition to visible light at the early stage of the oxidative photodecomposition reaction. The reason may be that the C-Cl bond was cleaved by high energy ultraviolet light and then the oxidative photodecomposition reaction of chloroform progressed by radical chain mechanism even by using visible light only thereafter.

### EXPLANATION OF REFERENCES

1: Syringe pump, 2: Mass flow controller, 3: Heater,
4: Flow photoreaction device, 5: Back pressure regulator,
6: Reaction vessel, 7: Trap vessel,
8: Syringe pump for injecting reaction substrate,
9: Coil reaction device, 10: Recovery vessel,
11: Light source, 12: Photoreaction device, 13: Bath,
14: Stirring bar, 15: Cooling condenser,
16: Reaction vessel, 17: Tube reactor

## Claims

1. A method for producing a carbonyl halide, comprising the steps of:
preparing a mixed gas comprising oxygen and a halogenated methane having one or more halogeno groups selected from the group consisting of chloro, bromo and iodo, and
flowing the mixed gas and irradiating a high energy light containing light having a wavelength of 180 nm or more and 830 nm or less to the flowed mixed gas.

2. The method according to claim 1, wherein a shortest distance from a light source of the high energy light to the flowed mixed gas is 1 m or less.

3. The method according to claim 1 or 2, wherein time to irradiate the high energy light to the flowed mixed gas is 1 second or more and 10000 seconds or less.

4. The method according to any one of claims 1 to 3, wherein a temperature during the irradiation of the high energy light to the flowed mixed gas is 40°C or higher and 200°C or lower.

5. The method according to any one of claims 1 to 4, wherein the high energy light contains light having a wavelength of 180 nm or more and 500 nm or less.

6. A method for producing a fluorinated carbonate compound, comprising the steps of:
producing a carbonyl halide by the method according to any one of claims 1 to 5, and
reacting a fluorinated alcohol compound and the carbonyl halide,
wherein a molar ratio of the fluorinated alcohol compound to the halogenated methane is adjusted to 1 or more.

7. A method for producing a non-fluorinated carbonate compound, comprising the steps of:
producing a carbonyl halide by the method according to any one of claims 1 to 5, and
reacting a non-fluorinated alcohol compound and the carbonyl halide,
wherein a molar ratio of the non-fluorinated alcohol compound to the halogenated methane is adjusted to 1 or more.

8. A method for producing a halogenated formic acid fluorinated ester compound, comprising the steps of:
producing a carbonyl halide by the method according to any one of claims 1 to 5, and
reacting a fluorinated alcohol compound and the carbonyl halide,
wherein a molar ratio of the fluorinated alcohol compound to the halogenated methane is adjusted to less than 1.

9. A method for producing a halogenated formic acid non-fluorinated ester compound, comprising the steps of:
producing a carbonyl halide by the method according to any one of claims 1 to 5, and
reacting a non-fluorinated alcohol compound and the carbonyl halide, wherein a molar ratio of the non-fluorinated alcohol compound to the halogenated methane is adjusted to less than 1.

10. A method for producing an isocyanate compound, comprising the steps of:
producing a carbonyl halide by the method according to any one of claims 1 to 5, and
reacting a primary amine compound and the carbonyl halide,
wherein a molar ratio of the primary amine compound to the halogenated methane is adjusted to less than 1.

11. A method for producing an amino acid-N-carboxylic anhydride, comprising the steps of:
producing a carbonyl halide by the method according to any one of claims 1 to 5, and
reacting an amino acid compound represented by the following formula (VII) and the carbonyl halide,
wherein the amino acid-N-carboxylic anhydride is represented by the following formula (VIII): 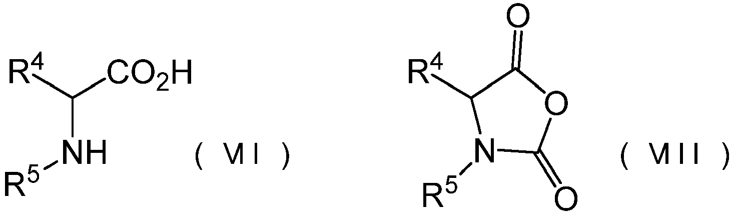 wherein
R⁴ is an amino acid side chain group wherein a reactive group is protected,
R⁵ is H or P¹-[-NH-CHR⁶-C(=O)-]_{I}- wherein R⁶ is an amino acid side chain group wherein a reactive group is protected, P¹ is a protective group of the amino group, I is an integer of 1 or more, and when I is an integer of 2 or more,
a plurality of R⁶ may be the same as or different from each other.

12. A method for producing a Vilsmeier reagent,
wherein the Vilsmeier reagent is a salt represented by the following formula (X): 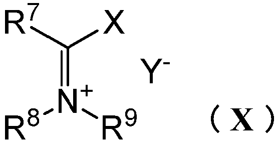 wherein
R⁷ is a hydrogen atom, a C₁₋₆ alkyl group or an optionally substituted C₆₋₁₂ aromatic hydrocarbon group,
R⁸ and R⁹ are independently a C₁₋₆ alkyl group or an optionally substituted C₆₋₁₂ aromatic hydrocarbon group, or R⁸ and R⁹ may form a 4 or more and 7 or less-membered ring structure together with each other,
X is a halogeno group selected from the group consisting of chloro, bromo and iodo,
Y⁻ is a counter anion,
comprising the steps of:
producing a carbonyl halide by the method according to any one of claims 1 to 5, and
reacting the carbonyl halide and an amide compound represented by the following formula (IX): 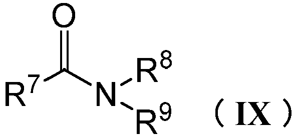
wherein R⁷ to R⁹ have the same meanings as the above.

## Patentansprüche

1. Verfahren zur Herstellung eines Carbonylhalogenids, umfassend die folgenden Schritte:
Zubereiten eines Mischgases, das Sauerstoff und ein halogeniertes Methan, das eine oder mehrere Halogengruppen aufweist, ausgewählt aus der Gruppe bestehend aus Chlor, Brom und Iod, umfasst, und
Strömen des Mischgases und Bestrahlen eines hochenergetischen Lichts, das Licht mit einer Wellenlänge von 180 nm oder mehr und 830 nm oder weniger enthält, auf das strömende Mischgas.

2. Verfahren nach Anspruch 1, wobei ein kürzester Abstand von einer Lichtquelle des hochenergetischen Lichts zum strömenden Mischgas 1 m oder weniger ist.

3. Verfahren nach Anspruch 1 oder 2, wobei die Zeit, um das hochenergetische Licht auf das strömende Mischgas zu bestrahlen, 1 Sekunde oder mehr und 10000 Sekunden oder weniger ist.

4. Verfahren nach einem der Ansprüche 1 bis 3, wobei eine Temperatur während der Bestrahlung des hochenergetischen Lichts auf das strömende Mischgas 40 °C oder höher und 200 °C oder niedriger ist.

5. Verfahren nach einem der Ansprüche 1 bis 4, wobei das hochenergetische Licht Licht, das eine Wellenlänge von 180 nm oder mehr und 500 nm oder weniger aufweist, enthält.

6. Verfahren zur Herstellung einer fluorierten Carbonatverbindung, umfassend die Schritte:
Herstellen eines Carbonylhalogenids durch das Verfahren nach einem der Ansprüche 1 bis 5 und
Umsetzen einer fluorierten Alkoholverbindung und des Carbonylhalogenids,
wobei ein Molverhältnis der fluorierten Alkoholverbindung zu dem halogenierten Methan auf 1 oder mehr eingestellt ist.

7. Verfahren zur Herstellung einer nicht fluorierten Carbonatverbindung, umfassend die Schritte:
Herstellen eines Carbonylhalogenids durch das Verfahren nach einem der Ansprüche 1 bis 5 und
Umsetzen einer nicht fluorierten Alkoholverbindung und des Carbonylhalogenids,
wobei ein Molverhältnis der nicht fluorierten Alkoholverbindung zu dem halogenierten Methan auf 1 oder mehr eingestellt ist.

8. Verfahren zur Herstellung einer halogenierten Ameisensäurefluorierten Esterverbindung umfassend die Schritte:
Herstellen eines Carbonylhalogenids durch das Verfahren nach einem der Ansprüche 1 bis 5 und
Umsetzen einer fluorierten Alkoholverbindung und des Carbonylhalogenids,
wobei ein Molverhältnis der fluorierten Alkoholverbindung zu dem halogenierten Methan auf weniger als 1 eingestellt ist.

9. Verfahren zur Herstellung einer halogenierten Ameisensäure-nicht fluorierten Esterverbindung, umfassend die Schritte:
Herstellung eines Carbonylhalogenids durch das Verfahren nach einem der Ansprüche 1 bis 5 und
Umsetzen einer nicht fluorierten Alkoholverbindung und des Carbonylhalogenids,
wobei ein Molverhältnis der nicht fluorierten Alkoholverbindung zu dem halogenierten Methan auf weniger als 1 eingestellt ist.

10. Verfahren zur Herstellung einer Isocyanatverbindung, umfassend die Schritte:
Herstellen eines Carbonylhalogenids durch das Verfahren nach einem der Ansprüche 1 bis 5 und
Umsetzen einer primären Aminverbindung und des Carbonylhalogenids,
wobei ein Molverhältnis der primären Aminverbindung zu dem halogenierten Methan auf weniger als 1 eingestellt ist.

11. Verfahren zur Herstellung eines Aminosäure-N-carbonsäureanhydrids, umfassend die Schritte:
Herstellen eines Carbonylhalogenids durch das Verfahren nach einem der Ansprüche 1 bis 5 und
Umsetzen einer Aminosäureverbindung dargestellt durch die folgende Formel (VII) und des Carbonylhalogenids,
wobei das Aminosäure-N-carbonsäureanhydrid durch die folgende Formel (VIII) dargestellt wird: 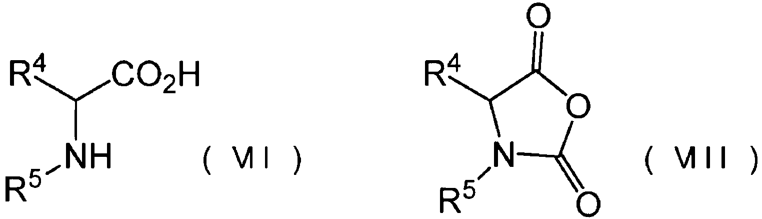 wobei
R⁴ eine Aminosäureseitenkettengruppe ist, wobei eine reaktive Gruppe geschützt ist,
R⁵ H oder P¹-[-NH-CHR⁶-C(=O)-]_{I-} ist, wobei R⁶ eine Aminosäureseitenkettengruppe ist, wobei eine reaktive Gruppe geschützt ist, P¹ eine Schutzgruppe der Aminogruppe ist, I eine ganze Zahl von 1 oder mehr ist, und wenn I eine ganze Zahl von 2 oder mehr ist, eine Vielzahl von R⁶ gleich oder voneinander verschieden sein kann.

12. Verfahren zur Herstellung eines Vilsmeier-Reagenzes,
wobei das Vilsmeier-Reagenz ein Salz ist, das durch die folgende Formel (X) dargestellt wird: 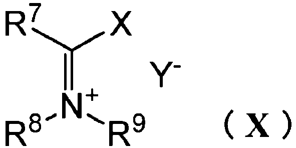 wobei
wobei R⁷ ein Wasserstoffatom, eine C₁₋₆-Alkylgruppe oder eine optional substituierte aromatische C₆₋₁₂ Kohlenwasserstoffgruppe ist,
R⁸ und R⁹ unabhängig voneinander eine C₁₋₆-Alkylgruppe oder eine optional substituierte aromatische C₆₋₁₂-Kohlenwasserstoffgruppe sind, oder R⁸ und R⁹ können zusammen miteinander eine 4- oder mehr- und 7- oder weniger-gliedrige Ringstruktur bilden,
X eine Halogengruppe ist, ausgewählt aus der Gruppe bestehend aus Chlor, Brom und Iod,
Y⁻ ein Gegenanion ist,
umfassend die Schritte:
Herstellen eines Carbonylhalogenids durch das Verfahren nach einem der Ansprüche 1 bis 5 und
Umsetzen des Carbonylhalogenids und einer Amidverbindung, dargestellt durch die folgende Formel (IX): 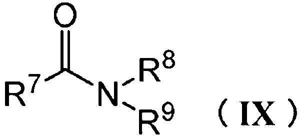
wobei R⁷ bis R⁹ die gleichen Bedeutungen wie oben aufweisen.

## Revendications

1. Procédé de production d'un halogénure de carbonyle comprenant les étapes de :
préparation d'un gaz mixte comprenant de l'oxygène et un méthane halogéné ayant un ou plusieurs groupes halogéno choisis dans le groupe constitué de chloro, bromo et iodo, et écoulement du gaz mixte et irradiation d'une lumière à haute énergie contenant une lumière ayant une longueur d'onde de 180 nm ou plus et 830 nm ou moins sur le gaz mixte s'écoulant.

2. Procédé selon la revendication 1, dans lequel une distance la plus courte depuis une source de lumière de la lumière à haute énergie au gaz mixte s'écoulant est de 1 m ou moins.

3. Procédé selon la revendication 1 ou 2, dans lequel le temps pour irradier la lumière à haute énergie sur le gaz mixte s'écoulant est de 1 seconde ou plus et de 10 000 secondes ou moins.

4. Procédé selon l'une quelconque des revendications 1 à 3, dans lequel une température pendant l'irradiation de la lumière à haute énergie sur le gaz mixte s'écoulant est de 40 °C ou plus et de 200 °C ou moins.

5. Procédé selon l'une quelconque des revendications 1 à 4, dans lequel la lumière à haute énergie contient une lumière ayant une longueur d'onde de 180 nm ou plus et 500 nm ou moins.

6. Procédé de production d'un composé de carbonate fluoré comprenant les étapes de :
production d'un halogénure de carbonyle par le procédé selon l'une quelconque des revendications 1 à 5 et
mise en réaction d'un composé de type alcool fluoré et de l'halogénure de carbonyle,
dans lequel un rapport molaire du composé de type alcool fluoré sur le méthane halogéné est ajusté jusqu'à 1 ou plus.

7. Procédé de production d'un composé de carbonate non fluoré comprenant les étapes de :
production d'un halogénure de carbonyle par le procédé selon l'une quelconque des revendications 1 à 5 et
mise en réaction d'un composé de type alcool non fluoré et de l'halogénure de carbonyle,
dans lequel un rapport molaire du composé de type alcool non fluoré sur le méthane halogéné est ajusté jusqu'à 1 ou plus.

8. Procédé de production d'un composé de type ester fluoré d'acide formique halogéné comprenant les étapes de :
production d'un halogénure de carbonyle par le procédé selon l'une quelconque des revendications 1 à 5 et
mise en réaction d'un composé de type alcool fluoré et de l'halogénure de carbonyle,
dans lequel un ratio molaire du composé de type alcool fluoré sur le méthane halogéné est ajusté jusqu'à moins de 1.

9. Procédé de production d'un composé de type ester non fluoré d'acide formique halogéné, comprenant les étapes de :
production d'un halogénure de carbonyle par le procédé selon l'une quelconque des revendications 1 à 5 et
mise en réaction d'un composé de type alcool non fluoré et de l'halogénure de carbonyle,
dans lequel un rapport molaire du composé de type alcool non fluoré sur le méthane halogéné est ajusté jusqu'à moins de 1.

10. Procédé de production d'un composé de type isocyanate comprenant les étapes de :
production d'un halogénure de carbonyle par le procédé selon l'une quelconque des revendications 1 à 5 et
mise en réaction d'un composé de type amine primaire et de l'halogénure de carbonyle,
dans lequel un rapport molaire du composé de type amine primaire sur le méthane halogéné est ajusté jusqu'à moins de 1.

11. Procédé de production d'un anhydride d'acide aminé-N-carboxylique comprenant les étapes de :
production d'un halogénure de carbonyle par le procédé selon l'une quelconque des revendications 1 à 5 et
mise en réaction d'un composé de type acide aminé représenté par la formule suivante (VII) et de l'halogénure de carbonyle,
dans lequel l'anhydride d'acide aminé-N-carboxylique est représenté par la formule suivante (VIII) : 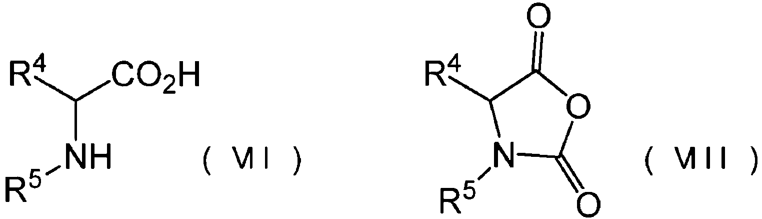
dans laquelle
R⁴ est un groupe de chaîne latérale d'acide aminé dans lequel un groupe réactif est protégé,
R⁵ est H ou P¹-[-NH-CHR⁶-C(=O)-]_{I}-, dans lequel R⁶ est un groupe de chaîne latérale d'acide aminé dans lequel un groupe réactif est protégé, P¹ est un groupe protecteur du groupe amino, I est un entier de 1 ou plus, et lorsque I est un entier de 2 ou plus,
une pluralité de R⁶ peuvent être identiques ou différents les uns des autres.

12. Procédé de production d'un réactif de Vilsmeier,
dans lequel le réactif de Vilsmeier est un sel représenté par la formule suivante (X) : 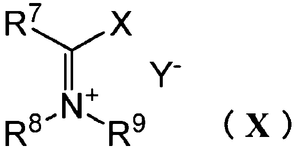
dans laquelle
R⁷ est un atome d'hydrogène, un groupe alkyle en C₁₋₆ ou un groupe hydrocarboné aromatique en C₆₋₁₂ éventuellement substitué,
R⁸ et R⁹ sont indépendamment un groupe alkyle en C₁₋₆ ou un groupe hydrocarboné aromatique en C₆₋₁₂ éventuellement substitué, ou R⁸ et R⁹ peuvent former ensemble une structure cyclique à 4 chaînons ou plus et 7 chaînons ou moins,
X est un groupe halogéno choisi dans le groupe constitué de chloro, bromo et iodo,
Y⁻ est un contre-anion,
comprenant les étapes de :
production d'un halogénure de carbonyle par le procédé selon l'une quelconque des revendications 1 à 5 et
mise en réaction de l'halogénure de carbonyle et d'un composé de type amide représenté par la formule suivante (IX) : 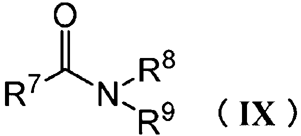
dans laquelle R⁷ à R⁹ ont les mêmes significations que ci-dessus.
